# EUROPEAN PATENT SPECIFICATION

(11) **EP 4 388 123 B1**
(45) Date of publication and mention of the grant of the patent: **18.06.2025**
(21) Application number: 22764553.8
(22) Date of filing: 11.08.2022
(51) Int. Cl.: C12Q 1/34

(54) **COMPOSITIONS, SYSTEMS AND METHODS FOR ENZYME OPTIMIZATION**
ZUSAMMENSETZUNGEN, SYSTEME UND VERFAHREN ZUR ENZYMOPTIMIERUNG
COMPOSITIONS, SYSTÈMES ET PROCÉDÉS D'OPTIMISATION ENZYMATIQUE

(30) Priority: 17.08.2021 US 202163233740 P
(43) Date of publication of application: 26.06.2024
(73) Proprietor: 10X Genomics, Inc., Pleasanton, CA 94588 (US)
(72) Inventor: GIBBONS, Michael, Pleasanton, California 94588 (US)
(74) Representative: J A Kemp LLP
(86) International application number: PCT/US2022/040122
(87) International publication number: WO 2023/022925

(56) References cited:
- WO-A1-2017/177153
- US-A1- 2020 407 467

## Description

### FIELD OF THE INVENTION

This description is generally directed towards reaction mixtures, compositions, systems, methods, and kits for enzyme detection and activity assays with enzyme optimization (e.g., CRISPR-based enzyme optimization). More specifically, the reaction mixtures, compositions, systems, methods, and kits include technologies for detecting and assessing substrate cleavage by an enzyme, wherein a substrate barcoded oligonucleotide (SBO) construct, including a barcode sequence, can be amplified upon substrate cleavage.

### BACKGROUND

Nucleotide modification machinery (e.g., CRISPR/Cas machinery) can be used to modify genomes of single cells and specific genes of interest. In particular, gene editing (e.g., CRISPR gene editing) is also useful for modifying the resulting polypeptide sequence of enzymes to optimize enzymatic activity for a specific or customized purpose or application. Unfortunately, once enzyme modification has occurred there are no currently existing technologies for detecting and assessing enzymatic activity on a small or single cell scale. Current enzymatic activity assays rely on bulk inputs requiring large quantities of enzymes and substrates resulting in a slow and costly process that is not commercially viable. As such, there is a need for technologies that can generate large pools of modified enzymes and then accurately measure enzymatic activity at a single cell level for each of those modified enzymes. The present disclosure addresses this and other needs.

### SUMMARY

The invention is as set forth in the appended claims.

Aspects of the disclosure comprise a reaction mixture for assessing enzymatic activity. In various embodiments the reaction mixture can comprise a cleavable substrate comprising a first portion linked to a second portion, a substrate barcoded oligonucleotide (SBO) construct, and a blocking construct. In some embodiments, the substrate barcoded oligonucleotide (SBO) construct can comprise a first oligonucleotide comprising a barcode sequence and a first linker that connects the first oligonucleotide to the first portion of the substrate. In some embodiments, the blocking construct can comprise a second oligonucleotide that is complementary to at least a portion of the first oligonucleotide and a second linker that connects the second oligonucleotide to the second portion of the substrate.

Aspects of the disclosure comprise a reaction mixture for assessing enzymatic activity. In various embodiments, the reaction mixture for assessing enzymatic activity can comprise a substrate complex comprising a plurality of cleavable substrates, a plurality of SBO constructs, and a plurality of blocking constructs. In some embodiments, the plurality of cleavable substrates can each a first portion linked to a second portion. In some embodiments the plurality of SBO constructs can each comprise a first oligonucleotide comprising a substrate barcode sequence that identifies the substrate and a first linker that connects the first oligonucleotide to a first portion of one of the substrates. In some embodiments, the plurality of blocking constructs can each comprise a second oligonucleotide that is complementary to at least a portion of the first oligonucleotide and a second linker that connects the second oligonucleotide to a second portion of one of the substrates.

Aspects of the disclosure comprise a method for assessing enzymatic activity. The method can comprise contacting a substrate complex with an enzyme, wherein the substrate complex comprises a cleavable substrate comprising a first portion linked to a second portion and a substrate barcoded oligonucleotide (SBO) construct. In some embodiments, the SBO construct can comprise a first oligonucleotide comprising a barcode sequence and a first linker that connects the first oligonucleotide to the first portion of the substrate. In some embodiments, the SBO construct can comprise a blocking construct, comprising a second oligonucleotide that is complementary to at least a portion of the first oligonucleotide and a second linker that connects the second oligonucleotide to the second portion of the substrate. In various embodiments, the method can comprise restricting movement of the blocking construct relative to the SBO construct to inhibit generation of a reverse complement of the barcode sequence with the blocking construct. In various embodiments, the method can comprise cleaving the cleavable substrate with the enzyme. In various embodiments, the method can comprise releasing the blocking construct from the SBO construct.

Aspects of the disclosure comprise a method for assessing and comparing enzymatic activity. In various embodiments, the method can comprise contacting a plurality of unique substrate complexes with a plurality of corresponding unique enzymes. In some embodiments, the plurality of unique substrate complexes can comprise a unique cleavable substrate comprising a first portion linked to a second portion. In some embodiments, the plurality of unique substrate complexes can comprise a unique substrate barcoded oligonucleotide (SBO) construct, comprising a first oligonucleotide comprising a substrate barcode sequence that identifies the unique cleavable substrate and a first linker that connects the first oligonucleotide to the first portion of the substrate. In some embodiments, the plurality of unique substrate complexes can comprise a blocking construct, comprising a second oligonucleotide that is complementary to at least a portion of the first oligonucleotide and a second linker that connects the second oligonucleotide to the second portion of the substrate. In various embodiments, the method can comprise restricting movement of the blocking constructs relative to the unique SBO constructs to inhibit generation of a reverse complement of the substrate barcode sequences with the blocking constructs. In various embodiments the method can comprise cleaving one or more of the unique substrates with its corresponding unique enzyme. In various embodiments, the method can comprise releasing one or more of the blocking constructs from the unique SBO constructs. In various embodiments, the method can comprise conducting an amplification reaction on the first oligonucleotides comprising the substrate barcode sequences. In various embodiments, the method can comprise assessing enzymatic activity based on a reaction product of the amplification reaction.

The invention provides a method for assessing enzymatic activity, comprising: (i) contacting a substrate complex with an enzyme, wherein the substrate complex comprises:
a cleavable substrate comprising a first portion linked to a second portion;
a substrate barcoded oligonucleotide (SBO) construct, comprising:
   a first oligonucleotide comprising a barcode sequence;
   and a first linker that connects the first oligonucleotide to the first portion of the substrate;
   a blocking construct, comprising a second oligonucleotide that is complementary to at least a portion of the first oligonucleotide;
   and a second linker that connects the second oligonucleotide to the second portion of the substrate;
   (ii) restricting movement of the blocking construct relative to the SBO construct to inhibit generation of a reverse complement of the barcode sequence with the blocking construct; (iii) cleaving the cleavable substrate with the enzyme; and
   (iv) releasing the blocking construct from the SBO construct.

In some embodiments, the method of assessing enzymatic activity is conducted within a partition. In various embodiments, the method can comprise providing a partition comprising a substrate complex and an enzyme. In various embodiments the substrate complex can comprise a cleavable substrate comprising a first portion linked to a second portion and a substrate barcoded oligonucleotide (SBO) construct, comprising a first oligonucleotide comprising a barcode sequence that identifies the cleavable substrate and a first linker that connects the first oligonucleotide to the first portion of the substrate. In various embodiments, the SBO can comprise a blocking construct, comprising a second oligonucleotide that is complementary to at least a portion of the first oligonucleotide and a second linker that connects the second oligonucleotide to the second portion of the cleavable substrate. In various embodiments, the method can comprise restricting movement of the blocking construct relative portion. In various embodiments, the partition can comprise a substrate barcoded oligonucleotide (SBO) construct, comprising a first oligonucleotide comprising a barcode sequence that identifies the cleavable substrate and a first linker that connects the first oligonucleotide to the first portion of the substrate. In various embodiments, the partition can comprise a blocking construct, comprising a second oligonucleotide that is complementary to at least a portion of the first oligonucleotide and a second linker that connects the second oligonucleotide to the second portion of the substrate.

Aspects of the disclosure can comprise a kit. In various embodiments, the kit can comprise a cleavable substrate comprising a first portion linked to a second portion. In various embodiments, the kit can comprise a substrate barcoded oligonucleotide (SBO) construct, comprising a first oligonucleotide comprising a barcode sequence that identifies the cleavable substrate and a first linker that connects the first oligonucleotide to the first portion of the substrate. In various embodiments, the kit can comprise a blocking construct, comprising a second oligonucleotide that is complementary to at least a portion of the first oligonucleotide and a second linker that connects the second oligonucleotide to the second portion of the substrate.

### BRIEF DESCRIPTION OF FIGURES

The novel features of the technology are set forth with particularity in the appended claims. A better understanding of the features and advantages of the present technology will be obtained by reference to the following detailed description that sets forth illustrative embodiments, in which the principles of the technology are utilized, and the accompanying drawings (also "Figure" and "FIG." herein). The accompanying drawings are not intended to be drawn to scale. Like reference numbers and designations in the various drawings indicate like elements. For purposes of clarity, not every component can be labeled in every drawing. In the drawings:
**FIG. 1** illustrates an iterative process for enzyme optimization according to various embodiments.
**FIG. 2** illustrates steps in a method for assessing enzymatic activity according to various embodiments.
**FIG. 3** is a schematic illustration showing an example of a microfluidic channel structure for partitioning individual analyte carriers according to various embodiments.
**FIG. 4** is a schematic illustration showing an example of a microfluidic channel structure for the controlled partitioning of beads into discrete droplets according to various embodiments.
**FIG. 5** illustrates an example of a barcode-carrying bead according to various embodiments.
**FIG. 6** illustrates another example of a barcode-carrying bead according to various embodiments.
**FIG. 7** is a schematic illustration of an example microwell array according to various embodiments.
**FIG. 8** is a schematic illustration of an example workflow for processing nucleic acid molecules according to various embodiments.
**FIG. 9** is a schematic illustration of example labelling agents with nucleic acid molecules attached thereto according to various embodiments.
**FIG. 10A** is a schematic illustration of example labelling agents according to various embodiments. **FIG. 10B** schematically shows another example workflow for processing nucleic acid molecules according to various embodiments. **FIG. 10C** schematically shows another example workflow for processing nucleic acid molecules according to various embodiments.
**FIG. 11** schematically shows another example of a barcode-carrying bead according to various embodiments.
**FIG. 12A** illustrates a composition in a reaction mixture for assessing enzymatic activity **1200** according to various embodiments.
**FIG. 12B** illustrates compositions in a reaction mixture for assessing enzymatic activity **1200** wherein a cleavable substrate **1202** has been cleaved **1202a, 1212b** and the first and second adapters **1222, 1224** as well as an enzyme **1226** are present and available for hybridization according to various embodiments.
**FIG. 12C** illustrates compositions in a reaction mixture for assessing enzymatic activity **1200** wherein a second adapter **1224** is hybridized to a second adapter binding site **1220** and an extension product **1230** has been generated according to various embodiments.
**FIG. 12D** illustrates compositions in a reaction mixture for assessing enzymatic activity **1200** wherein an extension product **1230** has been completed, thereby, generating a reverse complement of a substrate barcode sequence **1208rc** and a reverse complement of a first adapter sequence **1218rc** comprising a completed extension product resulting from an extension of a second adapter **1224** according to various embodiments.
**FIG. 12E** illustrates compositions in a reaction mixture for assessing enzymatic activity **1200** comprising a forward adapter **1231** that binds to a reverse complement of a first adapter sequence **1218rc** and a reverse adapter **1233** that binds to a second adapter **1220** according to various embodiments.
**FIG. 12F** illustrates compositions in a reaction mixture for assessing enzymatic activity **1200** comprising an extension product **1230** hybridized to a third oligonucleotide **1299** according to various embodiments.
**FIG. 12G** illustrates compositions in a reaction mixture for assessing enzymatic activity **1200** comprising a completed extension product **1230** coupled to a third oligonucleotide **1299** using a splint oligonucleotide **1297** according to various embodiments.
**FIG. 12H** illustrates compositions in a reaction mixture for assessing enzymatic activity **1200** comprising a completed extension product **1230** coupled to a third oligonucleotide **1299** using a splint oligonucleotide **1297** according to various embodiments.
**FIG. 12I** illustrates compositions in a reaction mixture for assessing enzymatic activity **1200** comprising a first oligonucleotide **1206** coupled to a third oligonucleotide using a splint oligonucleotide **1297.**
**FIG. 13** illustrates a partition (e.g., a droplet) comprising a bead comprising several oligonucleotides extending therefrom, where the oligonucleotides comprise a variety of different cleavable substrates.
**FIG. 14** illustrates steps in a method for assessing enzymatic activity according to various embodiments.
**FIG. 15** illustrates steps in a method for assessing enzymatic activity according to various embodiments.

It is to be understood that the figures are not necessarily drawn to scale, nor are the objects in the figures necessarily drawn to scale in relationship to one another. The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.

### DETAILED DESCRIPTION

This specification describes various exemplary embodiments of technologies for detecting and assessing enzymatic activity. More specifically, one or more enzymes can be modified to generate many different variants, e.g., by using CRISPR gene editing or other technologies and then the modified enzymes can be screened. Such technologies enable researchers and medical practitioners to optimize an enzymatic activity for a specific application. The disclosure, however, is not limited to these exemplary embodiments and applications or to the manner in which the exemplary embodiments and applications operate or are described herein. Moreover, the figures may show simplified or partial views, and the dimensions of elements in the figures may be exaggerated or otherwise not in proportion.

In addition, where reference is made to a list of elements (e.g., elements a, b, c), such reference is intended to include any one of the listed elements by itself, any combination of less than all of the listed elements, and/or a combination of all of the listed elements. Section divisions in the specification are for ease of review only and do not limit any combination of elements discussed.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

It should be understood that any uses of subheadings herein are for organizational purposes and should not be read to limit the application of those subheaded features to the various embodiments herein. Each and every feature described herein is applicable and usable in all the various embodiments discussed herein and that all features described herein can be used in any contemplated combination, regardless of the specific example embodiments that are described herein. It should further be noted that exemplary descriptions of specific features are used, largely for informational purposes, and not in any way to limit the design, subfeature, and functionality of the specifically described feature.

Unless otherwise defined, scientific and technical terms used in connection with the present teachings described herein shall have the meanings that are commonly understood by those of ordinary skill in the art. Further, unless otherwise required by context, singular terms shall include pluralities and plural terms shall include the singular. Generally, nomenclatures utilized in connection with, and techniques of, chemistry, biochemistry, molecular biology, pharmacology and toxicology are described herein are those available and commonly used in the art.

### Definitions:

The term "3' end blocker," "PCR blocker," or "PCR blocker oligonucleotides" may be used interchangeably and as used herein, generally refer to anything that can inhibit a polymerase during the elongation step of a polymerase chain reaction. In various embodiments disclosed herein, 3' end blockers lack a hydroxyl group at the 3' end required for extension in PCR. There are a variety of 3' end blockers known in the art, which may include, without limitation, 3' spacers, 3' phosphates, dideoxy cytidines (ddCs), and 3' inverted ends.

The terms "adapter(s)", "adapter(s)" and "tag(s)" may be used synonymously. An adapter or tag can be coupled to a polynucleotide sequence to be "tagged" by any approach, including ligation, hybridization, or other approaches.

Analytes can be derived from cells and can include at least a portion of a targeting genomic locus, a reverse complement thereof, or a derivative thereof. Analytes can include at least a portion of a tagging genomic locus, a reverse complement thereof, or a derivative thereof.

The term "analyte," as used herein, generally refers to a species of interest for detection. An analyte can be a biological analyte, such as a nucleic acid molecule or protein. An analyte can be an atom or molecule. An analyte can be a subunit of a larger unit, such as, e.g., a given sequence of a polynucleotide sequence or a sequence as part of a larger sequence. An analyte of the present disclosure includes a secreted analyte, a soluble analyte, and/or an extracellular analyte.

As used herein, the term "barcode," generally refers to a label, or identifier, that conveys or is capable of conveying information about an analyte. A barcode can be part of an analyte. A barcode can be independent of an analyte. A barcode can be a tag attached to an analyte (e.g., nucleic acid molecule) or a combination of the tag in addition to an endogenous characteristic of the analyte (e.g., size of the analyte or end sequence(s)). A barcode can be unique. Barcodes can have a variety of different formats. For example, barcodes can include: polynucleotide barcodes or barcode sequences; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads.

As used herein, the term "barcoded nucleic acid molecule" generally refers to a nucleic acid molecule that results from, for example, the processing of a nucleic acid barcode molecule with a nucleic acid sequence (e.g., nucleic acid sequence complementary to a nucleic acid primer sequence encompassed by the nucleic acid barcode molecule). The nucleic acid sequence can be a targeted sequence or a non-targeted sequence. The nucleic acid barcode molecule can be coupled to or attached to the nucleic acid molecule comprising the nucleic acid sequence. For example, in the methods and compositions described herein, hybridization and reverse transcription of a nucleic acid molecule (e.g., a messenger RNA (mRNA) molecule) of a cell with a nucleic acid barcode molecule (e.g., a nucleic acid barcode molecule containing a barcode sequence and a nucleic acid primer sequence complementary to a nucleic acid sequence of the mRNA molecule) results in a barcoded nucleic acid molecule that has a sequence corresponding to the nucleic acid sequence of the mRNA and the barcode sequence (or a reverse complement thereof). The processing of the nucleic acid molecule comprising the nucleic acid sequence, the nucleic acid barcode molecule, or both, can include a nucleic acid reaction, such as, in non-limiting examples, reverse transcription, nucleic acid extension, ligation, etc. The nucleic acid reaction can be performed prior to, during, or following barcoding of the nucleic acid sequence to generate the barcoded nucleic acid molecule. For example, the nucleic acid molecule comprising the nucleic acid sequence can be subjected to reverse transcription and then be attached to the nucleic acid barcode molecule to generate the barcoded nucleic acid molecule, or the nucleic acid molecule comprising the nucleic acid sequence can be attached to the nucleic acid barcode molecule and subjected to a nucleic acid reaction (e.g., extension, ligation) to generate the barcoded nucleic acid molecule. A barcoded nucleic acid molecule can serve as a template, such as a template polynucleotide, that can be further processed (e.g., amplified) and sequenced to obtain the target nucleic acid sequence. For example, in the methods and systems described herein, a barcoded nucleic acid molecule can be further processed (e.g., amplified) and sequenced to obtain the nucleic acid sequence of the nucleic acid molecule (e.g., mRNA).

The term "bead," as used herein, generally refers to a particle. The bead can be a solid or semi-solid particle. The bead can be a gel bead. The gel bead can include a polymer matrix (e.g., matrix formed by polymerization or cross-linking). The polymer matrix can include one or more polymers (e.g., polymers having different functional groups or repeat units). Polymers in the polymer matrix can be randomly arranged, such as in random copolymers, and/or have ordered structures, such as in block copolymers. Cross-linking can be via covalent, ionic, or inductive, interactions, or physical entanglement. The bead can be a macromolecule. The bead can be formed of nucleic acid molecules bound or hybridized together. The bead can be formed via covalent or non-covalent assembly of molecules (e.g., macromolecules), such as monomers or polymers. Such polymers or monomers can be natural or synthetic. Such polymers or monomers can be or include, for example, nucleic acid molecules (e.g., DNA or RNA). The bead can be formed of a polymeric material. The bead can be magnetic or non-magnetic. The bead can be rigid. The bead can be flexible and/or compressible. The bead can be disruptable or dissolvable. The bead can be a solid particle (e.g., a metal-based particle including but not limited to iron oxide, gold or silver) covered with a coating comprising one or more polymers. Such coating can be disruptable or dissolvable.

The term "bead specific barcode" as used herein, generally refers to a barcode that identifies a bead. In various embodiments, a bead specific barcode can comprise a unique molecular identifier (UMI).

The term "binding agent," as used herein generally refers to a molecule capable of binding to one or more other molecules (e.g., analytes, receptors, other binding agents, etc.) and that comprises one or more portions. In various cases, a binding agent comprises at least one, at least two, at least three, or at least four portions. Each portion can comprise a polypeptide. The polypeptide of a specific portion can be capable of binding one or more molecules. For example, a polypeptide of a specific portion can bind to a molecule located on a surface of a cell, such as a cell surface protein or receptor (e.g., a CD surface marker such as CD45). A polypeptide of another portion of a binding agent can bind a molecule that can be secreted from a cell (e.g., T cell, B-cell, dendritic cell, etc.). The one or more portions of a binding agent can be directly or indirectly linked to, conjugated to, or fused to one another. For example, a first portion of a binding agent can be directly or indirectly linked to, conjugated to, or fused to a second portion of the binding agent. Moreover, the terms "binding agent," "polypeptide," and "antibody" can be used interchangeably herein.

The term "biological particle," as used herein, generally refers to a discrete biological system derived from a biological sample. The biological particle can be a macromolecule. The biological particle can be a small molecule. The biological particle can be a virus. The biological particle can be a cell or derivative of a cell. The biological particle can be an organelle. The biological particle can be a cell nucleus. The biological particle can be a rare cell from a population of cells. The biological particle can be any type of cell, including without limitation prokaryotic cells, eukaryotic cells, bacterial, fungal, plant, mammalian, or other animal cell type, mycoplasmas, normal tissue cells, tumor cells, or any other cell type, whether derived from single cell or multicellular organisms. The biological particle can be a constituent of a cell. The biological particle can be or can include DNA, RNA, organelles, proteins, or any combination thereof. The biological particle can be or can include a matrix (e.g., a gel or polymer matrix) comprising a cell or one or more constituents from a cell (e.g., cell bead), such as DNA, RNA, organelles, proteins, or any combination thereof, from the cell. The biological particle can be obtained from a tissue of a subject. The biological particle can be a hardened cell. Such hardened cell may or may not include a cell wall or cell membrane. The biological particle can include one or more constituents of a cell, but may not include other constituents of the cell. An example of such constituents is a nucleus or an organelle. A cell may be a live cell. The live cell can be capable of being cultured, for example, being cultured when enclosed in a gel or polymer matrix, or cultured when comprising a gel or polymer matrix.

As used herein, the term "blocking construct" means an oligonucleotide that is unable to act as a primer for a polymerase. In various embodiments, a 3' end can be non-reactive and can comprise a 3' end blocker. In various embodiments a "blocking construct" can comprise an oligonucleotide connected to a linker.

The term "cell bead," as used herein, generally refers to a hydrogel, polymeric, or crosslinked material that comprises (e.g., encapsulates, contains, etc.) a biological particle (e.g., a cell, a nucleus, a fixed cell, a cross-linked cell), a virus, components of or macromolecular constituents of or derived from a cell or virus. For example, a cell bead can comprise a virus and/or a cell. In various cases, a cell bead comprises a single cell. In various cases, a cell bead can comprise multiple cells adhered together. A cell bead can include any type of cell, including without limitation prokaryotic cells, eukaryotic cells, bacterial, fungal, plant, mammalian, or other animal cell types, mycoplasmas, normal tissue cells, tumor cells, immune cells, e.g., a T-cell (e.g., CD4 T-cell, CD4 T-cell that comprises a dormant copy of human immunodeficiency virus (HIV)), a B cell, or a dendritic cell, a fixed cell, a cross-linked cell, a rare cell from a population of cells, or any other cell type, whether derived from single cell or multicellular organisms. Furthermore, a cell bead can comprise a live cell, such as, for example, a cell can be capable of being cultured. Moreover, in various examples, a cell bead can comprise a derivative of a cell, such as one or more components of the cell (e.g., an organelle, a cell protein, a cellular nucleic acid, genomic nucleic acid, messenger ribonucleic acid, a ribosome, a cellular enzyme, etc.). In various examples, a cell bead can comprise material obtained from a biological tissue, such as, for example, obtained from a subject. In various cases, cells, viruses or macromolecular constituents thereof are encapsulated within a cell bead. Encapsulation can be within a polymer or gel matrix that forms a structural component of the cell bead. In various cases, a cell bead is generated by fixing a cell in a fixation medium or by cross-linking elements of the cell, such as the cell membrane, the cell cytoskeleton, etc.

As used herein, the terms "cleavable substrate" and "substrate" are used interchangeably herein and mean any substrate that can be subject to enzymatic activity. In various embodiments, "cleavable" means an enzyme can act to split a substrate into two or more parts that are separate from one another. Cleavable substrates can comprise proteins, carbohydrates, lipids, or nucleotides (e.g., double stranded polynucleotide sequences). Cleavable substrates can comprise naturally occurring compounds as well as synthetic organic compounds.

As used herein, the terms "comprise", "comprises", "comprising", "contain", "contains", "containing", "have", "having" "include", "includes", and "including" and their variants are not intended to be limiting, are inclusive or open-ended and do not exclude additional, unrecited additives, components, integers, elements or method steps. For example, a process, method, system, composition, kit, or apparatus that comprises a list of features is not necessarily limited only to those features but may include other features not expressly listed or inherent to such process, method, system, composition, kit, or apparatus.

The terms "coupled," "linked," "conjugated," "associated," "attached," "connected" or "fused," as used herein, may be used interchangeably herein and generally refer to one molecule (e.g., polypeptide, receptor, analyte, etc.) being attached or connected (e.g., chemically bound) to another molecule (e.g., polypeptide, receptor, analyte, etc.). In various embodiments described herein, linked comprises covalently linked or non-covalently linked.

The term "covalent" as used herein means a chemical bond involving the sharing of at least one electron pair. Accordingly, two molecules are "covalently linked" or "covalently attached" to one another when at least one atom in the first molecule shares at least one electron pair with at least one atom in the second molecule. In some circumstances, a covalent linkage between two molecules can involve one or more intermediary molecules, for example, where a first molecule and a second molecule are each covalently linked to a linker molecule. In such a circumstance, all three molecules (the first molecule, the second molecule, and the linker molecule) are covalently linked to one another. As used herein, the term "deoxyribonucleic acid" or "DNA" means a chain of nucleotides consisting of 4 types of nucleotides; A (adenine), T (thymine), C (cytosine), and G (guanine), and that RNA (ribonucleic acid) is comprised of 4 types of nucleotides; A, U (uracil), G, and C. Certain pairs of nucleotides specifically bind to one another in a complementary fashion (called complementary base pairing). That is, adenine (A) pairs with thymine (T) (in the case of RNA, however, adenine (A) pairs with uracil (U)), and cytosine (C) pairs with guanine (G). When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "nucleic acid sequence," "genomic sequence," "genetic sequence," or "fragment sequence," or "nucleic acid sequencing read" denotes any information or data that is indicative of the order of the nucleotide bases (e.g., adenine, guanine, cytosine, and thymine/uracil) in a molecule (e.g., whole genome, whole transcriptome, exome, oligonucleotide, polynucleotide, fragment, etc.) of DNA or RNA. It should be understood that the present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, electronic signature-based systems, etc.

As used herein, the term "inert polymer" means a polymer that is non-reactive to enzymatic activity. Inert polymers may be resistant to oxidation or reduction. Non-limiting examples of inert polymers include polyethylene glycol (PEG), Poly(N-vinylpyrrolidone) (PVP), Polyglycerol (PG), Poly(N-(2-hydroxypropyl) methacrylamide) (PHPMA), or Polyoxazolines (POZs).

The term "insert oligonucleotide," as used herein, generally means a nucleotide molecule including a targeting or tagging sequence with two flanking sequences on either side of the tagging sequence. In various aspects, insert oligonucleotides are configured to be inserted into a targeting genomic locus or a tagging genomic locus using a complementary sequence of the targeting or tagging sequences to the targeting or tagging genomic loci. In various embodiments, an insert oligonucleotide can comprise a tagging barcode.

As used herein, the term "linker" may be used interchangeably with the term "spacer" and means an inert polymer that can hold two or more molecules together. In various embodiments, linkers may act to distance two molecules away from one another. In various embodiments, a linker length may be selected for a specified application. In various embodiments, linkers may comprise polyethylene glycol (PEG), Poly(N-vinylpyrrolidone) (PVP), Polyglycerol (PG), Poly(N-(2-hydroxypropyl) methacrylamide) (PHPMA), Polyoxazolines (POZs), or any other known or useful non-reactive molecule able to connect two or one other molecules together. As used herein, linkers may be used to connect oligonucleotides to cleavable substrates.

As used herein, the term "locked nucleic acid (LNA)" also known as "bridged nucleic acid (BNA) and often referred to as inaccessible RNA means an inaccessible nucleotide such as RNA. In various embodiments, LNAs comprise a modified RNA nucleotide in which the ribose moiety is modified with an extra bridge connecting the 2' oxygen and 4' carbon.

The term "macromolecular constituent," as used herein, generally refers to a macromolecule contained within or from a biological particle. The macromolecular constituent may comprise a nucleic acid. In various cases, the biological particle may be a macromolecule. The macromolecular constituent may comprise DNA. The macromolecular constituent may comprise RNA. The RNA may be coding or non-coding. The RNA may be messenger RNA (mRNA), ribosomal RNA (rRNA) or transfer RNA (tRNA), for example. The RNA may be a transcript. The RNA may be small RNA that are less than 200 nucleic acid bases in length, or large RNA that are greater than 200 nucleic acid bases in length. Small RNAs may include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA may be double-stranded RNA or single-stranded RNA. The RNA may be circular RNA. The macromolecular constituent may comprise a protein. The macromolecular constituent may comprise a peptide. The macromolecular constituent may comprise a polypeptide.

The term "molecular tag," as used herein, generally refers to a molecule capable of binding to a macromolecular constituent. The molecular tag may bind to the macromolecular constituent with high affinity. The molecular tag may bind to the macromolecular constituent with high specificity. The molecular tag may comprise a nucleotide sequence. The molecular tag may comprise a nucleic acid sequence. The nucleic acid sequence may be at least a portion or an entirety of the molecular tag. The molecular tag may be a nucleic acid molecule or may be part of a nucleic acid molecule. The molecular tag may be an oligonucleotide or a polypeptide. The molecular tag may comprise a DNA aptamer. The molecular tag may be or comprise a primer. The molecular tag may be, or comprise, a protein. The molecular tag may comprise a polypeptide. The molecular tag may be a barcode.

As used herein, the term "ones" means more than one.

The term "partition," as used herein, generally, refers to a space or volume that may be suitable to contain one or more species or conduct one or more reactions. A partition can be a volume or subvolume wherein diffusion of contents beyond the volume or sub-volume is inhibited. For example, the partitions can include a porous matrix that is capable of entraining and/or retaining materials within its matrix.

A partition may be a physical compartment, such as a droplet or well. The partition may isolate space or volume from another space or volume. The droplet may be a first phase (e.g., aqueous phase) in a second phase (e.g., oil) immiscible with the first phase. The droplet may be a first phase in a second phase that does not phase separate from the first phase, such as, for example, a capsule or liposome in an aqueous phase. A partition may comprise one or more other (inner) partitions. In various cases, a partition may be a virtual compartment that can be defined and identified by an index (e.g., indexed libraries) across multiple and/or remote physical compartments. For example, a physical compartment may comprise a plurality of virtual compartments.

As used herein, the term "plurality" can be 2, 3, 4, 5, 6, 7, 8, 9, 10, or more.

A "polynucleotide", "nucleic acid", or "oligonucleotide" refers to a linear polymer of nucleotides (including deoxyribonucleotides, ribonucleotides, or analogs thereof) joined by internucleotidic linkages. Typically, a polynucleotide comprises at least three nucleotides. Usually oligonucleotides range in size from a few monomeric units, e.g. 3-4, to several hundreds of monomeric units. Whenever a polynucleotide such as an oligonucleotide is represented by a sequence of letters, such as "ATGCCTG," it will be understood that the nucleotides are in 5'->3' order from left to right and that "A" denotes deoxyadenosine, "C" denotes deoxycytidine, "G" denotes deoxyguanosine, and "T" denotes thymidine, unless otherwise noted. The letters A, C, G, and T may be used to refer to the bases themselves, to nucleosides, or to nucleotides comprising the bases, as is standard in the art.

The phrase "restricting movement," as used herein generally refers to inhibiting movement of two or more molecules relative to one another. More specifically, restricting movement comprises inhibiting movement and/or maintaining proximity of two oligonucleotides relative to one another at all times and particularly during a PCR amplification reaction and even more particularly during the denaturation step. In various embodiments described herein, restricting movement increases the likelihood that two specific oligonucleotides remain hybridized, anneal readily, and/or have a high or increased affinity for one another. In various embodiments, restricting movement of an SBO and a blocking construct prevents an adapter (e.g., a primer) from binding to the SBO construct and completing a PCR reaction. The two oligonucleotides described, whose movement is restricted with respect to one another, may comprise one or more locked nucleic acids (LNAs) which may create a more rigid structure and, therefore, increase their affinity for one another. Further, the presence of one or more LNAs in at least one strand of a double-stranded DNA sequence may serve to reduce breathing between the two complementary or partially complementary DNA sequences.

The term "sample," as used herein, generally refers to a biological sample of a subject. The biological sample may comprise any number of macromolecules, for example, cellular macromolecules. The sample may be a cell sample. The sample may be a cell line or cell culture sample. The sample can include one or more cells. The sample can include one or more microbes. The biological sample may be a nucleic acid sample or protein sample. The biological sample may also be a carbohydrate sample or a lipid sample. The biological sample may be derived from another sample. The sample may be a tissue sample, such as a biopsy, core biopsy, needle aspirate, or fine needle aspirate. The sample may be a fluid sample, such as a blood sample, urine sample, or saliva sample. The sample may be a skin sample. The sample may be a cheek swab. The sample may be a plasma or serum sample. The sample may be a cell-free or cell free sample. A cell-free sample may include extracellular polynucleotides. Extracellular polynucleotides may be isolated from a bodily sample that may be selected from the group consisting of blood, plasma, serum, urine, saliva, mucosal excretions, sputum, stool and tears.

The term "sequencing," as used herein, generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (e.g., single stranded DNA). Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). Alternatively, or in addition, sequencing may be performed using nucleic acid amplification, polymerase chain reaction (PCR) (e.g., digital PCR, quantitative PCR, or real time PCR), or isothermal amplification. Such systems may provide a plurality of raw genetic data corresponding to the genetic information of a subject (e.g., human), as generated by the systems from a sample provided by the subject. In various examples, such systems provide sequencing reads (also "reads" herein). A read may include a string of nucleic acid bases corresponding to a sequence of a nucleic acid molecule that has been sequenced. In various situations, systems and methods provided herein may be used with proteomic information.

As used herein, the term "substantially" means sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. When used with respect to numerical values or parameters or characteristics that can be expressed as numerical values, "substantially" means within ten percent.

The term "subject," as used herein, generally refers to an animal, such as a mammal (e.g., human) or avian (e.g., bird), or other organism, such as a plant. For example, the subject can be a vertebrate, a mammal, a rodent (e.g., a mouse), a primate, a simian or a human. Animals may include, but are not limited to, farm animals, sport animals, and pets. A subject can be a healthy or asymptomatic individual, an individual that has or is suspected of having a disease (e.g., cancer) or a pre-disposition to the disease, and/or an individual that is in need of therapy or suspected of needing therapy. A subject can be a patient. A subject can be a microorganism or microbe (e.g., bacteria, fungi, archaea, viruses). The term "non-human animals" includes all vertebrates, e.g., mammals, e.g., rodents, e.g., mice, non-human primates, and other mammals, such as e.g., sheep, dogs, cows, chickens, and non-mammals, such as amphibians, reptiles, etc.

As used herein, the term "substrate barcoded oligonucleotide" (SBO) construct means an oligonucleotide comprising first and second adapter binding sites on adjacent and opposing sides of a barcode sequence. In various embodiments, SBO constructs may comprise linkers having an end attached to a 3' end of an adapter binding site of the oligonucleotide and a cleavable substrate attached to an opposing end of the linker, thereby connecting the oligonucleotide to the cleavable substrate.

As used herein, the term "composition for assessing enzymatic activity" is interchangeable with or comprises a "substrate complex." As used herein, a substrate complex may comprise an SBO construct and a blocking construct attached to a cleavable substrate with linker molecules.

The term "transfection reagent," as used herein, generally means any reagent that increases the likelihood of a successful transfection. In various embodiments, a transfection agent can be lipid-based and configured to improve a transfection rate. Non-limiting examples of transfection reagents are lipofectamine and Mirus TransiT. Generally, in liposomal transfection, the positive surface charge of the liposomes mediates the interaction of the nucleic acid and the cell membrane, allowing for fusion of the liposome/nucleic acid transfection complex with the negatively charged cell membrane. The transfection complex can enter the cell through endocytosis.

The term "substrate barcode sequence," as used herein, generally refers to a barcode that identifies a cleavable substrate.

Unless otherwise defined, scientific and technical terms used in connection with the present teachings described herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those available and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. The techniques and procedures described herein are generally performed according to conventional methods well known in the art and as described in various general and more specific references that are cited and discussed throughout the instant specification. See, e.g., Sambrook et al., Molecular Cloning: A Laboratory Manual (Third ed., Cold Spring Harbor Laboratory Press, Cold Spring Harbor, N.Y. 2000). The nomenclatures utilized in connection with, and the laboratory procedures and techniques described herein are those available and commonly used in the art.

### I. OVERVIEW:

Expression or introduction of gene modification systems (e.g., CRISPR/Cas machinery) may be used to edit the genomes of cells and particularly target genes of interest. Such gene modification system (e.g., CRISPR/Cas systems) facilitated gene editing may allow for modification of a resulting polypeptide sequence encoding one or more enzymes to generate many enzyme variants having different enzymatic activity levels. Detecting and assessing (e.g., quantifying) the enzymatic activity level of the enzyme variants may allow researchers and medical practitioners to screen and select enzyme variants suitable for specific uses or applications based on their known, modified enzymatic activity level. Unfortunately, currently existing enzymatic activity assays are limited to bulk assays inhibited by low-throughput and require large quantities of raw input material, making them costly and inefficient. The technologies described herein solve this problem and many others. For example, the technologies and workflows described herein facilitate screening of large numbers of enzymatic variants by detecting the specific variant and assessing activity level of the enzyme variants at a single cell level, which has never been successfully accomplished until now. The technologies described herein also provide techniques for assessing enzymatic reactions (e.g., the quantity of product produced, or substrate consumed) either in bulk or at a single cell level. One non-limiting example of a biological endpoint assay provided herein comprises systems and methods for comparing treatment conditions versus control conditions. Additionally, the technologies described herein can comprises an interactive process enabling researchers to further optimize the previously modified enzyme variants and further fine-tune enzymatic activity and function.

### Enzymatic optimization systems and methods:

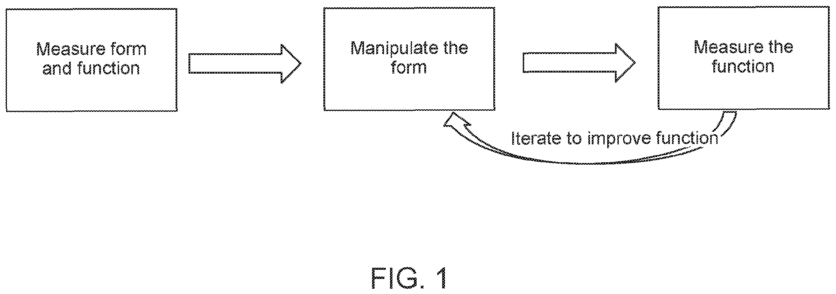

**FIG. 1** illustrates an iterative process for optimizing enzyme activity according to various embodiments. In various embodiments, an enzymatic function or activity may be assessed using the technologies disclosed herein. In various embodiments, measuring an enzymatic activity may comprise measuring, in units, a rate of reaction catalyzed by an enzyme. In various embodiments, the units may comprise micromoles of a substrate transformed per minute. In various embodiments, aspects of an enzymatic activity assay may need to be kept constant, such as pH, temperature, enzyme concentration, substrate concentration, etcetera to compare different variants of an enzyme. Additionally, some enzymes operate optimally under different environmental conditions, so the assays disclosed herein may be customized to suit specific enzymes.

In various embodiments, after a base or original enzyme has been evaluated for enzymatic activity, the enzyme form may be manipulated, e.g., using CRISPR editing technologies to edit the gene encoding the enzyme, thereby modifying an enzymatic activity (e.g., by modifying the amino acid sequence of the enzyme). In various embodiments, many variants may be generated and then screened for enzymatic activity and identified.

In various embodiments, a set of enzymatic assessment (e.g., quantification) results from identified variants may be compared. In various embodiments, one or more genes encoding the enzyme variants may be further edited or manipulated, e.g., using CRISPR editing technologies to generate another iteration of enzyme variants, where an enzymatic activity and each of those variants may have their enzymatic activity quantified using the technologies disclosed herein. In various embodiments, this iterative process may be performed 1, 2, 3, 4, 5, 6, 7, 8, 9, 10 or any number of times to optimize an enzyme's activity for a specific application or purpose. Purposes can include research, industrial, or therapeutic activities. For example, an enzyme's activity may be increased to produce a commercial product at an increased rate for a lower cost.

### II. REACTION MIXTURES AND COMPOSITIONS:

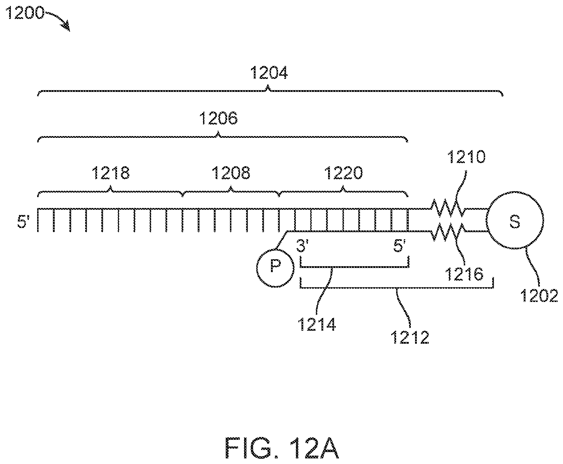

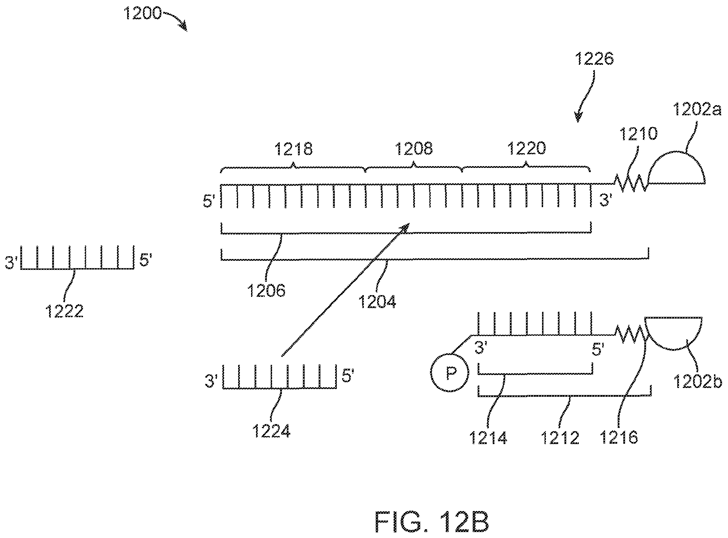

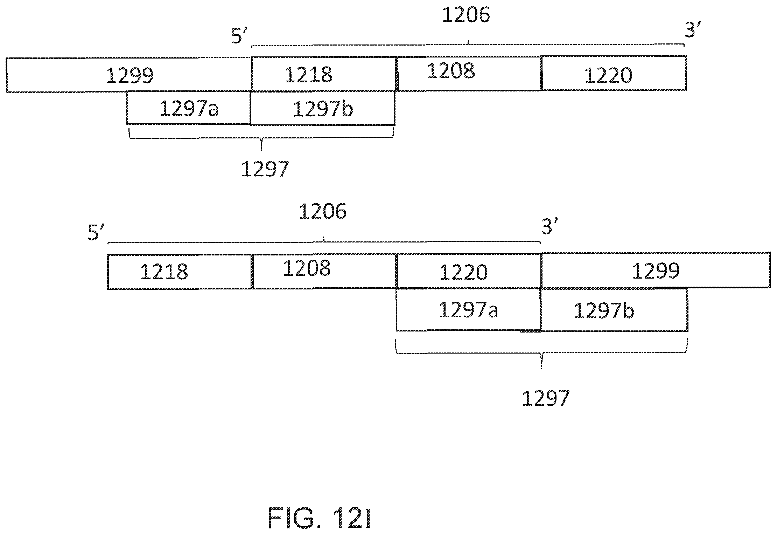

**FIG. 12A** and **12B** illustrate compositions that can be included in a reaction mixture for assessing enzymatic activity **1200** according to various embodiments. In various embodiments, a composition for assessing enzymatic activity **1200** can comprise a substrate barcoded oligonucleotide (SBO) construct **1204** hybridized to a blocking construct **1212 (****FIG. 12A****).** In various embodiments, the composition can further comprise a cleavable substrate **1202** and linkers **1210, 1216.** In various embodiments, the SBO construct **1204** can be coupled to the cleavable substrate **1202** with a linker **1210.** In various embodiments, the blocking construct **1212** can be coupled to the cleavable substrate **1202** with a linker **1216.**

Aspects of the disclosure can comprise an SBO construct **1204** comprising a first oligonucleotide **1206** and a linker **1210.** In various embodiments, the first oligonucleotide can comprise a barcode sequence **1208** having adaptors **1218, 1220** on adjacent and opposing sides of the barcode sequence **1208.**

Aspects of the disclosure can comprise a blocking construct **1212** comprising a second oligonucleotide **1214** and a linker **1216.**

In various embodiments, a reaction mixture for assessing enzymatic activity **1200** can comprise a first composition comprising a substrate barcoded nucleotide (SBO) construct **1204** and a second composition comprising a blocking construct **1212 (****FIG. 12B****).** In various embodiments, the first composition can further comprise any combination of the following components: a cleaved substrate **1202a,** a linker **1210,** adapters **1222, 1224,** and an amplification enzyme **1226.** In various embodiments, the second composition can further comprise any combination of the following components: a cleaved substrate **1202b** or a linker **1216.**

In various embodiments, the amplification enzyme can comprise any molecule having catalytic nucleotide extension properties, including, but not limited to, transcriptases and polymerases. In various embodiments, the amplification enzyme can comprise a reverse transcriptase. In various embodiments, the amplification enzyme can comprise a DNA polymerase. In various embodiments, the amplification enzyme can comprise an RNA polymerase.

In various embodiments, a substrate barcoded oligonucleotide (SBO) construct **1204** can comprise a first oligonucleotide **1206** comprising a barcode sequence **1208** having a first adapter sequence (or reverse complement thereof) **1218** and a second adapter binding site **1220** adjacent to and on opposing sides and a first linker **1210** linked or covalently linked to a first portion of a cleavable substrate **1202a.** In various embodiments the first linker **1210** can be attached to the 3' end of the first oligonucleotide **1206.**

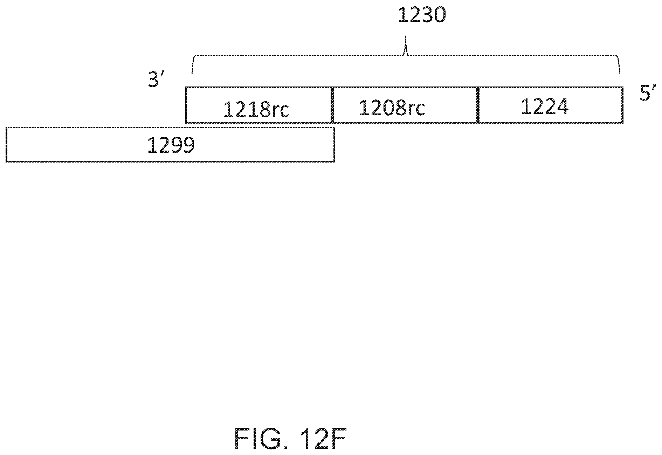

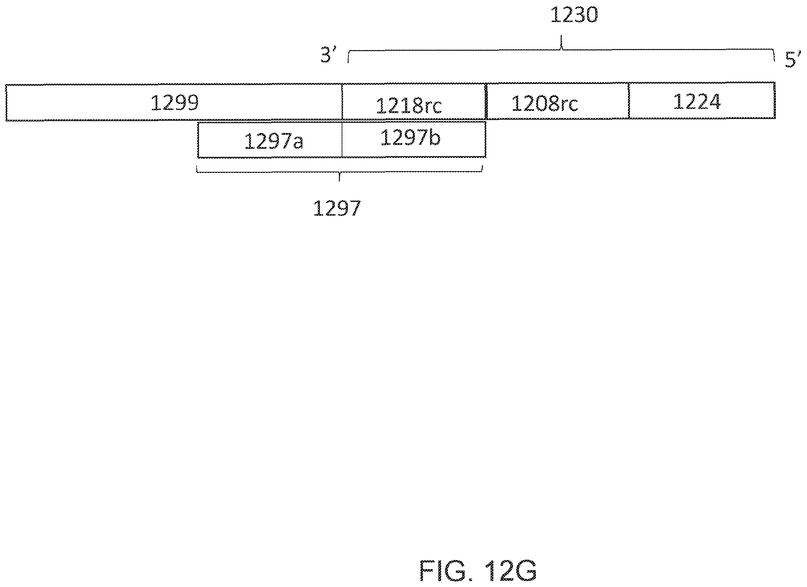

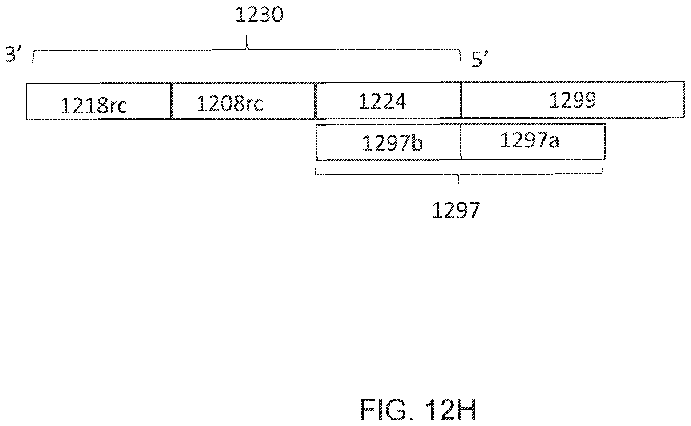

Adapter sequences in accordance with embodiments of the invention can include, e.g., primer binding sites, sequences complementary to a capture sequence, sequences complementary to a portion of a splint oligonucleotide, or any combination thereof. In various embodiments, a first adapter sequence **1218** can comprise a first adapter binding site **1218** as described herein. In various embodiments, a first adapter sequence can comprise a capture sequence. In various embodiments, the capture sequence can be complementary to the third oligonucleotide **1299, 1308** as described herein. In various embodiments, the third oligonucleotide **1299, 1308** can be a gel bead oligonucleotide as described herein. In various embodiments, a first adapter sequence **1218** can comprise a sequence complementary to at least a portion of a splint oligonucleotide **1297, 1297a, 1297b** (see **FIGS. 12F****,** **12G****,** **12H****)** as described herein. An adapter can comprise a functional sequence, e.g., a functional sequence described herein.

In various embodiments, the compositions in a reaction mixture for assessing enzymatic activity **1200** can comprise a blocking construct **1212** comprising a second oligonucleotide **1214** and a second linker **1216** that can connect the second oligonucleotide **1214** to the second portion of the cleavable substrate **1202b.**

In various embodiments, the first oligonucleotide **1206** can comprise a first adapter binding site **1218** adjacent to a 5' end of the barcode sequence. In various embodiments, the SBO construct **1204** can comprise a second adapter binding site **1220** adjacent to a 3' end of the barcode sequence. In various embodiments, a plurality of first adapters **1222** comprising a sequence that is complementary to at least a portion of the first adapter binding site can be added to the compositions. In various embodiments, a plurality of second adapters **1224** comprising a sequence that can be complementary to at least a portion of the second adapter binding site **1220** can be added to the compositions. In various embodiments, one or more of amplification enzymes **1226** is used in an amplification reaction to generate a reverse complement of the barcode sequence by extending the second adapter **1224.** In some embodiments, the one or more amplification enzymes comprises a polymerase. In some embodiments, the one or more amplification enzymes comprises a reverse transcriptase.

Amplification reactions in accordance with embodiments of the invention can include, e.g., methods to extend adapters/primers, polymerase chain reaction (PCR), reverse transcription or any combination thereof. In various embodiments, an amplification reaction can comprise PCR. In various embodiments, an amplification reaction can comprise reverse transcription.

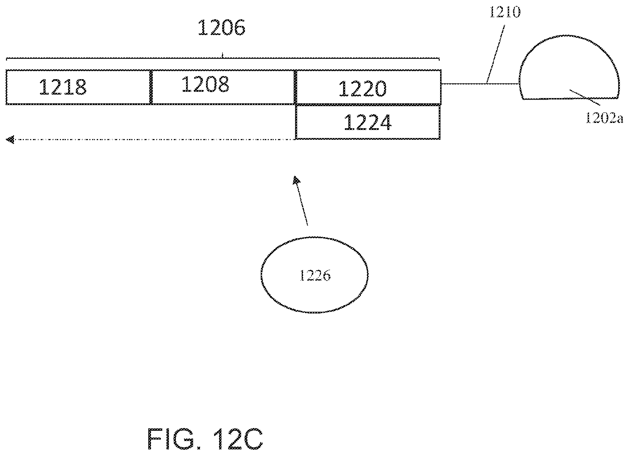

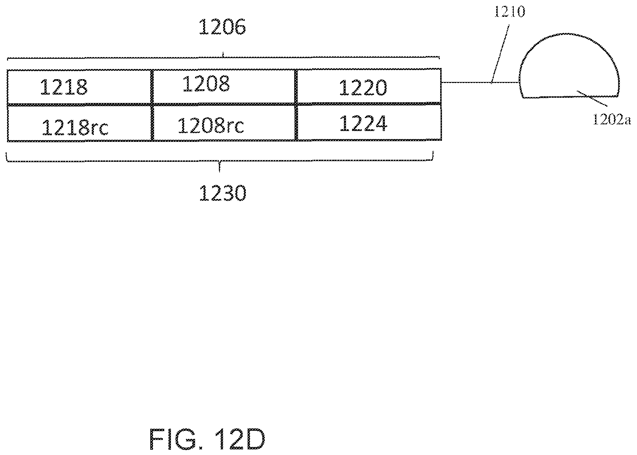

**FIG. 12C** illustrates exemplary compositions that can be included in a reaction mixture for assessing enzymatic activity **1200** comprising a first oligonucleotide **1206** hybridized to a second adapter **1224** (e.g., a primer molecule) via second adapter binding site **1220** and an amplification enzyme **1226.** In some embodiments, the amplification enzyme **1226** is a reverse transcriptase. In some embodiments, the amplification enzyme **1226** (e.g., reverse transcriptase) carries out an extension reaction to generate an extension product 1230 **(****FIG. 12D****).**

Extension products in accordance with embodiments of the invention can include, e.g., nucleotide sequences, ssDNA molecules, dsDNA molecules, RNA molecules, or any combination thereof.

In various embodiments, the extension product **1230** comprises a second adapter **1224,** a reverse complement of a substrate barcode sequence **1208rc,** and a reverse complement of a first adapter sequence **1218rc (****FIG. 12D****).**

**FIG. 12D** illustrates a non-limiting example of an extension product **1230** resulting from the extension of the second adapter in **FIG. 12C** according to various embodiments. In various embodiments, an extension product **1230** can be generated using reverse transcription, thereby generating a reverse complement of the first oligonucleotide **1206,** which can comprise a substrate barcode sequence **1208rc** and a reverse complement of a first adapter sequence **1218rc** comprising a completed extension product resulting from an extension of a second adapter **1224** (e.g., a second adapter) according to various embodiments. In various embodiments, the first oligonucleotide **1206** can be coupled to a cleaved substrate **1202a** through a linker **1210.**

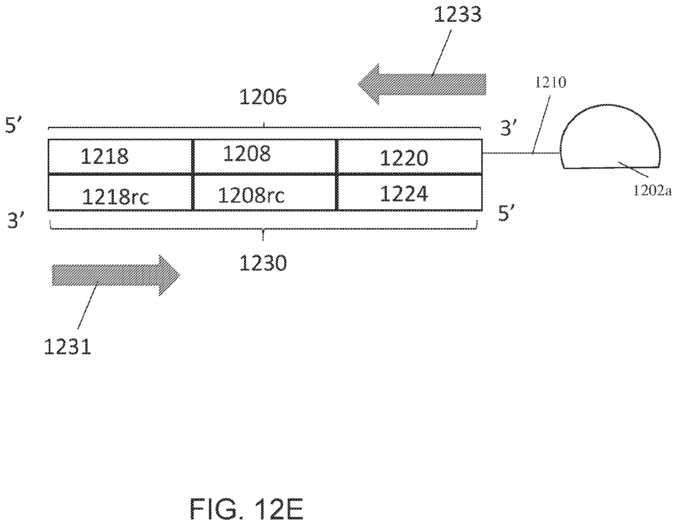

**FIG. 12E** illustrates compositions that can be included in a reaction mixture for assessing enzymatic activity **1200** for amplifying the first oligonucleotide **1206** and the extension product **1230** shown in **FIG. 12D****.** In various embodiments, the reaction mixture comprises a forward adapter **1231** (e.g., a forward primer) that can hybridize to a reverse complement of a first adapter sequence **1218rc** and a reverse adapter **1233** (e.g., a reverse primer) that can hybridize to a second adapter binding site **1220** according to various embodiments. In various embodiments, a reaction mixture can comprise a forward primer that can hybridize to a portion of the extension product and a reverse primer that can hybridize to the extension product. In various embodiments, a reaction mixture can comprise a forward primer that can hybridize to the first oligonucleotide and reverse primer that can hybridize to the first oligonucleotide. In various embodiments, the reaction mixture can comprise adaptors (e.g. primers) for amplifying any particular region of interest in any combination. In various embodiments, the reaction mixture can comprise an amplification enzyme **1226** (not shown) for carrying out an amplification reaction (e.g., a polymerase). In various embodiments, the resulting amplification reaction can enable downstream assessment of enzymatic activity using the resulting, amplified first oligonucleotides **1206** and the extension products **1230.** In various embodiments, the first oligonucleotide **1206** may be coupled to a cleaved substrate **1202a** through a linker **1210.**

In various embodiments, the compositions can comprise a cleavable substrate **1202** that can be selected for based on a specific enzyme under investigation. Specifically, cleavable substrates can be substrates that can be reacted with or cleaved by the enzyme under investigation. In various embodiments, the enzyme under investigation can cleave the cleavable substrate **1202** such that a blocking construct **1212** can be released from the SBO construct. In various embodiments, cleaving the cleavable substrate **1202** can reduce the affinity of the blocking construct **1212** to the SBO construct **1204.** In various embodiments, the cleavable substrate **1202** can comprise a protein, carbohydrate, double stranded nucleotide, or a synthetic compound.

In various embodiments, the first and second linkers **1210, 1212** can comprise an inert polymer. In various embodiments, the inert polymer can comprise polyethylene glycol (PEG). In various embodiments, the inert polymer can be a compound resistant to oxidation or reduction.

In various embodiments, the second oligonucleotide **1214** can comprise at least one locked nucleic acid (LNA) for increasing the affinity between the first oligonucleotide **1206** and the second oligonucleotide **1214** relative to when no LNAs are present. In various embodiments... relative to other adaptors, other sequences present, etc. In various embodiments, the second oligonucleotide **1214** can comprise 1, 2, 3, 4, 5, 6, 7, 8, 9, 10, 11, 12, 13, 14, 15, 16, 17, 18, 19, 20, or any number of LNAs. In various embodiments, the entire second oligonucleotide is comprised of LNAs. In various embodiments 5%, 10%, 15%, 20%, 25%, 30% 35%, 40%, 45%, 50%, 55%, 60%, 65%, 70%, 75%, 80%, 85%, 90%, 95%, or 100% of the nucleotides within the second oligonucleotide **1214** can comprise LNAs or include a range between any of these percentages. In various embodiments, the second oligonucleotide **1214** comprises no LNAs.

In various embodiments, the first oligonucleotide **1206** can comprise at least one locked nucleic acid (LNA) for increasing the affinity between the first oligonucleotide **1206** and the second oligonucleotide **1214.** In various embodiments, the first oligonucleotide **1206** can comprise 1, 2, 3, 4, 5, 6, 7, 8, 9, 10, 11, 12, 13, 14, 15, 16, 17, 18, 19, 20, or any number of LNAs. In various embodiments, the entire first oligonucleotide **1206** is comprised of LNAs. In various embodiments 5%, 10%, 15%, 20%, 25%, 30% 35%, 40%, 45%, 50%, 55%, 60%, 65%, 70%, 75%, 80%, 85%, 90%, 95%, or 100% of the nucleotides within the first oligonucleotide **1206** can comprise LNAs or include a range between any of these percentages. In various embodiments, the first oligonucleotide **1206** comprises no LNAs.

In various embodiments, a portion of the first oligonucleotide **1206** can be complementary to the second oligonucleotide **1214.** In various embodiments, when the first oligonucleotide **1206** is hybridized to the second oligonucleotide, an amplification reaction is prevented from occurring (e.g., an amplification reaction using an amplification enzyme **1226** to extend second adapter **1224** [e.g., a second primer]). In various embodiments, the second oligonucleotide **1214** of the blocking construct **1212** competes with a second adapter **1224** for hybridization with the first oligonucleotide **1206** of the SBO construct **1204.**

In various embodiments, the second oligonucleotide **1214** comprises a non-reactive 3' end or 3' end blocker, thereby preventing amplification or polymerization of a reverse complement of the first oligonucleotide **1206** of the SBO construct **1204.** In various embodiments, amplification of the first oligonucleotide **1206** can be prevented by a hybridized second oligonucleotide **1214** of a blocking construct **1212** comprising a non-reactive 3' end. In various embodiments, the 3' end can comprise a phosphorylated 3' end.

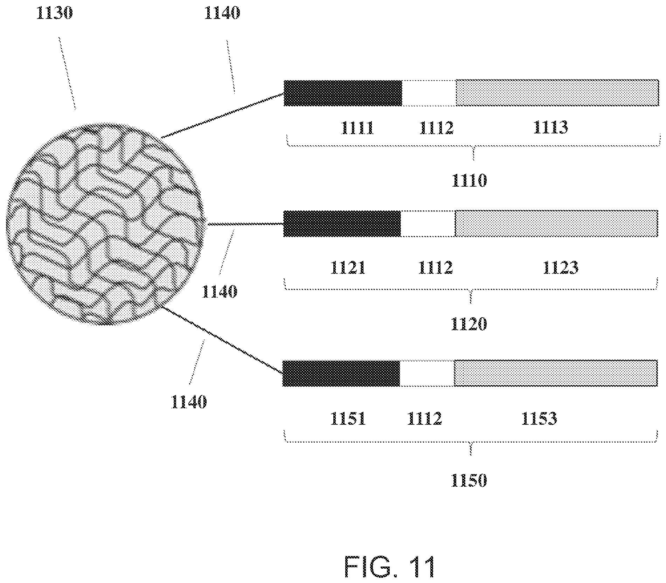

Now, additionally, referring to **FIGS. 11****,** **12F****,** and **13****,** compositions in a reaction mixture for assessing enzymatic activity can comprise a bead **1130** coupled to a third oligonucleotide **1299, 1308.** In various embodiments, the third oligonucleotide **1299, 1308** can be or comprise a nucleic acid barcode molecule (e.g., **1110, 1120, 1150)** comprising a barcode sequence **1112** and a capture sequence (e.g., **1113, 1123, 1153).** In some embodiments, barcode sequence **1112** is a partition-specific barcode sequence. In some cases, additionally to the barcode sequence **1112,** the nucleic acid barcode molecule comprises a UMI sequence. In some cases, alternatively to the barcode sequence **1112,** the nucleic acid barcode molecule comprises a UMI sequence. In some cases, the nucleic acid barcode molecule (e.g., **1110, 1120, 1150)** comprises an adapter sequence (e.g., **1111, 1121, 1151).**

**FIG. 12F** illustrates compositions in a reaction mixture for assessing enzymatic activity **1200** comprising an extension product **1230** hybridized to a third oligonucleotide **1299, 1308** according to various embodiments. In various embodiments, the bead **1130** described above can be coupled to the third oligonucleotide **1299, 1308.** In some embodiments, the bead **1130** can be used in the single cell workflows described herein.

**FIG. 12G** illustrates a non-limiting example of compositions that can be included in a reaction mixture for assessing enzymatic activity **1200.** In various embodiments, extension product **1230** can be coupled to a third oligonucleotide **(1229, 1308)** by ligation using a splint oligonucleotide **1297.** For example, a first portion **1297a** of splint oligonucleotide **1297** can be complementary to a sequence of third oligonucleotide **1299.** For example, a second portion **1297b** of splint oligonucleotide can be complementary to a sequence of the extension product **1230** (e.g., can be complementary to **1218rc).** In various embodiments, hybridization of third oligonucleotide **1299** to the first portion **1297a** of the splint oligonucleotide and hybridization of the extension product **1230** to the second portion **1297b** of the splint oligonucleotide facilitates attachment (e.g., ligation) of the third oligonucleotide **1299** to the extension product **1230.** In various embodiments, the reaction mixture further comprises a ligase (not shown) for attaching the third oligonucleotide **1299** to the extension product **1230** via ligation.

**FIG. 12H** illustrates another non-limiting example of compositions that can be included in a reaction mixture for assessing enzymatic activity **1200.** In various embodiments, extension product **1230** can be coupled to a third oligonucleotide **(1229, 1308)** by ligation using a splint oligonucleotide **1297.** For example, a first portion **1297a** of splint oligonucleotide **1297** can be complementary to a sequence of third oligonucleotide **1299.** For example, a second portion **1297b** of splint oligonucleotide can be complementary to a sequence of the extension product **1230** (e.g., can be complementary to **1224).** In various embodiments, hybridization of third oligonucleotide **1299** to the first portion **1297a** of the splint oligonucleotide and hybridization of the extension product **1230** to the second portion **1297b** of the splint oligonucleotide facilitates attachment (e.g., ligation) of the third oligonucleotide **1299** to the extension product **1230.** In various embodiments, the reaction mixture further comprises a ligase (not shown) for attaching the third oligonucleotide **1299** to the extension product **1230** via ligation.

**FIG. I** illustrates another non-limiting example of compositions that can be included in a reaction mixture for assessing enzymatic activity **1200.** In various embodiments, first oligonucleotide **1206** can be coupled to a third oligonucleotide **(1229, 1308)** by ligation using a splint oligonucleotide **1297.** For example, a second portion **1297b** of splint oligonucleotide can be complementary to and hybridize with a first adaptor **1218** and a first portion **1297a** of splint oligonucleotide can be complementary to a third oligonucleotide **1299.**

In various embodiments, first oligonucleotide **1206** can be coupled to a third oligonucleotide **(1229, 1308)** by ligation using a splint oligonucleotide **1297.** For example, a first portion **1297a** can be complementary and hybridize to a first adaptor sequence **1220** and a second portion **1297b** can be complementary to and hybridize with a third oligonucleotide **1299.**

In some cases, the capture sequence is complementary to at least a portion of the extension product **1230** generated by extension of second adapter **1224** (e.g., a second primer). For example, the capture sequence can be complementary to sequence **1218rc** of the extension product **1230** generated by extension of second adapter **1224** (e.g., a second primer).

In some cases, the capture sequence is configured to couple to an amplification product of the amplification reaction **1510** via ligation using a splint oligonucleotide. For example, the capture sequence can be complementary to a first portion of a splint oligonucleotide, and the amplification product of the amplification reaction **1510** can be complementary to a second portion of the splint oligonucleotide. Accordingly, the splint oligonucleotide can hybridize to both the capture sequence of the nucleic acid barcode molecule and the amplification product of amplification reaction **1510.** Ligation of the nucleic acid barcode molecule and the amplification product of amplification reaction 1510 can be catalyzed by a ligase. The ligase can be a ligase suitable for single stranded (ss) nucleic acid ligation, e.g., suitable for ss DNA ligation. Suitable ligases include, e.g., CircLigase, T4 DNA ligase, T7 DNA ligase, SplintR^{®}, T4RNA Ligase, KOD RNA Ligase, double-stranded DNA ligase, ATCV1 ligase, and the like.

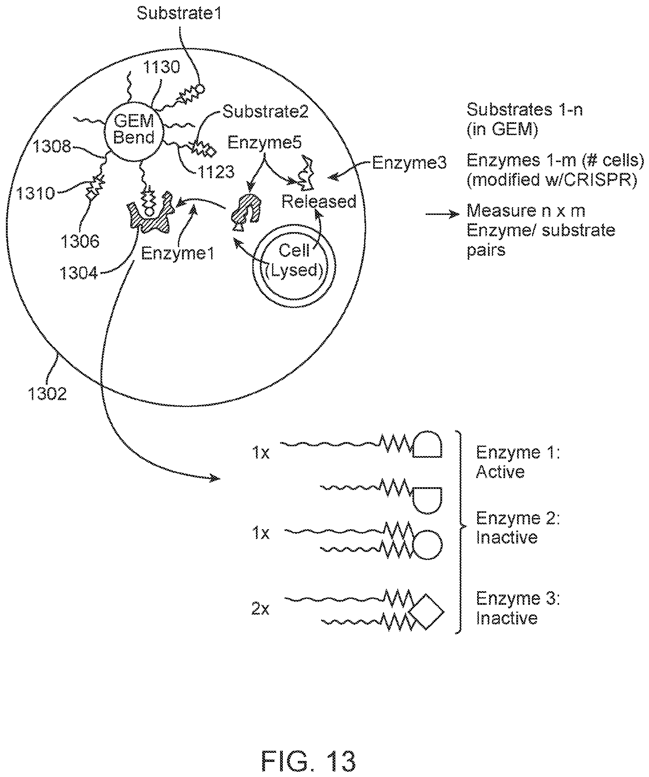

Now referring to **FIGS. 12a****,** **12b****,** and **13****,** in various embodiments, multiple enzymes **1304** can be screened in the same assay. In various embodiments, multiple enzyme **1304** variants can be screened in the same assay. In various embodiments, substrate barcode sequences **1208** can be assist in identifying which enzyme **1304** variant acts on which specific (i.e., unique) cleavable substrate **1306.**

In various embodiments, a composition in a reaction mixture for assessing enzymatic activity **1200** can comprise a cleavable substrate complex as the composition for assessing enzymatic activity. In various embodiments, the composition in a reaction mixture for assessing enzymatic activity **1200** can comprise a plurality of cleavable substrates **1202,** each comprising a first portion **1202a** covalently linked to a second portion **1202b.**

In various embodiments, compositions in a reaction mixture for assessing enzymatic activity **1200** can comprise a plurality of SBO constructs **1204.** In various embodiments, the plurality of SBO constructs **1204** can each comprise a first oligonucleotide **1206** comprising a substrate barcode sequence **1208** and a first linker **1210** that connects the first oligonucleotide **1206** to a first portion **1202a** of one of the cleavable substrates **1306.**

In various embodiments, compositions in a reaction mixture for assessing enzymatic activity **1200** can further comprise a plurality of blocking constructs **1212.** In various embodiments, each of the blocking constructs **1212** can comprise a second oligonucleotide **1214** that can be complementary to at least a portion of the first oligonucleotide **1206** and a second linker **1216** that connects the second oligonucleotide **1214** to a second portion **1202b** of one of the cleavable substrates **1202.**

In various embodiments, each of the first oligonucleotides **1206** of each of the SBO constructs **1204** in each of the substrate complexes can comprise a first adapter binding site **1218** adjacent to a 5' end of the barcode sequence **1208** and a second adapter binding site **1220** adjacent to a 3' end of the barcode sequence **1208.** In various embodiments, the reaction mixture or composition can further comprise a plurality of first adapters (e.g. forward primers) **1222** comprising a sequence that is complementary to at least a portion of the first adapter binding site **1218.** In various embodiments, a plurality of second adapters (e.g. reverse primers) **1224** comprising a sequence that is complementary to at least a portion of the second adapter binding site **1220.**

In various embodiments, a plurality of the cleavable substrates **1202** can be different from one another. In such embodiments, an enzymatic activity of more than one enzyme **1304** can be quantified in the same experiment or assay using different cleavable substrates **1306.** In various embodiments, different cleavable substrates **1306** can allow for assessment (e.g., quantification) of a variety of different enzyme variants.

Third oligonucleotides in accordance with embodiments of the invention can include, e.g., nucleic acid barcode molecules comprising bead specific barcodes, capture sequences, complementary sequences to at least a portion of a first oligonucleotide, unique molecular identifiers or any combination thereof. In various embodiments, the reaction mixture or compositions can further comprise a bead **1130** coupled to a plurality of third oligonucleotides 1308. In various embodiments, third oligonucleotides can comprise a nucleic acid barcode molecule comprising a bead specific barcode. In various embodiments, third oligonucleotides can comprise a capture sequence. In various embodiments, the capture sequence can be complementary to at least a portion of the first oligonucleotide of the plurality of SBO constructs. In various embodiments, third oligonucleotides **1308** can, optionally, comprise a unique molecular identifier (UMI). In various embodiments, the capture sequence **1123** can be complementary to at least a portion of the first oligonucleotide **1206** of the plurality of SBO constructs **1204.** In various embodiments, each of the third oligonucleotides can comprise a bead specific barcode. In various embodiments, the bead specific barcode can identify an enzyme. In various embodiments, the UMI can identify a cell of origin.

### III. PARTITIONS AND METHODS:

**FIG. 13** illustrates a partition comprising a bead **1130** having several oligonucleotides **1308** extending therefrom, the oligonucleotides **1308** comprising a variety of different cleavable substrates **1304, 1306.**

In various embodiments, a partition **1302** can comprise a well, droplet, or other structure or device for partitioning an assay or experiment. In various embodiments, a partition **1302** can, optionally, comprise a bead **1130** having a plurality of oligonucleotides **1308** extending therefrom. In various embodiments, an SBO construct **1310** can be hybridized to an oligonucleotide **1308** for downstream sequencing. In various embodiments, the SBO constructs **1310** in the partition **1302** can comprise a plurality of unique cleavable substrates **1306** that can react to one or more unique (e.g. modified) enzymes **1304.** In various embodiments, a capture sequence **1123** can include a complementary sequence to an adapter binding site of an SBO construct **1310.**

Partitions in accordance with embodiments of the invention can include, e.g., third oligonucleotides comprising nucleic acid barcode molecules, partition-specific barcode sequences, captures sequences, UMIs, splint oligonucleotides, or any combination thereof. In various embodiments, a third oligonucleotide can comprise a nucleic acid barcode molecule comprising a partition-specific barcode sequence and a capture sequence, wherein the capture sequence can be complementary to at least a portion of the first oligonucleotide of an SBO construct. In various embodiments, the third oligonucleotide can, optionally, comprise a UMI. In various embodiments, the capture sequence can be complementary to at least a portion of a first adapter binding site. In various embodiments, the partition can further comprise a splint oligonucleotide that can hybridize to the third oligonucleotide and at least a portion of the first oligonucleotide. In various embodiments, the partition can further comprise a splint oligonucleotide that can hybridize to the third oligonucleotide and at least a portion of a reverse complement of the first oligonucleotide. In various embodiments, the third oligo can be coupled to a bead, wherein the partition-specific barcode sequence can be a bead-specific barcode sequence.

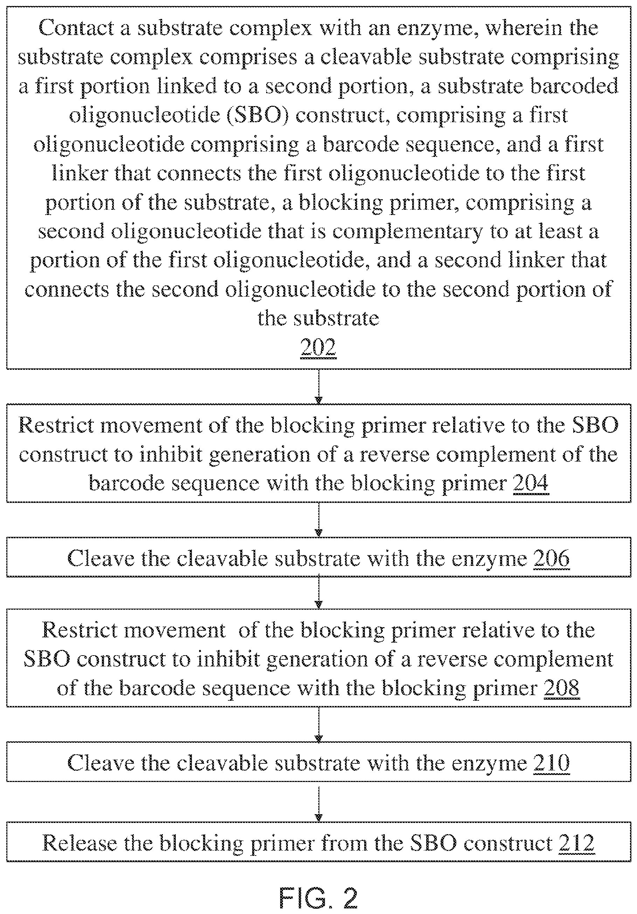

In various aspects of the disclosure, a method for assessing enzymatic activity is described. Referring to **FIG. 2****,** the method can comprise the step of contacting a substrate complex with an enzyme **202.** In various embodiments, the substrate complex can comprise a cleavable substrate comprising a first portion linked or covalently linked to a second portion. In various embodiments, the substrate complex can comprise a substrate barcoded oligonucleotide (SBO) construct, comprising a first oligonucleotide comprising a barcode sequence and a first linker that connects the first oligonucleotide to the first portion of the cleavable substrate. In various embodiments, the substrate complex can comprise a blocking construct, comprising a second oligonucleotide that can be complementary to at least a portion of the first oligonucleotide and a second linker that can connect the second oligonucleotide to the second portion of the cleavable substrate.

In various embodiments, the method can comprise the step of restricting movement of the blocking construct relative to the SBO construct to inhibit generation of a reverse complement of the barcode sequence with the blocking construct **204.** In various embodiments, the physical presence of the blocking construct can prevent a primer from binding, thereby, preventing amplification. In various embodiments, physically restricting movement of the blocking construct relative to the SBO construct comprises increases the binding affinity between the blocking construct and the first oligonucleotide.

In various embodiments, the method can comprise the step of cleaving the cleavable substrate with the enzyme **206.** In various embodiments, the cleavable substrate can be what holds the SBO construct and the blocking primary in close proximity to one another. In various embodiments, ensuring the blocking construct is nearby and not free-floating in a solution increases the affinity of the blocking construct to the SBO construct.

In various embodiments, the method can comprise the step of releasing the blocking construct from the SBO construct **208.** In various embodiments, when the blocking construct is released the second adapter can more easily more in and hybridize with the first oligonucleotide. In various embodiments, the second adapter can outcompete the blocking construct for hybridization once release occurs. In various embodiments, the second adapter sequence is more complementary to the second adapter binding site of the first oligonucleotide than the blocking construct. In various embodiments, the increase complementation ensures the second adapter has more affinity to the second adapter binding site of the first oligonucleotide than the blocking construct.

In various embodiments, the method further comprises the step of conducting an amplification reaction on the first oligonucleotide comprising the barcode sequence **210.** In various embodiments, the amplification reaction can comprise annealing a first adapter to a first adapter binding site adjacent to a 5' end of the barcode sequence and a second adapter to a second adapter binding site adjacent to a 3' end of the barcode sequence and generating a reverse complement of the barcode sequence with a polymerase. In various embodiments, the step of conducting an amplification reaction can result in producing an extension product.

Amplification reactions in accordance with embodiments of the invention can include, e.g., methods to extend adapters/primers, polymerase chain reaction (PCR), reverse transcription or any combination thereof. In various embodiments, an amplification reaction can comprise PCR. In various embodiments, an amplification reaction can comprise reverse transcription.

In various embodiments, the method can further comprise the step of assessing enzymatic activity based on a reaction product of the amplification reaction 212. In various embodiments, the reaction product is detectable. In various embodiments, the reaction product comprises a detectable label. In various embodiments, the reaction product encodes a detectable label. In various embodiments, the detectable label comprises a fluorescent molecule.

In various embodiments, the method further comprises the step of identifying the enzyme using the barcode sequence.

In various embodiments, the method further comprises the step of modifying an amino acid sequence of the enzyme. In various embodiments, modifying an amino acid sequence of the enzyme comprises a CRISPR-mediated amino acid sequence modification.

In various embodiments, the cleavable substrate can comprise a protein. In various embodiments, the cleavable substrate can comprise a carbohydrate. In various embodiments, the cleavable substrate can comprise a double stranded nucleotide. In various embodiments, the cleavable substrate can comprise a synthetic compound. In various embodiments, the cleavable substrate can comprise a polynucleotide. In various embodiments, the first and second linkers can each comprise an inert polymer. In various embodiments, the inert polymer can comprise polyethylene glycol (PEG).

In various embodiments, the method can comprise the step of increasing an affinity between the SBO constructs and the blocking constructs. In various embodiments, the second oligonucleotide can comprise at least one LNA. In various embodiments, the second oligonucleotide comprises only LNAs. In various embodiments, the portion of the first oligonucleotide that is complementary to the second nucleotide can comprise at least one LNA. In various embodiments the portion of the first oligonucleotide that is complementary to the second nucleotide can be comprised of only LNAs.

In various embodiments, the blocking construct can prohibit the amplification reaction. In various embodiments, the second oligonucleotide can comprise a non-reactive 3' end. In various embodiments, the non-reactive end can be phosphorylated or designed to not be extendable using a polymerase.

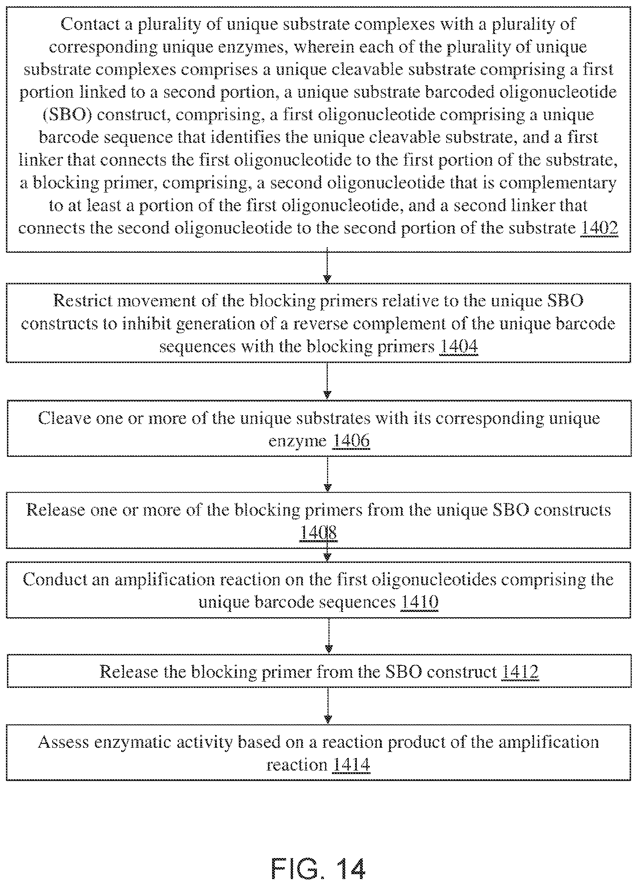

Referring to **FIG. 14****,** a method for assessing and comparing enzymatic activity is disclosed. In various embodiments, the method comprises the step of contacting a plurality of unique substrate complexes with a plurality of corresponding unique enzymes **1402.** In various embodiments, each of the plurality of unique substrate complexes comprises a unique cleavable substrate comprising a first portion linked to a second portion, a unique substrate barcoded oligonucleotide (SBO) construct, comprising, a first oligonucleotide comprising a substrate barcode sequence that identifies the unique cleavable substrate, and a first linker that connects the first oligonucleotide to the first portion of the cleavable substrate, a blocking construct, comprising, a second oligonucleotide that is complementary to at least a portion of the first oligonucleotide, and a second linker that connects the second oligonucleotide to the second portion of the cleavable substrate.

In various embodiments, the method can further comprise restricting movement of the blocking constructs relative to the unique SBO constructs to inhibit generation of a reverse complement of the substrate barcode sequences with the blocking constructs **1404.**

In various embodiments, the method can further comprise cleaving one or more of the unique cleavable substrates with its corresponding unique enzyme **1406.** In various embodiments, unique cleavable substrates can be specifically selected as targets for specific enzymes. In various embodiments, the unique cleavable substrates can be targets for the same enzymes, however, substrate cleavage can occur at different rates depending on which substrates are selected. In various embodiments, the cleavable substrates can be customized. In various embodiments, the cleavable substrates can be naturally occurring. In various embodiments, the cleavable substrates can be engineered.

In various embodiments, the method can comprise the step of releasing one or more of the blocking constructs from the unique SBO constructs **1408.**

In various embodiments, the method can comprise the step of conducting an amplification reaction on the first oligonucleotides comprising the substrate barcode sequences **1410.**

In various embodiments, the method can comprise the step of releasing the blocking construct from the SBO construct **1412**

In various embodiments, the method can comprise the step of assessing enzymatic activity based on a reaction product of the amplification reaction **1414.**

In various embodiments, the method can comprise the step of linking the substrate complexes (or extension products thereof) to a plurality of beads (e.g. beads that comprise capture sequences, e.g., a capture sequence disclosed herein). In various embodiments, the method can comprise the step of linking one or more extension products to a plurality of beads. In various embodiments, linking comprises hybridization. In various embodiments, linking comprises hybridization of two singled stranded nucleic acids. In various embodiments, each bead can be coupled to a plurality of third oligonucleotides. In various embodiments, the third oligonucleotides can each comprise a nucleic acid barcode molecule comprising a bead-specific barcode and a capture sequence that is complementary to at least a portion of a portion of the first oligonucleotide, a portion of an extension product of the first oligonucleotide (e.g. a portion of any of, reverse complement of, or overlapping region of the following: adaptor binding sequences, adaptors, barcoded sequences), or a portion of a splint oligonucleotide. In various embodiments, the method can comprise hybridizing a portion of the third oligonucleotides (e.g. a capture sequence) to a portion of the first oligonucleotides (e.g. a complementary sequence to the capture sequence). In various embodiments, the third oligonucleotide can, optionally, comprise a UMI. In various embodiments, the third oligonucleotides can each comprise a bead specific barcode and a capture sequence. In various embodiments, the third oligonucleotides can each comprise a bead specific barcode, a UMI, and a capture sequence.

In various embodiments, the method can further comprise the step of identifying a cell of origin using the bead specific barcode. In various embodiments, the bead specific barcode can identify an enzyme or modified enzyme.

In various embodiments, the method can further comprise the step of identifying one or more of the enzymes using the substrate barcode nucleotides.

In various embodiments, the method can further comprise the step of modifying an amino acid sequence of one or more of the enzymes. In various embodiments, being able to easily modify the amino acid sequence of one or more of the enzymes and to then be able to quantify an enzymatic activity for each of the enzymes allows for massive parallel enzymatic assays of a pool of modified enzymes allowing for fast optimization (e.g. increased or decreased enzymatic activity based on a specified application by a researcher).

In various embodiments, the method can comprise the step of modifying an amino acid sequence of one or more of the enzymes comprises a CRISPR-mediated amino acid sequence modification. In various embodiments, using CRISPIR gene editing enables modification of many enzymes in parallel. In various embodiments, the step of modifying the amino acid sequence of the one or more enzymes occurs prior to the step of contacting the substrate complex with the enzyme.

In various embodiments, the method can comprise the step of comparing enzymatic activity between two or more enzymes having different amino acid sequences.

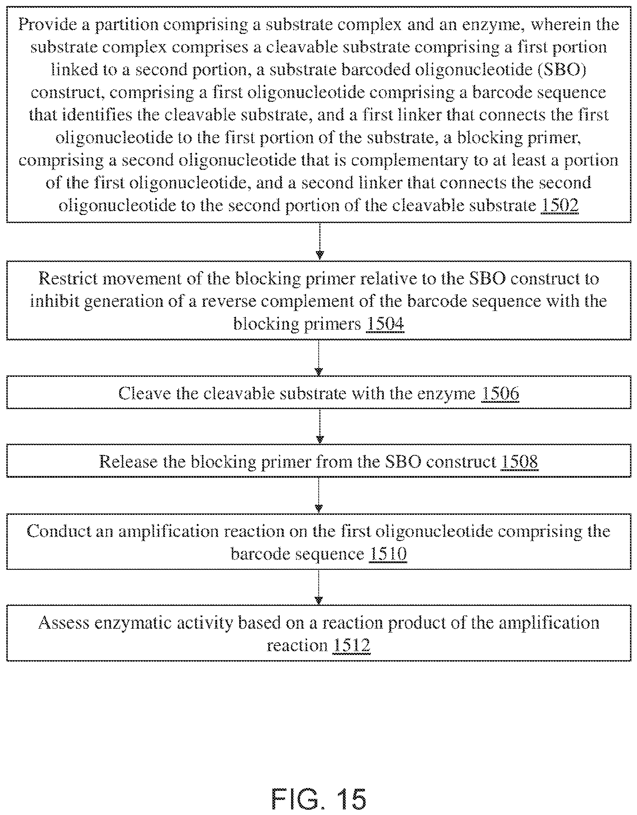

Referring to **FIG. 15****,** a method of assessing enzymatic activity within a partition is disclosed according to various embodiments. In various embodiments, the method can comprise the step of providing a partition comprising a substrate complex and an enzyme **1502.** In various embodiments, the substrate complex comprises a cleavable substrate comprising a first portion linked to a second portion, a substrate barcoded oligonucleotide (SBO) construct, comprising a first oligonucleotide comprising a barcode sequence that identifies the cleavable substrate, and a first linker that connects the first oligonucleotide to the first portion of the substrate, a blocking construct, comprising a second oligonucleotide that is complementary to at least a portion of the first oligonucleotide, and a second linker that connects the second oligonucleotide to the second portion of the cleavable substrate.

In various embodiments, the method can comprise the step of restricting movement of the blocking construct relative to the SBO construct to inhibit generation of a reverse complement of the barcode sequence with the blocking constructs **1504.**

In various embodiments, the method can comprise the step of cleaving the cleavable substrate with the enzyme **1506.**

In various embodiments, the method can comprise the step of releasing the blocking construct from the SBO construct **1508.**

In various embodiments, the method can comprise the step of conducting an amplification reaction on the first oligonucleotide comprising the barcode sequence **1510.**

In various embodiments, the method can comprise the step of assessing enzymatic activity based on a reaction product of the amplification reaction **1512.**

Aspects of the invention relate to partitions which can include, e.g., cells lysed cells, enzymes expressed by cells, adaptor sequences, substrate complexes, or any combination thereof. Additionally, aspects of the invention relate to creating such partitions. In various embodiments, a partition can comprise a cell. In various embodiments, the enzyme can be expressed by the cell. In various embodiments, the partition can comprise a lysed cell which can cause release of the enzyme into the partition and which can enable the step of contacting the enzyme with the substrate complex. In various embodiments, the cell can comprise a plurality of different enzymes. In various embodiments, many cells can each comprise a different set of enzymes. In various embodiments, the method can include many cells and each cell can comprise a single enzyme variant.

In various embodiments, screening a plurality of enzymes can be useful in functional assays. For example, glucose metabolism requires many different enzymatic reactions. Such embodiments, can allow for determination of mechanisms and actions and impact of novel therapeutics. In various embodiments, screening a plurality of enzymes can be useful in characterizing cell pathways.

In various embodiments, the method can comprise the step of modifying an amino acid sequence of at least one of the enzymes, e.g., by using CRISPR. In various embodiments, the step of modifying can comprise a CRISPR-mediated amino acid sequence modification. In various embodiments, the step of modifying the amino acid sequence can occur prior to providing the partition.

In various embodiments, the partition can comprise a bead, wherein each bead can be coupled to a plurality of third oligonucleotides. In various embodiments, each of the third oligonucleotides can comprise a nucleic acid barcode molecule comprising a unique molecular identifier (UMI) and a capture sequence that can be complementary to at least a portion of the first oligonucleotide sequences. In various embodiments, the third oligonucleotide can comprise a bead specific barcode and a capture sequence. In various embodiments, the third oligonucleotide can comprise a bead specific barcode, a UMI, and a capture sequence.

In various embodiments, the method can comprise the step of identifying the cell using the UMI. In various embodiments, the method can comprise the step of identifying the enzyme using the bead specific barcode.

In various embodiments, the method can comprise the step of generating a plurality of partitions and repeating steps any or all of the above steps for each partition. In various embodiments, the method can the step of comparing the enzymatic activity of each of the enzymes in each of the partitions.

In various embodiments, the partitions can be wells, tubes, chambers, or any other rigid housing.

In various embodiments, the partitions can be droplets. In various embodiments, the droplets can be water in oil. In various embodiments, the droplets be oil in water.

### IV. KITS:

In various aspects of the disclosure, kits are provided for carrying out the methods described herein.

In various embodiments, kits can comprise a cleavable substrate comprising a first portion linked or covalently linked to a second portion. In various embodiments, the kit can comprise a substrate barcoded oligonucleotide (SBO) construct, comprising a first oligonucleotide comprising a barcode sequence and a first linker that connects the first oligonucleotide to the first portion of the cleavable substrate. In various embodiments, the kit can comprise a blocking construct, comprising a second oligonucleotide that can be complementary to at least a portion of the first oligonucleotide and a second linker that connects the second oligonucleotide to the second portion of the cleavable substrate.

In various embodiments, kits can comprise a plurality of first adapters comprising a sequence that is complementary to at least a portion of the first oligonucleotide and a plurality of second adapters comprising a sequence that is complementary to at least a portion of the first oligonucleotide.

In various embodiments, kits can comprise one or more reagents for conducting an amplification reaction. In various embodiments, reagents can comprise buffers, nucleotides (e.g. dNTPs), Taq polymerase, and adapters.

In various embodiments, kits can comprise a bead coupled to a plurality of third oligonucleotides, wherein the plurality of third oligonucleotide can each comprise a nucleic acid barcode molecule comprising a unique molecular identifier (UMI) and a capture sequence, wherein the capture sequence can be complementary to at least a portion of the first oligonucleotide of the SBO construct. In various embodiments, the third oligonucleotide can comprise a bead specific barcode and a capture sequence. In various embodiments, the third oligonucleotide can comprise only a capture sequence. In various embodiments, the third oligonucleotide can comprise a bead specific barcode and a capture sequence. In various embodiments, the third oligonucleotide can comprise a bead specific barcode, a UMI, and a capture sequence.

In various embodiments, kits can comprise one or more reagents for conducting a CRISPR modification reaction. In various embodiments, the reagents can comprise one or more exogenous CRISPR-associated endonuclease (Cas) molecules, one or more exogenous insert oligonucleotide comprising a tagging sequence, a plurality of exogenous targeting guide ribonucleic acids (gRNAs) configured to modify a targeting genomic locus in the cell, and a plurality of exogenous tagging gRNAs configured to modify a tagging genomic locus in the cell.

### V. SYSTEMS AND METHODS FOR SAMPLE COMPARTMENTALIZATION:

In an aspect, the systems and methods described herein provide for the compartmentalization, depositing, or partitioning of one or more particles (e.g., biological particles, macromolecular constituents of biological particles, beads, reagents, etc.) into discrete compartments or partitions (referred to interchangeably herein as partitions), where each partition maintains separation of its own contents from the contents of other partitions. The partition can be a droplet in an emulsion or a well. A partition can comprise one or more other partitions.

A partition can include one or more particles. A partition can include one or more types of particles. For example, a partition of the present disclosure can comprise one or more biological particles and/or macromolecular constituents thereof. A partition can comprise one or more beads. A partition can comprise one or more gel beads. A partition can comprise one or more cell beads. A partition can include a single gel bead, a single cell bead, or both a single cell bead and single gel bead. A partition can include one or more reagents. Alternatively, a partition can be unoccupied. For example, a partition may not comprise a bead.

Unique identifiers, such as barcodes, can be injected into the droplets prior to, subsequent to, or concurrently with droplet generation, such as via a bead, as described elsewhere herein.

The methods and systems of the present disclosure can comprise methods and systems for generating one or more partitions such as droplets. The droplets can comprise a plurality of droplets in an emulsion. In various examples, the droplets can comprise droplets in a colloid. In various cases, the emulsion can comprise a microemulsion or a nanoemulsion. In various examples, the droplets can be generated with aid of a microfluidic device and/or by subjecting a mixture of immiscible phases to agitation (e.g., in a container). In various cases, a combination of the mentioned methods can be used for droplet and/or emulsion formation.

The partitions described herein may comprise small volumes, for example, less than about 10 microliters (µL), 5µL, 1µL, 900 picoliters (pL), 800 pL, 700 pL, 600 pL, 500 pL, 400pL, 300 pL, 200 pL, 100pL, 50 pL, 20 pL, 10 pL, 1 pL, 500 nanoliters (nL), 100 nL, 50 nL, or less.

For example, in the case of droplet-based partitions, the droplets may have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. Where co-partitioned with beads, it will be appreciated that the sample fluid volume, e.g., including co-partitioned biological particles and/or beads, within the partitions may be less than about 90% of the above described volumes, less than about 80%, less than about 70%, less than about 60%, less than about 50%, less than about 40%, less than about 30%, less than about 20%, or less than about 10% of the above described volumes.

As is described elsewhere herein, partitioning species may generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions may comprise both unoccupied partitions (e.g., empty partitions) and occupied partitions.

Droplets can be formed by creating an emulsion by mixing and/or agitating immiscible phases. Mixing or agitation may comprise various agitation techniques, such as vortexing, pipetting, tube flicking, or other agitation techniques. In various cases, mixing or agitation may be performed without using a microfluidic device. In various examples, the droplets may be formed by exposing a mixture to ultrasound or sonication. Systems and methods for droplet and/or emulsion generation by agitation are described in International Application No. PCT/US20/17785.

### Microfluidic systems:

Microfluidic devices or platforms comprising microfluidic channel networks (e.g., on a chip) can be utilized to generate partitions such as droplets and/or emulsions as described herein. Methods and systems for generating partitions such as droplets, methods of encapsulating biological particles in partitions, methods of increasing the throughput of droplet generation, and various geometries, architectures, and configurations of microfluidic devices and channels are described in U.S. Patent Publication Nos. 2019/0367997 and 2019/0064173.

In various examples, individual particles can be partitioned to discrete partitions by introducing a flowing stream of particles in an aqueous fluid into a flowing stream or reservoir of a non-aqueous fluid, such that droplets may be generated at the junction of the two streams/reservoir, such as at the junction of a microfluidic device provided elsewhere herein.

The methods of the present disclosure may comprise generating partitions and/or encapsulating particles, such as biological particles, in various cases, individual biological particles such as single cells. In various examples, reagents may be encapsulated and/or partitioned (e.g., co-partitioned with biological particles) in the partitions. Various mechanisms may be employed in the partitioning of individual particles. An example may comprise porous membranes through which aqueous mixtures of cells may be extruded into fluids (e.g., non-aqueous fluids).

The partitions can be flowable within fluid streams. The partitions may comprise, for example, micro-vesicles that have an outer barrier surrounding an inner fluid center or core. In various cases, the partitions may comprise a porous matrix that is capable of entraining and/or retaining materials within its matrix. The partitions can be droplets of a first phase within a second phase, wherein the first and second phases are immiscible. For example, the partitions can be droplets of aqueous fluid within a non-aqueous continuous phase (e.g., oil phase). In another example, the partitions can be droplets of a non-aqueous fluid within an aqueous phase. In various examples, the partitions may be provided in a water-in-oil emulsion or oil-in-water emulsion. A variety of different vessels are described in, for example, U.S. Patent Application Publication No. 2014/0155295. Emulsion systems for creating stable droplets in non-aqueous or oil continuous phases are described in, for example, U.S. Patent Application Publication No. 2010/0105112.

Fluid properties (e.g., fluid flow rates, fluid viscosities, etc.), particle properties (e.g., volume fraction, particle size, particle concentration, etc.), microfluidic architectures (e.g., channel geometry, etc.), and other parameters may be adjusted to control the occupancy of the resulting partitions (e.g., number of biological particles per partition, number of beads per partition, etc.). For example, partition occupancy can be controlled by providing the aqueous stream at a certain concentration and/or flow rate of particles. To generate single biological particle partitions, the relative flow rates of the immiscible fluids can be selected such that, on average, the partitions may contain less than one biological particle per partition in order to ensure that those partitions that are occupied are primarily singly occupied. In various cases, partitions among a plurality of partitions may contain at most one biological particle (e.g., bead, DNA, cell or cellular material). In various embodiments, the various parameters (e.g., fluid properties, particle properties, microfluidic architectures, etc.) may be selected or adjusted such that a majority of partitions are occupied, for example, allowing for only a small percentage of unoccupied partitions. The flows and channel architectures can be controlled as to ensure a given number of singly occupied partitions, less than a certain level of unoccupied partitions and/or less than a certain level of multiply occupied partitions.

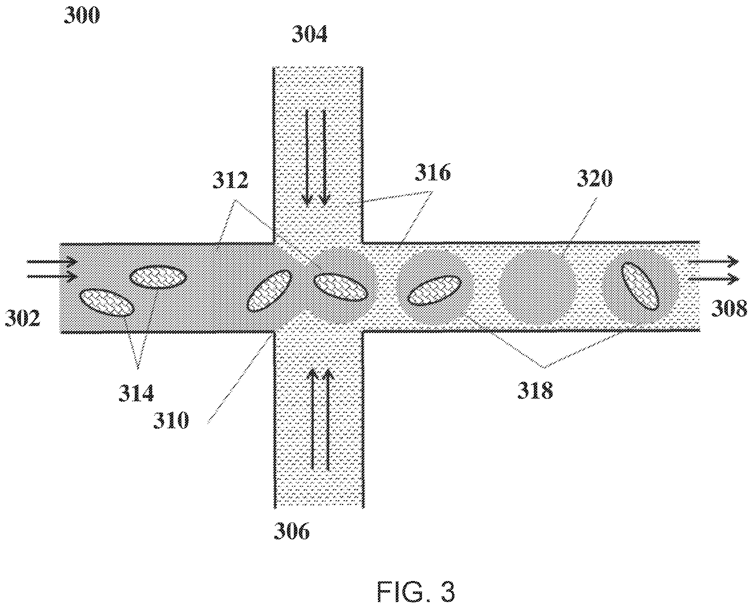

**FIG. 3** shows an example of a microfluidic channel structure **306** for partitioning individual biological particles according to various embodiments. The channel structure **306** can include channel segments **302, 304, 306** and **308** communicating at a channel junction **310.** In operation, a first aqueous fluid **312** that includes suspended biological particles (or cells) **314** may be transported along channel segment **302** into junction **310,** while a second fluid **316** that is immiscible with the aqueous fluid **312** is delivered to the junction **310** from each of channel segments **304** and **306** to create discrete droplets **318, 320** of the first aqueous fluid **312** flowing into channel segment **308,** and flowing away from junction **310.** The channel segment **308** may be fluidically coupled to an outlet reservoir where the discrete droplets can be stored and/or harvested. A discrete droplet generated may include an individual biological particle **314** (such as droplets **318).** A discrete droplet generated may include more than one individual biological particle **314** (not shown in **FIG. 3****).** A discrete droplet may contain no biological particle **314** (such as droplet **320).** Each discrete partition may maintain separation of its own contents (e.g., individual biological particle **314)** from the contents of other partitions.

The second fluid **316** can comprise an oil, such as a fluorinated oil, that includes a fluorosurfactant for stabilizing the resulting droplets, for example, inhibiting subsequent coalescence of the resulting droplets **318, 320.** Examples of particularly useful partitioning fluids and fluorosurfactants are described, for example, in U.S. Patent Application Publication No. 2010/0105112.

As will be appreciated, the channel segments described herein may be coupled to any of a variety of different fluid sources or receiving components, including reservoirs, tubing, manifolds, or fluidic components of other systems. As will be appreciated, the microfluidic channel structure **300** may have other geometries. For example, a microfluidic channel structure can have more than one channel junction. For example, a microfluidic channel structure can have 2, 3, 4, or 5 channel segments each carrying particles (e.g., biological particles, cell beads, and/or gel beads) that meet at a channel junction. Fluid may be directed to flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

The generated droplets may comprise two subsets of droplets: (1) occupied droplets **318,** containing one or more biological particles **314,** and (2) unoccupied droplets **320,** not containing any biological particles **314.** Occupied droplets **318** may comprise singly occupied droplets (having one biological particle) and multiply occupied droplets (having more than one biological particle). As described elsewhere herein, in various cases, the majority of occupied partitions can include no more than one biological particle per occupied partition and various of the generated partitions can be unoccupied (of any biological particle). In various cases, though, various of the occupied partitions may include more than one biological particle. In various cases, the partitioning process may be controlled such that fewer than about 25% of the occupied partitions contain more than one biological particle, and in many cases, fewer than about 20% of the occupied partitions have more than one biological particle, while in various cases, fewer than about 10% or even fewer than about 5% of the occupied partitions include more than one biological particle per partition.

In various cases, it may be desirable to minimize the creation of excessive numbers of empty partitions, such as to reduce costs and/or increase efficiency. While this minimization may be achieved by providing a sufficient number of biological particles (e.g., biological particles **314)** at the partitioning junction **310,** such as to ensure that at least one biological particle is encapsulated in a partition, the Poissonian distribution may expectedly increase the number of partitions that include multiple biological particles. As such, where singly occupied partitions are to be obtained, at most about 95%, 90%, 85%, 80%, 75%, 70%, 65%, 60%, 55%, 50%, 45%, 40%, 35%, 30%, 25%, 20%, 15%, 10%, 5% or less of the generated partitions can be unoccupied.

In various cases, flows can be controlled so as to present a non-Poissonian distribution of single-occupied partitions while providing lower levels of unoccupied partitions (e.g., no more than about 50%, about 25%, or about 10% unoccupied). The above noted ranges of unoccupied partitions can be achieved while still providing any of the single occupancy rates described above.

As will be appreciated, the above-described occupancy rates are also applicable to partitions that include both biological particles and additional reagents, such as beads (e.g., gel beads) carrying nucleic acid barcode molecules (e.g., oligonucleotides).

In various examples, a partition of the plurality of partitions may comprise a single biological particle (e.g., a single cell or a single nucleus of a cell). In various examples, a partition of the plurality of partitions may comprise multiple biological particles. Such partitions may be referred to as multiply occupied partitions, and may comprise, for example, two, three, four or more cells and/or beads (e.g., beads) comprising nucleic acid barcode molecules within a single partition. Accordingly, as noted above, the flow characteristics of the biological particle and/or bead containing fluids and partitioning fluids may be controlled to provide for such multiply occupied partitions. In particular, the flow parameters may be controlled to provide a given occupancy rate at greater than about 50% of the partitions, greater than about 75%, and in various cases greater than about 80%, 90%, 95%, or higher.

Microfluidic systems for partitioning are further described in U.S. Patent Application Pub. No. US 2015/0376609.

### Controlled Partitioning:

In various aspects, provided are systems and methods for controlled partitioning. Droplet size may be controlled by adjusting certain geometric features in channel architecture (e.g., microfluidics channel architecture). For example, an expansion angle, width, and/or length of a channel may be adjusted to control droplet size.

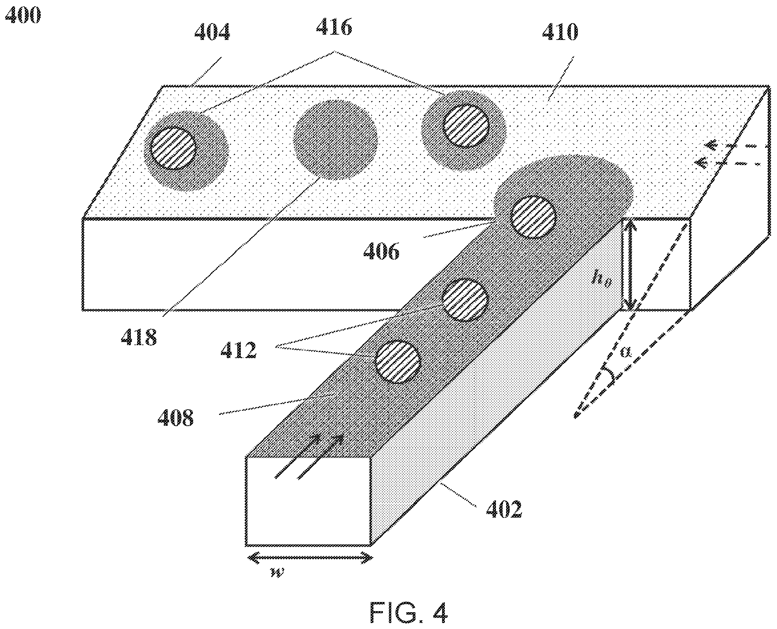

**FIG. 4** shows an example of a microfluidic channel structure for the controlled partitioning of beads into discrete droplets. A channel structure **400** can include a channel segment **402** communicating at a channel junction **406** (or intersection) with a reservoir **404.** The reservoir **404** can be a chamber. Any reference to "reservoir," as used herein, can also refer to a "chamber." In operation, an aqueous fluid **408** that includes suspended beads **412** may be transported along the channel segment **402** into the junction **406** to meet a second fluid **410** that is immiscible with the aqueous fluid **408** in the reservoir **404** to create droplets **416, 418** of the aqueous fluid **408** flowing into the reservoir **404.** At the junction **406** where the aqueous fluid **408** and the second fluid **410** meet, droplets can form based on factors such as the hydrodynamic forces at the junction **406,** flow rates of the two fluids **408, 410,** fluid properties, and certain geometric parameters (e.g., *w, h₀, α,* etc.) of the channel structure **400.** A plurality of droplets can be collected in the reservoir **404** by continuously injecting the aqueous fluid **408** from the channel segment **402** through the junction **406.**

In various instances, the aqueous fluid **408** can have a substantially uniform concentration or frequency of beads **412.** The beads **412** can be introduced into the channel segment **402** from a separate channel (not shown in **FIG. 4****).** The frequency of beads **412** in the channel segment **402** may be controlled by controlling the frequency in which the beads **412** are introduced into the channel segment **202** and/or the relative flow rates of the fluids in the channel segment **402** and the separate channel. In various instances, the beads can be introduced into the channel segment **402** from a plurality of different channels, and the frequency controlled accordingly.

In various instances, the aqueous fluid **408** in the channel segment **402** can comprise biological particles. In various instances, the aqueous fluid **408** can have a substantially uniform concentration or frequency of biological particles. As with the beads, the biological particles can be introduced into the channel segment **402** from a separate channel. The frequency or concentration of the biological particles in the aqueous fluid **408** in the channel segment **402** may be controlled by controlling the frequency in which the biological particles are introduced into the channel segment **402** and/or the relative flow rates of the fluids in the channel segment **402** and the separate channel. In various instances, the biological particles can be introduced into the channel segment **402** from a plurality of different channels, and the frequency controlled accordingly. In various instances, a first separate channel can introduce beads and a second separate channel can introduce biological particles into the channel segment **402.** The first separate channel introducing the beads may be upstream or downstream of the second separate channel introducing the biological particles.

The second fluid **410** can comprise an oil, such as a fluorinated oil, that includes a fluorosurfactant for stabilizing the resulting droplets, for example, inhibiting subsequent coalescence of the resulting droplets.

In various instances, the second fluid **410** may not be subjected to and/or directed to any flow in or out of the reservoir **404.** For example, the second fluid **410** may be substantially stationary in the reservoir **404.** In various instances, the second fluid **410** may be subjected to flow within the reservoir **404,** but not in or out of the reservoir **404,** such as via application of pressure to the reservoir **404** and/or as affected by the incoming flow of the aqueous fluid **408** at the junction **406.** Alternatively, the second fluid **410** may be subjected and/or directed to flow in or out of the reservoir **404.** For example, the reservoir **404** can be a channel directing the second fluid **410** from upstream to downstream, transporting the generated droplets.

Systems and methods for controlled partitioning are described further in PCT/US2018/047551.

### Beads:

Nucleic acid barcode molecules may be delivered to a partition (e.g., a droplet or well) via a solid support or carrier (e.g., a bead). In various cases, nucleic acid barcode molecules are initially associated with the solid support and then released from the solid support upon application of a stimulus, which allows the nucleic acid barcode molecules to dissociate or to be released from the solid support. In specific examples, nucleic acid barcode molecules are initially associated with the solid support (e.g., bead) and then released from the solid support upon application of a biological stimulus, a chemical stimulus, a thermal stimulus, an electrical stimulus, a magnetic stimulus, and/or a photo stimulus.

The solid support may be a bead. A solid support, e.g., a bead, may be porous, non-porous, hollow, solid, semi-solid, and/or a combination thereof. Beads may be solid, semi-solid, semi-fluidic, fluidic, and/or a combination thereof. In various instances, a solid support, e.g., a bead, may be at least partially dissolvable, disruptable, and/or degradable. In various cases, a solid support, e.g., a bead, may not be degradable. In various cases, the solid support, e.g., a bead, may be a gel bead. A gel bead may be a hydrogel bead. A gel bead may be formed from molecular precursors, such as a polymeric or monomeric species. A semi-solid support, e.g., a bead, may be a liposomal bead. Solid supports, e.g., beads, may comprise metals including iron oxide, gold, and silver. In various cases, the solid support, e.g., the bead, may be a silica bead. In various cases, the solid support, e.g., a bead, can be rigid. In other cases, the solid support, e.g., a bead, may be flexible and/or compressible.

A partition may comprise one or more unique identifiers, such as barcodes. Barcodes may be previously, subsequently or concurrently delivered to the partitions that hold the compartmentalized or partitioned biological particle. For example, barcodes may be injected into droplets or deposited in microwells previous to, subsequent to, or concurrently with droplet generation or providing of reagents in the microwells, respectively. The delivery of the barcodes to a particular partition allows for the later attribution of the characteristics of the individual biological particle to the particular partition. Barcodes may be delivered, for example on a nucleic acid molecule (e.g., via a nucleic acid barcode molecule), to a partition via any suitable mechanism. Nucleic acid barcode molecules can be delivered to a partition via a bead. Beads are described in further detail below.

In various cases, nucleic acid barcode molecules can be initially associated with the bead and then released from the bead. Release of the nucleic acid barcode molecules can be passive (e.g., by diffusion out of the bead). In addition or alternatively, release from the bead can be upon application of a stimulus which allows the nucleic acid barcode molecules to dissociate or to be released from the bead. Such stimulus may disrupt the bead, an interaction that couples the nucleic acid barcode molecules to or within the bead, or both. Such stimulus can include, for example, a thermal stimulus, photo-stimulus, chemical stimulus (e.g., change in pH or use of a reducing agent(s)), a mechanical stimulus, a radiation stimulus; a biological stimulus (e.g., enzyme), or any combination thereof.

Methods and systems for partitioning barcode carrying beads into droplets are provided herein, and in in US. Patent Publication Nos. 2019/0367997 and 2019/0064173, and International Application No. PCT/US20/17785.

A bead may be porous, non-porous, solid, semi-solid, semi-fluidic, fluidic, and/or a combination thereof. In various instances, a bead may be dissolvable, disruptable, and/or degradable. Degradable beads, as well as methods for degrading beads, are described in PCT/US2014/044398. In various cases, any combination of stimuli, e.g., stimuli described in PCT/US2014/044398 and US Patent Application Pub. No. 2015/0376609, may trigger degradation of a bead. For example, a change in pH may enable a chemical agent (e.g., DTT) to become an effective reducing agent.

In various cases, a bead may not be degradable. In various cases, the bead may be a gel bead. A gel bead may be a hydrogel bead. A gel bead may be formed from molecular precursors, such as a polymeric or monomeric species. A semi-solid bead may be a liposomal bead. Solid beads may comprise metals including iron oxide, gold, and silver. In various cases, the bead may be a silica bead. In various cases, the bead can be rigid. In other cases, the bead may be flexible and/or compressible.

A bead may be of any suitable shape. Examples of bead shapes include, but are not limited to, spherical, non-spherical, oval, oblong, amorphous, circular, cylindrical, and variations thereof.

Beads may be of uniform size or heterogeneous size. In various cases, the diameter of a bead may be at least about 10 nanometers (nm), 100 nm, 500 nm, 1 micrometer (µm), 5µm, 10µm, 20µm, 30µm, 40µm, 50µm, 60µm, 70µm, 80µm, 90µm, 100µm, 250µm, 500µm, 1mm, or greater. In various cases, a bead may have a diameter of less than about 10 nm, 100 nm, 500 nm, 1µm, 5µm, 10µm, 20µm, 30µm, 40µm, 50µm, 60µm, 70µm, 80µm, 90µm, 100µm, 250µm, 500µm, 1mm, or less. In various cases, a bead may have a diameter in the range of about 40-75µm, 30-75µm, 20-75µm, 40-85µm, 40-95µm, 20-100µm, 10-100µm, 1-100µm, 20-250µm, or 20-500µm.

In certain aspects, beads can be provided as a population or plurality of beads having a relatively monodisperse size distribution. Where it may be desirable to provide relatively consistent amounts of reagents within partitions, maintaining relatively consistent bead characteristics, such as size, can contribute to the overall consistency. In particular, the beads described herein may have size distributions that have a coefficient of variation in their cross-sectional dimensions of less than 50%, less than 40%, less than 30%, less than 20%, and in various cases less than 15%, less than 10%, less than 5%, or less.

A bead may comprise natural and/or synthetic materials. For example, a bead can comprise a natural polymer, a synthetic polymer or both natural and synthetic polymers. See, e.g., PCT/US2014/044398. Beads may also be formed from materials other than polymers, including lipids, micelles, ceramics, glass-ceramics, material composites, metals, other inorganic materials, and others.

In various cases, the bead may comprise covalent or ionic bonds between polymeric precursors (e.g., monomers, oligomers, linear polymers), nucleic acid barcode molecules (e.g., oligonucleotides), primers, and other entities. In various cases, the covalent bonds can be carbon-carbon bonds, thioether bonds, or carbon-heteroatom bonds.

In various cases, a plurality of nucleic acid barcode molecules may be attached to a bead. The nucleic acid barcode molecules may be attached directly or indirectly to the bead. In various cases, the nucleic acid barcode molecules may be covalently linked to the bead. In various cases, the nucleic acid barcode molecules are covalently linked to the bead via a linker. In various cases, the linker is a degradable linker. In various cases, the linker comprises a labile bond configured to release said nucleic acid barcode molecule of said plurality of nucleic acid barcode molecules. In various cases, the labile bond comprises a disulfide linkage.

Activation of disulfide linkages within a bead can be controlled such that only a small number of disulfide linkages are activated. Methods of controlling activation of disulfide linkages within a bead are described in PCT/US2014/044398.

In various cases, a bead may comprise an acrydite moiety, which in certain aspects may be used to attach one or more nucleic acid barcode molecules (e.g., barcode sequence, nucleic acid barcode molecule, barcoded oligonucleotide, primer, or other oligonucleotide) to the bead. Acrydite moieties, as well as their uses in attaching nucleic acid molecules to beads, are described in PCT/US2014/044398.

For example, precursors (e.g., monomers, cross-linkers) that are polymerized to form a bead may comprise acrydite moieties, such that when a bead is generated, the bead also comprises acrydite moieties. The acrydite moieties can be attached to a nucleic acid molecule, e.g., a nucleic acid barcode molecule described herein.

In various cases, precursors comprising a functional group that is reactive or capable of being activated such that it becomes reactive can be polymerized with other precursors to generate gel beads comprising the activated or activatable functional group. The functional group may then be used to attach additional species (e.g., disulfide linkers, primers, other oligonucleotides, etc.) to the gel beads. Exemplary precursors comprising functional groups are described in PCT/US2014/044398.

Other non- limiting examples of labile bonds that may be coupled to a precursor or bead are described in PCT/US2014/044398. A bond may be cleavable via other nucleic acid molecule targeting enzymes, such as restriction enzymes (e.g., restriction endonucleases), as described further below.

Species may be encapsulated in beads during bead generation (e.g., during polymerization of precursors). Such species may or may not participate in polymerization. See, e.g., PCT/US2014/044398. Such species may include, for example, nucleic acid molecules (e.g., oligonucleotides), reagents for a nucleic acid amplification reaction (e.g., primers, polymerases, dNTPs, co-factors (e.g., ionic co-factors), buffers) including those described herein, reagents for enzymatic reactions (e.g., enzymes, co-factors, substrates, buffers), reagents for nucleic acid modification reactions such as polymerization, ligation, or digestion, and/or reagents for template preparation (e.g., tagmentation) for one or more sequencing platforms (e.g., Nextera^{®} for Illumina^{®}). Such species may include one or more enzymes described herein, including without limitation, polymerase, reverse transcriptase, restriction enzymes (e.g., endonuclease), transposase, ligase, proteinase K, DNAse, etc. Such species may include one or more reagents described elsewhere herein (e.g., lysis agents, inhibitors, inactivating agents, chelating agents, stimulus). Alternatively, or in addition, species may be partitioned in a partition (e.g., droplet) during or subsequent to partition formation. Such species may include, without limitation, the abovementioned species that may also be encapsulated in a bead.

In various cases, beads can be non-covalently loaded with one or more reagents. The beads can be non-covalently loaded by, for instance, subjecting the beads to conditions sufficient to swell the beads, allowing sufficient time for the reagents to diffuse into the interiors of the beads, and subjecting the beads to conditions sufficient to de-swell the beads. The swelling of the beads may be accomplished, for instance, by placing the beads in a thermodynamically favorable solvent, subjecting the beads to a higher or lower temperature, subjecting the beads to a higher or lower ion concentration, and/or subjecting the beads to an electric field. The swelling of the beads may be accomplished by various swelling methods. The de-swelling of the beads may be accomplished, for instance, by transferring the beads in a thermodynamically unfavorable solvent, subjecting the beads to lower or high temperatures, subjecting the beads to a lower or higher ion concentration, and/or removing an electric field. The de-swelling of the beads may be accomplished by various de-swelling methods. Transferring the beads may cause pores in the bead to shrink. The shrinking may then hinder reagents within the beads from diffusing out of the interiors of the beads. The hindrance may be due to steric interactions between the reagents and the interiors of the beads. The transfer may be accomplished microfluidically. For instance, the transfer may be achieved by moving the beads from one co-flowing solvent stream to a different co-flowing solvent stream. The swellability and/or pore size of the beads may be adjusted by changing the polymer composition of the bead.

Any suitable number of molecular tag molecules (e.g., primer, barcoded oligonucleotide) can be associated with a bead such that, upon release from the bead, the molecular tag molecules (e.g., primer, e.g., barcoded oligonucleotide) are present in the partition at a pre-defined concentration. Such pre-defined concentration may be selected to facilitate certain reactions for generating a sequencing library, e.g., amplification, within the partition. In various cases, the pre-defined concentration of the primer can be limited by the process of producing oligonucleotide bearing beads.

### Nucleic Acid Barcode Molecules:

A nucleic acid barcode molecule may contain one or more barcode sequences. A plurality of nucleic acid barcode molecules may be coupled to a bead. The one or more barcode sequences may include sequences that are the same for all nucleic acid molecules coupled to a given bead and/or sequences that are different across all nucleic acid molecules coupled to the given bead. The nucleic acid molecule may be incorporated into the bead.

Nucleic acid barcode molecules can comprise one or more functional sequences for coupling to an analyte or analyte tag such as a reporter oligonucleotide. Such functional sequences can include, e.g., a template switch oligonucleotide (TSO) sequence, a primer sequence (e.g., a poly T sequence, or a nucleic acid primer sequence complementary to a target nucleic acid sequence and/or for amplifying a target nucleic acid sequence, a random primer, and a primer sequence for messenger RNA).

In various cases, the nucleic acid barcode molecule can further comprise a unique molecular identifier (UMI). In various cases, the nucleic acid barcode molecule can comprise one or more functional sequences, for example, for attachment to a sequencing flow cell, such as, for example, a P5 sequence (or a portion thereof) for Illumina^{®} sequencing. In various cases, the nucleic acid barcode molecule or derivative thereof (e.g., oligonucleotide or polynucleotide generated from the nucleic acid molecule) can comprise another functional sequence, such as, for example, a P7 sequence (or a portion thereof) for attachment to a sequencing flow cell for Illumina sequencing. In various cases, the nucleic acid molecule can comprise an R1 primer sequence for Illumina sequencing. In various cases, the nucleic acid molecule can comprise an R2 primer sequence for Illumina sequencing. In various cases, a functional sequence can comprise a partial sequence, such as a partial barcode sequence, partial anchoring sequence, partial sequencing primer sequence (e.g., partial R1 sequence, partial R2 sequence, etc.), a partial sequence configured to attach to the flow cell of a sequencer (e.g., partial P5 sequence, partial P7 sequence, etc.), or a partial sequence of any other type of sequence described elsewhere herein. A partial sequence may contain a contiguous or continuous portion or segment, but not all, of a full sequence, for example. In various cases, a downstream procedure may extend the partial sequence, or derivative thereof, to achieve a full sequence of the partial sequence, or derivative thereof.

Examples of such nucleic acid molecules (e.g., oligonucleotides, polynucleotides, etc.) and uses thereof, as may be used with compositions, devices, methods and systems of the present disclosure, are provided in U.S. Patent Pub. Nos. 2014/0378345 and 2015/0376609.

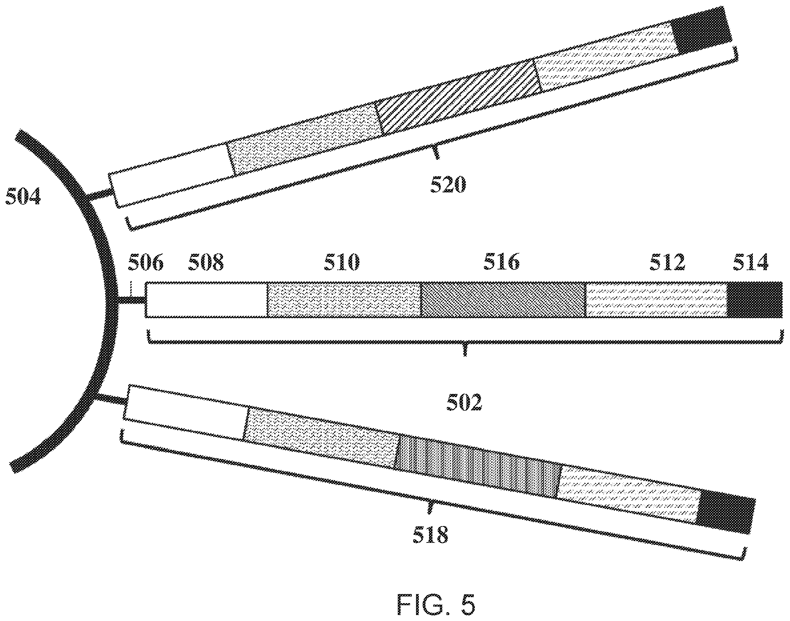

**FIG. 5** illustrates an example of a barcode carrying bead. A nucleic acid barcode molecule **502** can be coupled to a bead **504** by a releasable linkage **506,** such as, for example, a disulfide linker. The same bead **504** may be coupled (e.g., via releasable linkage) to one or more other nucleic acid barcode molecules **518, 520.** The nucleic acid barcode molecule **502** may be or comprise a barcode. As noted elsewhere herein, the structure of the barcode may comprise a number of sequence elements. The nucleic acid barcode molecule **502** may comprise a functional sequence **508** that may be used in subsequent processing. For example, the functional sequence **508** may include one or more of a sequencer specific flow cell attachment sequence (e.g., a P5 sequence for Illumina^{®} sequencing systems) and a sequencing primer sequence (e.g., a R1 primer for Illumina^{®} sequencing systems), or partial sequence(s) thereof. The nucleic acid barcode molecule **502** may comprise a barcode sequence **510** for use in barcoding the sample (e.g., DNA, RNA, protein, etc.). In various cases, the barcode sequence **510** can be bead-specific such that the barcode sequence **510** is common to all nucleic acid barcode molecules (e.g., including nucleic acid barcode molecule **502)** coupled to the same bead **504.** Alternatively or in addition, the barcode sequence **510** can be partition-specific such that the barcode sequence **510** is common to all nucleic acid barcode molecules coupled to one or more beads that are partitioned into the same partition. The nucleic acid barcode molecule **502** may comprise sequence **512** complementary to an analyte of interest, e.g., a priming sequence. Sequence **512** can be a poly-T sequence complementary to a poly-A tail of an mRNA analyte, a targeted priming sequence, and/or a random priming sequence. The nucleic acid barcode molecule **502** may comprise an anchoring sequence **514** to ensure that the specific priming sequence **512** hybridizes at the sequence end (e.g., of the mRNA). For example, the anchoring sequence **514** can include a random short sequence of nucleotides, such as a 1-mer, 2-mer, 3-mer or longer sequence, which can ensure that a poly-T segment is more likely to hybridize at the sequence end of the poly-A tail of the mRNA.

The nucleic acid barcode molecule **502** may comprise a unique molecular identifying (UMI) sequence **516.** In various cases, the unique molecular identifying sequence **516** may comprise from about 5 to about 8 nucleotides. Alternatively, the unique molecular identifying sequence **516** may compress less than about 5 or more than about 8 nucleotides. The unique molecular identifying sequence **516** may be a unique sequence that varies across individual nucleic acid barcode molecules (e.g., **502, 518, 520,** etc.) coupled to a single bead (e.g., bead **504).** In various cases, the unique molecular identifying sequence **516** may be a random sequence (e.g., such as a random N-mer sequence). For example, the UMI **516** may provide a unique identifier of the starting analyte (e.g., mRNA) molecule that was captured, in order to allow quantitation of the number of original expressed RNA molecules. As will be appreciated, although **FIG. 5** shows three nucleic acid barcode molecules **502, 518, 520** coupled to the surface of the bead **504,** an individual bead may be coupled to any number of individual nucleic acid barcode molecules, for example, from one to tens to hundreds of thousands, millions, or even a billion of individual nucleic acid barcode molecules. The respective barcodes for the individual nucleic acid barcode molecules can comprise both common sequence segments or relatively common sequence segments (e.g., **508, 510, 512,** etc.) and variable or unique sequence segments (e.g., **516)** between different individual nucleic acid barcode molecules coupled to the same bead.

In operation, a biological particle (e.g., cell, DNA, RNA, etc.) can be co-partitioned along with a barcode bearing bead **504.** The nucleic acid barcode molecules **502, 518, 520** can be released from the bead **504** in the partition. By way of example, in the context of analyzing sample RNA, the poly-T segment (e.g., **512)** of one of the released nucleic acid barcode molecules (e.g., **502)** can hybridize to the poly-A tail of a mRNA molecule. Reverse transcription may result in a cDNA transcript of the mRNA, but which transcript includes each of the sequence segments **508, 510, 516** of the nucleic acid barcode molecule **502.** Because the nucleic acid barcode molecule **502** comprises an anchoring sequence **514,** it will more likely hybridize to and prime reverse transcription at the sequence end of the poly-A tail of the mRNA. cDNA transcripts of the individual mRNA molecules from any given partition may include a common barcode sequence segment 510. Within any given partition, all of the DNA transcripts of the individual mRNA molecules may include a common barcode sequence segment **510.** However, the transcripts made from the different mRNA molecules within a given partition may vary at the unique molecular identifying sequence **516** segment (e.g., UMI segment). Beneficially, even following any subsequent amplification of the contents of a given partition, the number of different UMIs **516** can be indicative of the quantity of mRNA originating from a given partition, and thus from the biological particle (e.g., cell). As noted above, the transcripts can be amplified, cleaned up and sequenced to identify the sequence of the cDNA transcript of the mRNA, as well as to sequence the barcode segment and the UMI segment. While a poly-T primer sequence is described, other targeted or random priming sequences may also be used in priming the reverse transcription reaction. Likewise, although described as releasing the barcoded oligonucleotides into the partition, in various cases, the nucleic acid barcode molecules bound to the bead (e.g., gel bead) may be used to hybridize and capture the mRNA on the solid phase of the bead, for example, in order to facilitate the separation of the RNA from other cell contents. In such cases, further processing may be performed, in the partitions or outside the partitions (e.g., in bulk). For instance, the RNA molecules on the beads may be subjected to reverse transcription or other nucleic acid processing, additional adapter sequences may be added to the barcoded nucleic acid molecules, or other nucleic acid reactions (e.g., amplification, nucleic acid extension) may be performed. The beads or products thereof (e.g., barcoded nucleic acid molecules) may be collected from the partitions, and/or pooled together and subsequently subjected to clean up and further characterization (e.g., sequencing).

The operations described herein may be performed at any useful or convenient step. For instance, the beads comprising nucleic acid barcode molecules may be introduced into a partition (e.g., well or droplet) prior to, during, or following introduction of a sample into the partition. The nucleic acid molecules of a sample may be subjected to barcoding, which may occur on the bead (in cases where the nucleic acid molecules remain coupled to the bead) or following release of the nucleic acid barcode molecules into the partition. In cases where analytes from the sample are captured by the nucleic acid barcode molecules in a partition (e.g., by hybridization), captured analytes from various partitions may be collected, pooled, and subjected to further processing (e.g., reverse transcription, adapter attachment, amplification, clean up, sequencing). For example, in cases wherein the nucleic acid molecules from the sample remain attached to the bead, the beads from various partitions may be collected, pooled, and subjected to further processing (e.g., reverse transcription, adapter attachment, amplification, clean up, sequencing). In other instances, the processing may occur in the partition. In some instances, one or more of the processing methods, e.g., reverse transcription, may occur in the partition. For example, conditions sufficient for barcoding, adapter attachment, reverse transcription, or other nucleic acid processing operations may be provided in the partition and performed prior to clean up and sequencing.

In various instances, a bead may comprise a capture sequence or binding sequence configured to bind to a corresponding capture sequence or binding sequence. In various instances, a bead may comprise a plurality of different capture sequences or binding sequences configured to bind to different respective corresponding capture sequences or binding sequences. For example, a bead may comprise a first subset of one or more capture sequences each configured to bind to a first corresponding capture sequence, a second subset of one or more capture sequences each configured to bind to a second corresponding capture sequence, a third subset of one or more capture sequences each configured to bind to a third corresponding capture sequence, etc. A bead may comprise any number of different capture sequences. In various instances, a bead may comprise at least 2, 3, 4, 5, 6, 7, 8, 9, 10 or more different capture sequences or binding sequences configured to bind to different respective capture sequences or binding sequences, respectively. Alternatively or in addition, a bead may comprise at most about 10, 9, 8, 7, 6, 5, 4, 3, or 2 different capture sequences or binding sequences configured to bind to different respective capture sequences or binding sequences. In various instances, the different capture sequences or binding sequences may be configured to facilitate analysis of a same type of analyte. In various instances, the different capture sequences or binding sequences may be configured to facilitate analysis of different types of analytes (with the same bead). The capture sequence may be designed to attach to a corresponding capture sequence. Beneficially, such corresponding capture sequence may be introduced to, or otherwise induced in, an biological particle (e.g., cell, cell bead, etc.) for performing different assays in various formats (e.g., barcoded antibodies comprising the corresponding capture sequence, barcoded MHC dextramers comprising the corresponding capture sequence, barcoded guide RNA molecules comprising the corresponding capture sequence, etc.), such that the corresponding capture sequence may later interact with the capture sequence associated with the bead. In various instances, a capture sequence coupled to a bead (or other support) may be configured to attach to a linker molecule, such as a splint molecule, wherein the linker molecule is configured to couple the bead (or other support) to other molecules through the linker molecule, such as to one or more analytes or one or more other linker molecules.

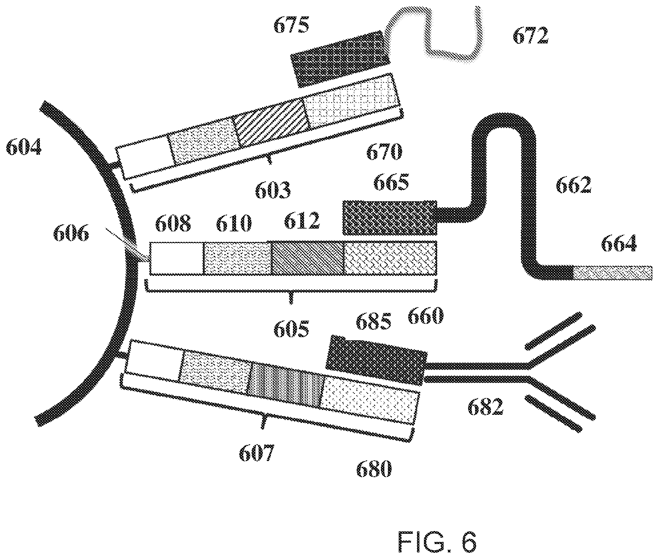

**FIG. 6** illustrates another example of a barcode carrying bead. A nucleic acid barcode molecule **605,** such as an oligonucleotide, can be coupled to a bead **604** by a releasable linkage **606,** such as, for example, a disulfide linker. The nucleic acid barcode molecule **605** may comprise a first capture sequence **660.** The same bead **604** may be coupled (e.g., via releasable linkage) to one or more other nucleic acid molecules **603, 607** comprising other capture sequences. The nucleic acid barcode molecule **605** may be or comprise a barcode. As noted elsewhere herein, the structure of the barcode may comprise a number of sequence elements, such as a functional sequence **608** (e.g., flow cell attachment sequence, sequencing primer sequence, etc.), a barcode sequence **610** (e.g., bead-specific sequence common to bead, partition-specific sequence common to partition, etc.), and a unique molecular identifier **612** (e.g., unique sequence within different molecules attached to the bead), or partial sequences thereof. The capture sequence **660** may be configured to attach to a corresponding capture sequence **665.** In various instances, the corresponding capture sequence **665** may be coupled to another molecule that may be an analyte or an intermediary carrier. For example, as illustrated in **FIG. 6****,** the corresponding capture sequence **665** is coupled to a guide RNA molecule **662** comprising a target sequence **664,** wherein the target sequence **664** is configured to attach to the analyte. Another oligonucleotide molecule **607** attached to the bead **604** comprises a second capture sequence **680** which is configured to attach to a second corresponding capture sequence **685.** As illustrated in **FIG. 6****,** the second corresponding capture sequence **685** is coupled to an antibody **682.** In various cases, the antibody **682** may have binding specificity to an analyte (e.g., surface protein). Alternatively, the antibody **682** may not have binding specificity. Another oligonucleotide molecule **603** attached to the bead **604** comprises a third capture sequence **670** which is configured to attach to a third corresponding capture sequence **675.** As illustrated in **FIG. 6****,** the third corresponding capture sequence **675** is coupled to a molecule **672.** The molecule **672** may or may not be configured to target an analyte. The other oligonucleotide molecules **603, 607** may comprise the other sequences (e.g., functional sequence, barcode sequence, UMI, etc.) described with respect to oligonucleotide molecule **605.** While a single oligonucleotide molecule comprising each capture sequence is illustrated in **FIG. 6****,** it will be appreciated that, for each capture sequence, the bead may comprise a set of one or more oligonucleotide molecules each comprising the capture sequence. For example, the bead may comprise any number of sets of one or more different capture sequences. Alternatively, or in addition, the bead **604** may comprise other capture sequences. Alternatively, or in addition, the bead **604** may comprise fewer types of capture sequences (e.g., two capture sequences). Alternatively, or in addition, the bead **604** may comprise oligonucleotide molecule(s) comprising a priming sequence, such as a specific priming sequence such as an mRNA specific priming sequence (e.g., poly-T sequence), a targeted priming sequence, and/or a random priming sequence, for example, to facilitate an assay for gene expression.

In operation, the barcoded oligonucleotides may be released (e.g., in a partition), as described elsewhere herein. Alternatively, the nucleic acid molecules bound to the bead (e.g., gel bead) may be used to hybridize and capture analytes (e.g., one or more types of analytes) on the solid phase of the bead.

A bead injected or otherwise introduced into a partition may comprise releasably, cleavably, or reversibly attached barcodes. A bead injected or otherwise introduced into a partition may comprise activatable barcodes. A bead injected or otherwise introduced into a partition may be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage may be achieved through reduction of di-sulfide bonds, use of restriction enzymes, photo-activated cleavage, or cleavage via other types of stimuli (e.g., chemical, thermal, pH, enzymatic, etc.) and/or reactions, such as described elsewhere herein. Releasable barcodes may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

As will be appreciated from the above disclosure, the degradation of a bead may refer to the disassociation of a bound or entrained species from a bead, both with and without structurally degrading the physical bead itself. For example, the degradation of the bead may involve cleavage of a cleavable linkage via one or more species and/or methods described elsewhere herein. In another example, entrained species may be released from beads through osmotic pressure differences due to, for example, changing chemical environments. See, e.g., PCT/US2014/044398.

A degradable bead may be introduced into a partition, such as a droplet of an emulsion or a well, such that the bead degrades within the partition and any associated species (e.g., oligonucleotides) are released within the droplet when the appropriate stimulus is applied. The free species (e.g., oligonucleotides, nucleic acid molecules) may interact with other reagents contained in the partition. See, e.g., PCT/US2014/044398.

As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

In various cases, a species (e.g., oligonucleotide molecules comprising barcodes) that are attached to a solid support (e.g., a bead) may comprise a U-excising element that allows the species to release from the bead. In various cases, the U-excising element may comprise a single-stranded DNA (ssDNA) sequence that contains at least one uracil. The species may be attached to a solid support via the ssDNA sequence containing the at least one uracil. The species may be released by a combination of uracil-DNA glycosylase (e.g., to remove the uracil) and an endonuclease (e.g., to induce an ssDNA break). If the endonuclease generates a 5' phosphate group from the cleavage, then additional enzyme treatment may be included in downstream processing to eliminate the phosphate group, e.g., prior to ligation of additional sequencing handle elements, e.g., Illumina full P5 sequence, partial P5 sequence, full R1 sequence, and/or partial R1 sequence.

The barcodes that are releasable as described herein may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

The nucleic acid barcode sequences can include from about 6 to about 20 or more nucleotides within the sequence of the nucleic acid molecules (e.g., oligonucleotides). The nucleic acid barcode sequences can include from about 6 to about 20, 30, 40, 50, 60, 70, 80, 90, 100 or more nucleotides. In various cases, the length of a barcode sequence may be about 6, 7, 8, 9, 10, 11, 12, 13, 14, 15, 16, 17, 18, 19, 20 nucleotides or longer. In various cases, the length of a barcode sequence may be at least about 6, 7, 8, 9, 10, 11, 12, 13, 14, 15, 16, 17, 18, 19, 20 nucleotides or longer. In various cases, the length of a barcode sequence may be at most about 6, 7, 8, 9, 10, 11, 12, 13, 14, 15, 16, 17, 18, 19, 20 nucleotides or shorter. These nucleotides may be completely contiguous, i.e., in a single stretch of adjacent nucleotides, or they may be separated into two or more separate subsequences that are separated by 1 or more nucleotides. In various cases, separated barcode subsequences can be from about 4 to about 16 nucleotides in length. In various cases, the barcode subsequence may be about 4, 5, 6, 7, 8, 9, 10, 11, 12, 13, 14, 15, 16 nucleotides or longer. In various cases, the barcode subsequence may be at least about 4, 5, 6, 7, 8, 9, 10, 11, 12, 13, 14, 15, 16 nucleotides or longer. In various cases, the barcode subsequence may be at most about 4, 5, 6, 7, 8, 9, 10, 11, 12, 13, 14, 15, 16 nucleotides or shorter.

The co-partitioned nucleic acid molecules can also comprise other functional sequences useful in the processing of the nucleic acids from the co-partitioned biological particles. These sequences include, e.g., targeted or random/universal amplification primer sequences for amplifying nucleic acids (e.g., mRNA, the genomic DNA) from the individual biological particles within the partitions while attaching the associated barcode sequences, sequencing primers or primer recognition sites, hybridization or probing sequences, e.g., for identification of presence of the sequences or for pulling down barcoded nucleic acids, or any of a number of other potential functional sequences. Other mechanisms of co-partitioning oligonucleotides may also be employed, including, e.g., coalescence of two or more droplets, where one droplet contains oligonucleotides, or microdispensing of oligonucleotides (e.g., attached to a bead) into partitions, e.g., droplets within microfluidic systems.

In an example, beads, such as beads, are provided that each include large numbers of the above described nucleic acid barcode molecules releasably attached to the beads, where all of the nucleic acid barcode molecules attached to a particular bead will include a common nucleic acid barcode sequence, but where a large number of diverse barcode sequences are represented across the population of beads used. In various embodiments, hydrogel beads, e.g., comprising polyacrylamide polymer matrices, are used as a solid support and delivery vehicle for the nucleic acid barcode molecules into the partitions, as they are capable of carrying large numbers of nucleic acid barcode molecules, and may be configured to release those nucleic acid molecules upon exposure to a particular stimulus, as described elsewhere herein. In various cases, the population of beads provides a diverse barcode sequence library that includes at least about 1,000 different barcode sequences, at least about 5,000 different barcode sequences, at least about 10,000 different barcode sequences, at least about 50,000 different barcode sequences, at least about 100,000 different barcode sequences, at least about 1,000,000 different barcode sequences, at least about 5,000,000 different barcode sequences, or at least about 10,000,000 different barcode sequences, or more. In various cases, the population of beads provides a diverse barcode sequence library that includes about 1,000 to about 10,000 different barcode sequences, about 5,000 to about 50,000 different barcode sequences, about 10,000 to about 100,000 different barcode sequences, about 50,000 to about 1,000,000 different barcode sequences, or about 100,000 to about 10,000,000 different barcode sequences.

Additionally, each bead can be provided with large numbers of nucleic acid (e.g., oligonucleotide) molecules attached. In particular, the number of molecules of nucleic acid molecules including the barcode sequence on an individual bead can be at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in various cases at least about 1 billion nucleic acid molecules, or more. In various embodiments, the number of nucleic acid molecules including the barcode sequence on an individual bead is between about 1,000 to about 10,000 nucleic acid molecules, about 5,000 to about 50,000 nucleic acid molecules, about 10,000 to about 100,000 nucleic acid molecules, about 50,000 to about 1,000,000 nucleic acid molecules, about 100,000 to about 10,000,000 nucleic acid molecules, about 1,000,000 to about 1 billion nucleic acid molecules.

Nucleic acid molecules of a given bead can include identical (or common) barcode sequences, different barcode sequences, or a combination of both. Nucleic acid molecules of a given bead can include multiple sets of nucleic acid molecules. Nucleic acid molecules of a given set can include identical barcode sequences. The identical barcode sequences can be different from barcode sequences of nucleic acid molecules of another set. In some embodiments, such different barcode sequences can be associated with a given bead.

Moreover, when the population of beads is partitioned, the resulting population of partitions can also include a diverse barcode library that includes at least about 1,000 different barcode sequences, at least about 5,000 different barcode sequences, at least about 10,000 different barcode sequences, at least at least about 50,000 different barcode sequences, at least about 100,000 different barcode sequences, at least about 1,000,000 different barcode sequences, at least about 5,000,000 different barcode sequences, or at least about 10,000,000 different barcode sequences. Additionally, each partition of the population can include at least about 1,000 nucleic acid barcode molecules, at least about 5,000 nucleic acid barcode molecules, at least about 10,000 nucleic acid barcode molecules, at least about 50,000 nucleic acid barcode molecules, at least about 100,000 nucleic acid barcode molecules, at least about 500,000 nucleic acids, at least about 1,000,000 nucleic acid barcode molecules, at least about 5,000,000 nucleic acid barcode molecules, at least about 10,000,000 nucleic acid barcode molecules, at least about 50,000,000 nucleic acid barcode molecules, at least about 100,000,000 nucleic acid barcode molecules, at least about 250,000,000 nucleic acid barcode molecules and in various cases at least about 1 billion nucleic acid barcode molecules.

In various cases, the resulting population of partitions provides a diverse barcode sequence library that includes about 1,000 to about 10,000 different barcode sequences, about 5,000 to about 50,000 different barcode sequences, about 10,000 to about 100,000 different barcode sequences, about 50,000 to about 1,000,000 different barcode sequences, or about 100,000 to about 10,000,000 different barcode sequences. Additionally, each partition of the population can include between about 1,000 to about 10,000 nucleic acid barcode molecules, about 5,000 to about 50,000 nucleic acid barcode molecules, about 10,000 to about 100,000 nucleic acid barcode molecules, about 50,000 to about 1,000,000 nucleic acid barcode molecules, about 100,000 to about 10,000,000 nucleic acid barcode molecules, about 1,000,000 to about 1 billion nucleic acid barcode molecules.

In various cases, it may be desirable to incorporate multiple different barcodes within a given partition, either attached to a single or multiple beads within the partition. For example, in various cases, a mixed, but known set of barcode sequences may provide greater assurance of identification in the subsequent processing, e.g., by providing a stronger address or attribution of the barcodes to a given partition, as a duplicate or independent confirmation of the output from a given partition.

The nucleic acid molecules (e.g., oligonucleotides) are releasable from the beads upon the application of a particular stimulus to the beads. In various cases, the stimulus may be a photo-stimulus, e.g., through cleavage of a photo-labile linkage that releases the nucleic acid molecules. In other cases, a thermal stimulus may be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of biological particles, and may be degraded for release of the attached nucleic acid molecules through exposure to a reducing agent, such as DTT.

### Reagents:

In accordance with certain aspects, biological particles may be partitioned along with lysis reagents in order to release the contents of the biological particles within the partition. In such cases, the lysis agents can be contacted with the biological particle suspension concurrently with, or immediately prior to, the introduction of the biological particles into the partitioning junction/droplet generation zone (e.g., junction 410), such as through an additional channel or channels upstream of the channel junction. In accordance with other aspects, additionally or alternatively, biological particles may be partitioned along with other reagents, as will be described further below.

The methods and systems of the present disclosure may comprise microfluidic devices and methods of use thereof, which may be used for co-partitioning biological particles with reagents. Such systems and methods are described in U.S. Patent Publication No. US/20190367997.

Beneficially, when lysis reagents and biological particles are co-partitioned, the lysis reagents can facilitate the release of the contents of the biological particles within the partition. The contents released in a partition may remain discrete from the contents of other partitions.

As will be appreciated, the channel segments of the microfluidic devices described elsewhere herein may be coupled to any of a variety of different fluid sources or receiving components, including reservoirs, tubing, manifolds, or fluidic components of other systems. As will be appreciated, the microfluidic channel structures may have various geometries and/or configurations. For example, a microfluidic channel structure can have more than two channel junctions. For example, a microfluidic channel structure can have 2, 3, 4, 5 channel segments or more each carrying the same or different types of beads, reagents, and/or biological particles that meet at a channel junction. Fluid flow in each channel segment may be controlled to control the partitioning of the different elements into droplets. Fluid may be directed flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), as well as other commercially available lysis enzymes. Other lysis agents may additionally or alternatively be co-partitioned with the biological particles to cause the release of the biological particle's contents into the partitions. For example, in various cases, surfactant-based lysis solutions may be used to lyse cells, although these may be less desirable for emulsion-based systems where the surfactants can interfere with stable emulsions. In various cases, lysis solutions may include non-ionic surfactants such as, for example, TritonX-100 and Tween 20. In various cases, lysis solutions may include ionic surfactants such as, for example, sarcosyl and sodium dodecyl sulfate (SDS). Electroporation, thermal, acoustic or mechanical cellular disruption may also be used in certain cases, e.g., non-emulsion-based partitioning such as encapsulation of biological particles that may be in addition to or in place of droplet partitioning, where any pore size of the encapsulate is sufficiently small to retain nucleic acid fragments of a given size, following cellular disruption.

Alternatively or in addition to the lysis agents co-partitioned with the biological particles described above, other reagents can also be co-partitioned with the biological particles, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated biological particles (e.g., a cell or a nucleus in a polymer matrix), the biological particles may be exposed to an appropriate stimulus to release the biological particles or their contents from a co-partitioned bead. For example, in various cases, a chemical stimulus may be co-partitioned along with an encapsulated biological particle to allow for the degradation of the bead and release of the cell or its contents into the larger partition. In various cases, this stimulus may be the same as the stimulus described elsewhere herein for release of nucleic acid molecules (e.g., oligonucleotides) from their respective bead. In alternative examples, this may be a different and non-overlapping stimulus, in order to allow an encapsulated biological particle to be released into a partition at a different time from the release of nucleic acid molecules into the same partition. For a description of methods, compositions, and systems for encapsulating cells (also referred to as a "cell bead"), see, e.g., U.S. Pat. 10,428,326 and U.S. Pat. Pub. No. 20190100632.

Additional reagents may also be co-partitioned with the biological particle, such as endonucleases to fragment a biological particle's DNA, DNA polymerase enzymes and dNTPs used to amplify the biological particle's nucleic acid fragments and to attach the barcode molecular tags to the amplified fragments. Other enzymes may be co-partitioned, including without limitation, polymerase, transposase, ligase, proteinase K, DNAse, etc. Additional reagents may also include reverse transcriptase enzymes, including enzymes with terminal transferase activity, primers and oligonucleotides, and switch oligonucleotides (also referred to herein as "switch oligos" or "template switching oligonucleotides") which can be used for template switching.

In various cases, template switching can be used to increase the length of a cDNA. In various cases, template switching can be used to append a predefined nucleic acid sequence to the cDNA. Template switching is further described in PCT/US2017/068320. Template switching oligonucleotides may comprise a hybridization region and a template region. Template switching oligonucleotides are further described in PCT/US2017/068320.

Any of the reagents described in this disclosure may be encapsulated in, or otherwise coupled to, a droplet, or bead, with any chemicals, particles, and elements suitable for sample processing reactions involving biomolecules, such as, but not limited to, nucleic acid molecules and proteins. For example, a bead or droplet used in a sample preparation reaction for DNA sequencing may comprise one or more of the following reagents: enzymes, restriction enzymes (e.g., multiple cutters), ligase, polymerase, fluorophores, oligonucleotide barcodes, adapters, buffers, nucleotides (e.g., dNTPs, ddNTPs) and the like.

Additional examples of reagents include, but are not limited to: buffers, acidic solution, basic solution, temperature-sensitive enzymes, pH-sensitive enzymes, light-sensitive enzymes, metals, metal ions, magnesium chloride, sodium chloride, manganese, aqueous buffer, mild buffer, ionic buffer, inhibitor, enzyme, protein, polynucleotide, antibodies, saccharides, lipid, oil, salt, ion, detergents, ionic detergents, non-ionic detergents, and oligonucleotides.

Once the contents of the cells are released into their respective partitions, the macromolecular components (e.g., macromolecular constituents of biological particles, such as RNA, DNA, or proteins) contained therein may be further processed within the partitions. In accordance with the methods and systems described herein, the macromolecular component contents of individual biological particles can be provided with unique identifiers such that, upon characterization of those macromolecular components they may be attributed as having been derived from the same biological particle or particles. The ability to attribute characteristics to individual biological particles or groups of biological particles is provided by the assignment of unique identifiers specifically to an individual biological particle or groups of biological particles. Unique identifiers, e.g., in the form of nucleic acid barcodes can be assigned or associated with individual biological particles or populations of biological particles, in order to tag or label the biological particle's macromolecular components (and as a result, its characteristics) with the unique identifiers. These unique identifiers can then be used to attribute the biological particle's components and characteristics to an individual biological particle or group of biological particles. In various aspects, this is performed by co-partitioning the individual biological particle or groups of biological particles with the unique identifiers, such as described above (with reference to **FIGS.** 3 or 4).

In various cases, additional beads can be used to deliver additional reagents to a partition. In such cases, it may be advantageous to introduce different beads into a common channel or droplet generation junction, from different bead sources (e.g., containing different associated reagents) through different channel inlets into such common channel or droplet generation junction. In such cases, the flow and frequency of the different beads into the channel or junction may be controlled to provide for a certain ratio of beads from each source, while ensuring a given pairing or combination of such beads into a partition with a given number of biological particles (e.g., one biological particle and one bead per partition).

In some embodiments, following the generation of barcoded nucleic acid molecules according to methods disclosed herein, subsequent operations that can be performed can include generation of amplification products, purification (e.g., via solid phase reversible immobilization (SPRI)), further processing (e.g., shearing, ligation of functional sequences, and subsequent amplification (e.g., via PCR)). These operations may occur in bulk (e.g., outside the partition). In the case where a partition is a droplet in an emulsion, the emulsion can be broken and the contents of the droplet pooled for additional operations.

### Wells:

As described herein, one or more processes may be performed in a partition, which may be a well. The well may be a well of a plurality of wells of a substrate, such as a microwell of a microwell array or plate, or the well may be a microwell or microchamber of a device (e.g., microfluidic device) comprising a substrate. The well may be a well of a well array or plate, or the well may be a well or chamber of a device (e.g., fluidic device). In some embodiments, a well of a fluidic device is fluidically connected to another well of the fluidic device. Accordingly, the wells or microwells may assume an "open" configuration, in which the wells or microwells are exposed to the environment (e.g., contain an open surface) and are accessible on one planar face of the substrate, or the wells or microwells may assume a "closed" or "sealed" configuration, in which the microwells are not accessible on a planar face of the substrate. In various instances, the wells or microwells may be configured to toggle between "open" and "closed" configurations. For instance, an "open" microwell or set of microwells may be "closed" or "sealed" using a membrane (e.g., semi-permeable membrane), an oil (e.g., fluorinated oil to cover an aqueous solution), or a lid, as described elsewhere herein.

The well may have a volume of less than 1 milliliter (mL). For instance, the well may be configured to hold a volume of at most 1000 microliters (µL), at most 100 µL, at most 10 µL, at most 1 µL, at most 100 nanoliters (nL), at most 10 nL, at most 1 nL, at most 100 picoliters (pL), at most 10 (pL), or less. The well may be configured to hold a volume of about 1000 µL, about 100 µL, about 10 µL, about 1 µL, about 100 nL, about 10 nL, about 1 nL, about 100 pL, about 10 pL, etc. The well may be configured to hold a volume of at least 10 pL, at least 100 pL, at least 1 nL, at least 10 nL, at least 100 nL, at least 1 µL, at least 10 µL, at least 100 µL, at least 1000 µL, or more. The well may be configured to hold a volume in a range of volumes listed herein, for example, from about 5 nL to about 20 nL, from about 1 nL to about 100 nL, from about 500 pL to about 100 µL, etc. The well may be of a plurality of wells that have varying volumes and may be configured to hold a volume appropriate to accommodate any of the partition volumes described herein.

In various instances, a microwell array or plate comprises a single variety of microwells. In various instances, a microwell array or plate comprises a variety of microwells. For instance, the microwell array or plate may comprise one or more types of microwells within a single microwell array or plate. The types of microwells may have different dimensions (e.g., length, width, diameter, depth, cross-sectional area, etc.), shapes (e.g., circular, triangular, square, rectangular, pentagonal, hexagonal, heptagonal, octagonal, nonagonal, decagonal, etc.), aspect ratios, or other physical characteristics. The microwell array or plate may comprise any number of different types of microwells. For example, the microwell array or plate may comprise 1, 2, 3, 4, 5, 6, 7, 8, 9, 10, 20, 30, 40, 50, 60, 70, 80, 90, 100, 200, 300, 400, 500, 600, 700, 800, 900, 1000 or more different types of microwells. A well may have any dimension (e.g., length, width, diameter, depth, cross-sectional area, volume, etc.), shape (e.g., circular, triangular, square, rectangular, pentagonal, hexagonal, heptagonal, octagonal, nonagonal, decagonal, other polygonal, etc.), aspect ratios, or other physical characteristics described herein with respect to any well.

In certain instances, the microwell array or plate comprises different types of microwells that are located adjacent to one another within the array or plate. For instance, a microwell with one set of dimensions may be located adjacent to and in contact with another microwell with a different set of dimensions. Similarly, microwells of different geometries may be placed adjacent to or in contact with one another. The adjacent microwells may be configured to hold different articles; for example, one microwell may be used to contain a cell, cell bead, or other sample (e.g., cellular components, nucleic acid molecules, etc.) while the adjacent microwell may be used to contain a droplet, bead, or other reagent. In various cases, the adjacent microwells may be configured to merge the contents held within, e.g., upon application of a stimulus, or spontaneously, upon contact of the articles in each microwell.

As is described elsewhere herein, a plurality of partitions may be used in the systems, compositions, and methods described herein. For example, any suitable number of partitions (e.g., wells or droplets) can be generated or otherwise provided. For example, in the case when wells are used, at least about 1,000 wells, at least about 5,000 wells, at least about 10,000 wells, at least about 50,000 wells, at least about 100,000 wells, at least about 500,000 wells, at least about 1,000,000 wells, at least about 5,000,000 wells at least about 10,000,000 wells, at least about 50,000,000 wells, at least about 100,000,000 wells, at least about 500,000,000 wells, at least about 1,000,000,000 wells, or more wells can be generated or otherwise provided. Moreover, the plurality of wells may comprise both unoccupied wells (e.g., empty wells) and occupied wells.

A well may comprise any of the reagents described herein, or combinations thereof. These reagents may include, for example, barcode molecules, enzymes, adapters, and combinations thereof. The reagents may be physically separated from a sample (e.g., a cell, cell bead, or cellular components, e.g., proteins, nucleic acid molecules, etc.) that is placed in the well. This physical separation may be accomplished by containing the reagents within, or coupling to, a bead that is placed within a well. The physical separation may also be accomplished by dispensing the reagents in the well and overlaying the reagents with a layer that is, for example, dissolvable, meltable, or permeable prior to introducing the polynucleotide sample into the well. This layer may be, for example, an oil, wax, membrane (e.g., semi-permeable membrane), or the like. The well may be sealed at any point, for example, after addition of the bead, after addition of the reagents, or after addition of either of these components. The sealing of the well may be useful for a variety of purposes, including preventing escape of beads or loaded reagents from the well, permitting select delivery of certain reagents (e.g., via the use of a semi-permeable membrane), for storage of the well prior to or following further processing, etc.

Once sealed, the well may be subjected to conditions for further processing of a cell (or cells) in the well. For instance, reagents in the well may allow further processing of the cell, e.g., cell lysis, as further described herein. Alternatively, the well (or wells such as those of a well-based array) comprising the cell (or cells) may be subjected to freeze-thaw cycling to process the cell (or cells), e.g., cell lysis. The well containing the cell may be subjected to freezing temperatures (e.g., 0°C, below 0°C, -5°C, -10°C, -15°C, -20°C, -25°C, -30°C, -35°C, -40°C, -45°C, -50°C, -55°C, -60°C, -65°C, -70°C, -80°C, or -85°C). Freezing may be performed in a suitable manner, e.g., sub-zero freezer or a dry ice/ethanol bath. Following an initial freezing, the well (or wells) comprising the cell (or cells) may be subjected to freeze-thaw cycles to lyse the cell (or cells). In one embodiment, the initially frozen well (or wells) are thawed to a temperature above freezing (e.g., 4°C or above, 8°C or above, 12°C or above, 16°C or above, 20°C or above, room temperature, or 25°C or above). In another embodiment, the freezing is performed for less than 10 minutes (e.g., 5 minutes or 7 minutes) followed by thawing at room temperature for less than 10 minutes (e.g., 5 minutes or 7 minutes). This freeze-thaw cycle may be repeated a number of times, e.g., 2, 3, 4 or more times, to obtain lysis of the cell (or cells) in the well (or wells). In one embodiment, the freezing, thawing and/or freeze/thaw cycling is performed in the absence of a lysis buffer. Additional disclosure related to freeze-thaw cycling is provided in WO2019165181A1.

A well may comprise free reagents and/or reagents encapsulated in, or otherwise coupled to or associated with, beads, beads, or droplets.

The wells may be provided as a part of a kit. For example, a kit may comprise instructions for use, a microwell array or device, and reagents (e.g., beads). The kit may comprise any useful reagents for performing the processes described herein, e.g., nucleic acid reactions, barcoding of nucleic acid molecules, sample processing (e.g., for cell lysis, fixation, and/or permeabilization).

In various cases, a well comprises a bead, or droplet that comprises a set of reagents that has a similar attribute (e.g., a set of enzymes, a set of minerals, a set of oligonucleotides, a mixture of different barcode molecules, a mixture of identical barcode molecules). In other cases, a bead or droplet comprises a heterogeneous mixture of reagents. In various cases, the heterogeneous mixture of reagents can comprise all components necessary to perform a reaction. In various cases, such mixture can comprise all components necessary to perform a reaction, except for 1, 2, 3, 4, 5, or more components necessary to perform a reaction. In various cases, such additional components are contained within, or otherwise coupled to, a different droplet or bead, or within a solution within a partition (e.g., microwell) of the system.

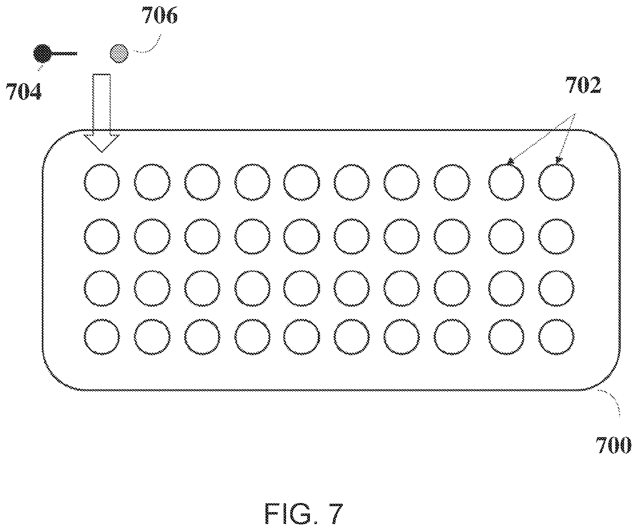

**FIG. 7** schematically illustrates an example of a microwell array. The array can be contained within a substrate **700.** The substrate **700** comprises a plurality of wells **702.** The wells **702** may be of any size or shape, and the spacing between the wells, the number of wells per substrate, as well as the density of the wells on the substrate **700** can be modified, depending on the particular application. In one such example application, a sample molecule **706,** which may comprise a cell or cellular components (e.g., nucleic acid molecules) is co-partitioned with a bead **704,** which may comprise a nucleic acid barcode molecule coupled thereto. The wells **702** may be loaded using gravity or other loading technique (e.g., centrifugation, liquid handler, acoustic loading, optoelectronic, etc.). In various instances, at least one of the wells **702** contains a single sample molecule **706** (e.g., cell) and a single bead **704.**

Reagents may be loaded into a well either sequentially or concurrently. In various cases, reagents are introduced to the device either before or after a particular operation. In various cases, reagents (which may be provided, in certain instances, in droplets, or beads) are introduced sequentially such that different reactions or operations occur at different steps. The reagents (or droplets, or beads) may also be loaded at operations interspersed with a reaction or operation step. For example, beads (or droplets) comprising reagents for fragmenting polynucleotides (e.g., restriction enzymes) and/or other enzymes (e.g., transposases, ligases, polymerases, etc.) may be loaded into the well or plurality of wells, followed by loading of droplets, or beads comprising reagents for attaching nucleic acid barcode molecules to a sample nucleic acid molecule. Reagents may be provided concurrently or sequentially with a sample, e.g., a cell or cellular components (e.g., organelles, proteins, nucleic acid molecules, carbohydrates, lipids, etc.). Accordingly, use of wells may be useful in performing multi-step operations or reactions.

As described elsewhere herein, the nucleic acid barcode molecules and other reagents may be contained within a bead, or droplet. These beads, or droplets may be loaded into a partition (e.g., a microwell) before, after, or concurrently with the loading of a cell, such that each cell is contacted with a different bead, or droplet. This technique may be used to attach a unique nucleic acid barcode molecule to nucleic acid molecules obtained from each cell. Alternatively, or in addition to, the sample nucleic acid molecules may be attached to a support. For instance, the partition (e.g., microwell) may comprise a bead which has coupled thereto a plurality of nucleic acid barcode molecules. The sample nucleic acid molecules, or derivatives thereof, may couple or attach to the nucleic acid barcode molecules on the support. The resulting barcoded nucleic acid molecules may then be removed from the partition, and in various instances, pooled and sequenced. In such cases, the nucleic acid barcode sequences may be used to trace the origin of the sample nucleic acid molecule. For example, polynucleotides with identical barcodes may be determined to originate from the same cell or partition, while polynucleotides with different barcodes may be determined to originate from different cells or partitions.

The samples or reagents may be loaded in the wells or microwells using a variety of approaches. The samples (e.g., a cell, cell bead, or cellular component) or reagents (as described herein) may be loaded into the well or microwell using an external force, e.g., gravitational force, electrical force, magnetic force, or using mechanisms to drive the sample or reagents into the well, e.g., via pressure-driven flow, centrifugation, optoelectronics, acoustic loading, electrokinetic pumping, vacuum, capillary flow, etc. In certain cases, a fluid handling system may be used to load the samples or reagents into the well. The loading of the samples or reagents may follow a Poissonian distribution or a non-Poissonian distribution, e.g., super Poisson or sub-Poisson. The geometry, spacing between wells, density, and size of the microwells may be modified to accommodate a useful sample or reagent distribution; for instance, the size and spacing of the microwells may be adjusted such that the sample or reagents may be distributed in a super-Poissonian fashion.

In one particular non-limiting example, the microwell array or plate comprises pairs of microwells, in which each pair of microwells is configured to hold a droplet (e.g., comprising a single cell) and a single bead (such as those described herein, which may, in various instances, also be encapsulated in a droplet). The droplet and the bead (or droplet containing the bead) may be loaded simultaneously or sequentially, and the droplet and the bead may be merged, e.g., upon contact of the droplet and the bead, or upon application of a stimulus (e.g., external force, agitation, heat, light, magnetic or electric force, etc.). In various cases, the loading of the droplet and the bead is super-Poissonian. In other examples of pairs of microwells, the wells are configured to hold two droplets comprising different reagents and/or samples, which are merged upon contact or upon application of a stimulus. In such instances, the droplet of one microwell of the pair can comprise reagents that may react with an agent in the droplet of the other microwell of the pair. For instance, one droplet can comprise reagents that are configured to release the nucleic acid barcode molecules of a bead contained in another droplet, located in the adjacent microwell. Upon merging of the droplets, the nucleic acid barcode molecules may be released from the bead into the partition (e.g., the microwell or microwell pair that are in contact), and further processing may be performed (e.g., barcoding, nucleic acid reactions, etc.). In cases where intact or live cells are loaded in the microwells, one of the droplets may comprise lysis reagents for lysing the cell upon droplet merging.

A droplet or bead may be partitioned into a well. The droplets may be selected or subjected to pre-processing prior to loading into a well. For instance, the droplets may comprise cells, and only certain droplets, such as those containing a single cell (or at least one cell), may be selected for use in loading of the wells. Such a pre-selection process may be useful in efficient loading of single cells, such as to obtain a non-Poissonian distribution, or to pre-filter cells for a selected characteristic prior to further partitioning in the wells. Additionally, the technique may be useful in obtaining or preventing cell doublet or multiplet formation prior to or during loading of the microwell.

In various instances, the wells can comprise nucleic acid barcode molecules attached thereto. The nucleic acid barcode molecules may be attached to a surface of the well (e.g., a wall of the well). The nucleic acid barcode molecules may be attached to a droplet or bead that has been partitioned into the well. The nucleic acid barcode molecule (e.g., a partition barcode sequence) of one well may differ from the nucleic acid barcode molecule of another well, which can permit identification of the contents contained with a single partition or well. In various cases, the nucleic acid barcode molecule can comprise a spatial barcode sequence that can identify a spatial coordinate of a well, such as within the well array or well plate. In various cases, the nucleic acid barcode molecule can comprise a unique molecular identifier for individual molecule identification. In various instances, the nucleic acid barcode molecules may be configured to attach to or capture a nucleic acid molecule within a sample or cell distributed in the well. For example, the nucleic acid barcode molecules may comprise a capture sequence that may be used to capture or hybridize to a nucleic acid molecule (e.g., RNA, DNA) within the sample. In various instances, the nucleic acid barcode molecules may be releasable from the microwell. In some instances, the nucleic acid barcode molecules may be releasable from the bead or droplet. For instance, the nucleic acid barcode molecules may comprise a chemical cross-linker which may be cleaved upon application of a stimulus (e.g., photo-, magnetic, chemical, biological, stimulus). The released nucleic acid barcode molecules, which may be hybridized or configured to hybridize to a sample nucleic acid molecule, may be collected and pooled for further processing, which can include nucleic acid processing (e.g., amplification, extension, reverse transcription, etc.) and/or characterization (e.g., sequencing). In some instances nucleic acid barcode molecules attached to a bead or droplet in a well may be hybridized to sample nucleic acid molecules, and the bead with the sample nucleic acid molecules hybridized thereto may be collected and pooled for further processing, which can include nucleic acid processing (e.g., amplification, extension, reverse transcription, etc.) and/or characterization (e.g., sequencing). In such cases, the unique partition barcode sequences may be used to identify the cell or partition from which a nucleic acid molecule originated.

Characterization of samples within a well may be performed. Such characterization can include, in non-limiting examples, imaging of the sample (e.g., cell, cell bead, or cellular components) or derivatives thereof. Characterization techniques such as microscopy or imaging may be useful in measuring sample profiles in fixed spatial locations. For instance, when cells are partitioned, optionally with beads, imaging of each microwell and the contents contained therein may provide useful information on cell doublet formation (e.g., frequency, spatial locations, etc.), cell-bead pair efficiency, cell viability, cell size, cell morphology, expression level of a biomarker (e.g., a surface marker, a fluorescently labeled molecule therein, etc.), cell or bead loading rate, number of cell-bead pairs, etc. In various instances, imaging may be used to characterize live cells in the wells, including, but not limited to: dynamic live-cell tracking, cell-cell interactions (when two or more cells are co-partitioned), cell proliferation, etc. Alternatively, or in addition to, imaging may be used to characterize a quantity of amplification products in the well.

In operation, a well may be loaded with a sample and reagents, simultaneously or sequentially. When cells or cell beads are loaded, the well may be subjected to washing, e.g., to remove excess cells from the well, microwell array, or plate. Similarly, washing may be performed to remove excess beads or other reagents from the well, microwell array, or plate. In the instances where live cells are used, the cells may be lysed in the individual partitions to release the intracellular components or cellular analytes. Alternatively, the cells may be fixed or permeabilized in the individual partitions. The intracellular components or cellular analytes may couple to a support, e.g., on a surface of the microwell, on a solid support (e.g., bead), or they may be collected for further downstream processing. For instance, after cell lysis, the intracellular components or cellular analytes may be transferred to individual droplets or other partitions for barcoding. Alternatively, or in addition to, the intracellular components or cellular analytes (e.g., nucleic acid molecules) may couple to a bead comprising a nucleic acid barcode molecule; subsequently, the bead may be collected and further processed, e.g., subjected to nucleic acid reaction such as reverse transcription, amplification, or extension, and the nucleic acid molecules thereon may be further characterized, e.g., via sequencing. Alternatively, or in addition to, the intracellular components or cellular analytes may be barcoded in the well (e.g., using a bead comprising nucleic acid barcode molecules that are releasable or on a surface of the microwell comprising nucleic acid barcode molecules). The barcoded nucleic acid molecules or analytes may be further processed in the well, or the barcoded nucleic acid molecules or analytes may be collected from the individual partitions and subjected to further processing outside the partition. Further processing can include nucleic acid processing (e.g., performing an amplification, extension) or characterization (e.g., fluorescence monitoring of amplified molecules, sequencing). At any convenient or useful step, the well (or microwell array or plate) may be sealed (e.g., using an oil, membrane, wax, etc.), which enables storage of the assay or selective introduction of additional reagents.

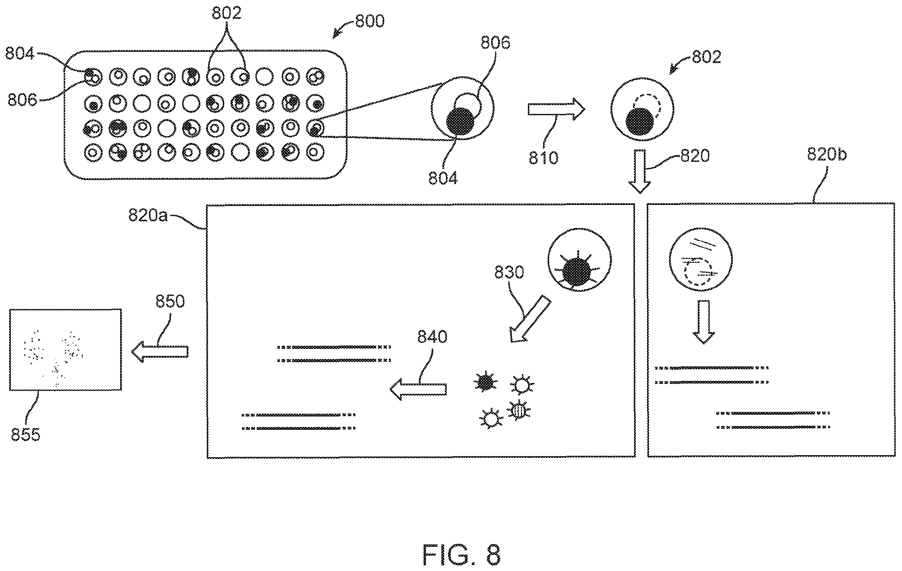

**FIG. 8** schematically shows an example workflow for processing nucleic acid molecules within a sample. A substrate **800** comprising a plurality of microwells **802** may be provided. A sample **806** which may comprise a cell, cell bead, cellular components or analytes (e.g., proteins and/or nucleic acid molecules) can be co-partitioned, in a plurality of microwells **802,** with a plurality of beads **804** comprising nucleic acid barcode molecules. During process **810,** the sample **806** may be processed within the partition. For instance, in the case of live cells, the cell may be subjected to conditions sufficient to lyse the cells and release the analytes contained therein. In process **820,** the bead **804** may be further processed. By way of example, processes **820a** and **820b** schematically illustrate different workflows, depending on the properties of the bead **804.**

In **820a,** the bead comprises nucleic acid barcode molecules that are attached thereto, and sample nucleic acid molecules (e.g., RNA, DNA) may attach, e.g., via hybridization of ligation, to the nucleic acid barcode molecules. Such attachment may occur on the bead. In process **830,** the beads **804** from multiple wells **802** may be collected and pooled. Further processing may be performed in process **840.** For example, one or more nucleic acid reactions may be performed, such as reverse transcription, nucleic acid extension, amplification, ligation, transposition, etc. In various instances, adapter sequences are ligated to the nucleic acid molecules, or derivatives thereof, as described elsewhere herein. For instance, sequencing primer sequences may be appended to each end of the nucleic acid molecule. In process **850,** further characterization, such as sequencing may be performed to generate sequencing reads. The sequencing reads may yield information on individual cells or populations of cells, which may be represented visually or graphically, e.g., in a plot **855.**

In **820b,** the bead comprises nucleic acid barcode molecules that are releasably attached thereto, as described below. The bead may degrade or otherwise release the nucleic acid barcode molecules into the well **802;** the nucleic acid barcode molecules may then be used to barcode nucleic acid molecules within the well **802.** Further processing may be performed either inside the partition or outside the partition. For example, one or more nucleic acid reactions may be performed, such as reverse transcription, nucleic acid extension, amplification, ligation, transposition, etc. In various instances, adapter sequences are ligated to the nucleic acid molecules, or derivatives thereof, as described elsewhere herein. For instance, sequencing primer sequences may be appended to each end of the nucleic acid molecule. In process 650, further characterization, such as sequencing may be performed to generate sequencing reads. The sequencing reads may yield information on individual cells or populations of cells, which may be represented visually or graphically, e.g., in a plot **855.**

### Targeted Enrichment

The methods provided herein may comprise the use of a targeting process to, for example, enrich selected nucleic acid molecules within a sample.

An exemplary target enrichment method may comprise providing a plurality of barcoded nucleic acid molecules and hybridizing barcoded nucleic acid molecules comprising targeted regions of interest to oligonucleotide probes ("baits") which are complementary to the targeted regions of interest (or to regions near or adjacent to the targeted regions of interest). Baits may be attached to a capture molecule, including without limitation a biotin molecule. The capture molecule (e.g., biotin) can be used to selectively pull down the targeted regions of interest (for example, with magnetic streptavidin beads) to thereby enrich the resultant population of barcoded nucleic acid molecules for those containing the targeted regions of interest.

### Multiplexing:

The present disclosure provides methods and systems for multiplexing, and otherwise increasing throughput in, analysis. For example, a single or integrated process workflow may permit the processing, identification, and/or analysis of more or multiple analytes, more or multiple types of analytes, and/or more or multiple types of analyte characterizations. For example, in the methods and systems described herein, one or more labelling agents capable of binding to or otherwise coupling to one or more cell features may be used to characterize biological particles and/or cell features. In various instances, cell features include cell surface features. Cell surface features may include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In various instances, cell features may include intracellular analytes, such as proteins, protein modifications (e.g., phosphorylation status or other post-translational modifications), nuclear proteins, nuclear membrane proteins, or any combination thereof. A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have a first reporter oligonucleotide coupled thereto, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat. Pub. 20190367969.

In a particular example, a library of potential cell feature labelling agents may be provided, where the respective cell feature labelling agents are associated with nucleic acid reporter molecules, such that a different reporter oligonucleotide sequence is associated with each labelling agent capable of binding to a specific cell feature. In various aspects, different members of the library may be characterized by the presence of a different oligonucleotide sequence label. For example, an antibody capable of binding to a first protein may have associated with it a first reporter oligonucleotide sequence, while an antibody capable of binding to a second protein may have a different reporter oligonucleotide sequence associated with it. The presence of the particular oligonucleotide sequence may be indicative of the presence of a particular antibody or cell feature which may be recognized or bound by the particular antibody.

Labelling agents capable of binding to or otherwise coupling to one or more biological particles may be used to characterize a biological particle as belonging to a particular set of biological particles. For example, labeling agents may be used to label a sample of cells or a group of cells. In this way, a group of cells may be labeled as different from another group of cells. In an example, a first group of cells may originate from a first sample and a second group of cells may originate from a second sample. Labelling agents may allow the first group and second group to have a different labeling agent (or reporter oligonucleotide associated with the labeling agent). This may, for example, facilitate multiplexing, where cells of the first group and cells of the second group may be labeled separately and then pooled together for downstream analysis. The downstream detection of a label may indicate analytes as belonging to a particular group.

For example, a reporter oligonucleotide may be linked to an antibody or an epitope binding fragment thereof, and labeling a biological particle may comprise subjecting the antibody-linked barcode molecule or the epitope binding fragment-linked barcode molecule to conditions suitable for binding the antibody to a molecule present on a surface of the biological particle. The binding affinity between the antibody or the epitope binding fragment thereof and the molecule present on the surface may be within a desired range to ensure that the antibody or the epitope binding fragment thereof remains bound to the molecule. For example, the binding affinity may be within a desired range to ensure that the antibody or the epitope binding fragment thereof remains bound to the molecule during various sample processing steps, such as partitioning and/or nucleic acid amplification or extension. A dissociation constant (Kd) between the antibody or an epitope binding fragment thereof and the molecule to which it binds may be less than about 100 µM, 90 µM, 80 µM, 70 µM, 60 µM, 50 µM, 40 µM, 30 µM, 20 µM, 10 µM, 9 µM, 8 µM, 7 µM, 6 µM, 5 µM, 4 µM, 3 µM, 2 µM, 1 µM, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, 10 pM, 9 pM, 8 pM, 7 pM, 6 pM, 5 pM, 4 pM, 3 pM, 2 pM, or 1 pM. For example, the dissociation constant may be less than about 10 µM.

In another example, a reporter oligonucleotide may be coupled to a cell-penetrating peptide (CPP), and labeling cells may comprise delivering the CPP coupled reporter oligonucleotide into a biological particle. Labeling biological particles may comprise delivering the CPP conjugated oligonucleotide into a cell and/or cell bead by the cell-penetrating peptide. A cell-penetrating peptide that can be used in the methods provided herein can comprise at least one non-functional cysteine residue, which may be either free or derivatized to form a disulfide link with an oligonucleotide that has been modified for such linkage. Non-limiting examples of cell-penetrating peptides that can be used in embodiments herein include penetratin, transportan, plsl, TAT(48-60), pVEC, MTS, and MAP. Cell-penetrating peptides useful in the methods provided herein can have the capability of inducing cell penetration for at least about 30%, 40%, 50%, 60%, 70%, 80%, 90%, 95%, 96%, 97%, 98%, 99%, or 100% of cells of a cell population. The cell-penetrating peptide may be an arginine-rich peptide transporter. The cell-penetrating peptide may be Penetratin or the Tat peptide.

In another example, a reporter oligonucleotide may be coupled to a fluorophore or dye, and labeling cells may comprise subjecting the fluorophore-linked barcode molecule to conditions suitable for binding the fluorophore to the surface of the biological particle. In various instances, fluorophores can interact strongly with lipid bilayers and labeling biological particles may comprise subjecting the fluorophore-linked barcode molecule to conditions such that the fluorophore binds to or is inserted into a membrane of the biological particle. In various cases, the fluorophore is a water-soluble, organic fluorophore. In various instances, the fluorophore is Alexa 532 maleimide, tetramethylrhodamine-5-maleimide (TMR maleimide), BODIPY-TMR maleimide, Sulfo-Cy3 maleimide, Alexa 546 carboxylic acid/succinimidyl ester, Atto 550 maleimide, Cy3 carboxylic acid/succinimidyl ester, Cy3B carboxylic acid/succinimidyl ester, Atto 565 biotin, Sulforhodamine B, Alexa 594 maleimide, Texas Red maleimide, Alexa 633 maleimide, Abberior STAR 635P azide, Atto 647N maleimide, Atto 647 SE, or Sulfo-Cy5 maleimide. See, e.g., Hughes L D, et al. PLoS One. 2014 Feb. 4; 9(2):e87649 for a description of organic fluorophores.

A reporter oligonucleotide may be coupled to a lipophilic molecule, and labeling biological particles may comprise delivering the nucleic acid barcode molecule to a membrane of the biological particle or a nuclear membrane by the lipophilic molecule. Lipophilic molecules can associate with and/or insert into lipid membranes such as cell membranes and nuclear membranes. In various cases, the insertion can be reversible. In various cases, the association between the lipophilic molecule and biological particle may be such that the biological particle retains the lipophilic molecule (e.g., and associated components, such as nucleic acid barcode molecules, thereof) during subsequent processing (e.g., partitioning, cell permeabilization, amplification, pooling, etc.). The reporter nucleotide may enter into the intracellular space and/or a cell nucleus.

A reporter oligonucleotide may be part of a nucleic acid molecule comprising any number of functional sequences, as described elsewhere herein, such as a target capture sequence, a random primer sequence, and the like, and coupled to another nucleic acid molecule that is, or is derived from, the analyte.

Prior to partitioning, the cells may be incubated with the library of labelling agents, that may be labelling agents to a broad panel of different cell features, e.g., receptors, proteins, etc., and which include their associated reporter oligonucleotides. Unbound labelling agents may be washed from the cells, and the cells may then be co-partitioned (e.g., into droplets or wells) along with partition-specific barcode oligonucleotides (e.g., attached to a support, such as a bead or gel bead) as described elsewhere herein. As a result, the partitions may include the cell or cells, as well as the bound labelling agents and their known, associated reporter oligonucleotides.

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide. For example, the first plurality of the labeling agent and second plurality of the labeling agent may interact with different cells, cell populations or samples, allowing a particular report oligonucleotide to indicate a particular cell population (or cell or sample) and cell feature. In this way, different samples or groups can be independently processed and subsequently combined together for pooled analysis (e.g., partition-based barcoding as described elsewhere herein). See, e.g., U.S. Pat. Pub. 20190323088.

As described elsewhere herein, libraries of labelling agents may be associated with a particular cell feature as well as be used to identify analytes as originating from a particular biological particle, population, or sample. The biological particles may be incubated with a plurality of libraries and a given biological particle may comprise multiple labelling agents. For example, a cell may comprise coupled thereto a lipophilic labeling agent and an antibody. The lipophilic labeling agent may indicate that the cell is a member of a particular cell sample, whereas the antibody may indicate that the cell comprises a particular analyte. In this manner, the reporter oligonucleotides and labelling agents may allow multi-analyte, multiplexed analyses to be performed.

In various instances, these reporter oligonucleotides may comprise nucleic acid barcode sequences that permit identification of the labelling agent to which the reporter oligonucleotide is coupled. The use of oligonucleotides as the reporter may provide advantages of being able to generate significant diversity in terms of sequence, while also being readily attachable to most biomolecules, e.g., antibodies, etc., as well as being readily detected, e.g., using sequencing or array technologies.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment), e.g., via a linker, using chemical conjugation techniques (e.g., Lightning-Link^{®} antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715. Likewise, protein and peptide biotinylation techniques have been developed and are readily available. See, e.g., U.S. Pat. No. 6,265,552. Furthermore, click reaction chemistry such as a Methyltetrazine-PEG5-NHS Ester reaction, a TCO-PEG4-NHS Ester reaction, or the like, may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the label agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In various embodiments, the reporter oligonucleotides are releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein. In various instances, the reporter oligonucleotides described herein may include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In various cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In various cases, a label is conjugated to an oligonucleotide that is complementary to a sequence of the reporter oligonucleotide, and the oligonucleotide may be allowed to hybridize to the reporter oligonucleotide.

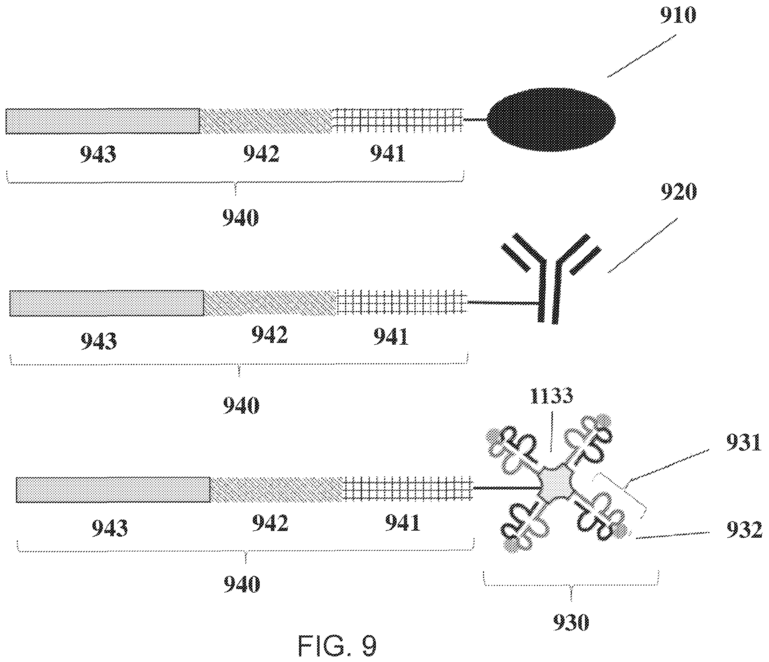

**FIG. 9** describes exemplary labelling agents **(910, 920, 930)** comprising reporter oligonucleotides **(940)** attached thereto. Labelling agent **910** (e.g., any of the labelling agents described herein) is attached (either directly, e.g., covalently attached, or indirectly) to reporter oligonucleotide **940.** Reporter oligonucleotide **940** may comprise barcode sequence **942** that identifies labelling agent **910.** Reporter oligonucleotide **940** may also comprise one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer binding sequence (such as an R1, R2, or partial R1 or R2 sequence).

Referring to **FIG. 9****,** in various instances, reporter oligonucleotide **940** conjugated to a labelling agent (e.g., **910, 920, 930)** comprises a functional sequence **941** (e.g., a primer sequence), a barcode sequence that identifies the labelling agent (e.g., **910, 920, 930),** and functional sequence **943.** Functional sequence **943** can be a reporter capture handle sequence configured to hybridize to a complementary sequence, such as a complementary sequence present on a nucleic acid barcode molecule **990** (not shown), such as those described elsewhere herein. In various instances, nucleic acid barcode molecule **990** is attached to a support (e.g., a bead, such as a gel bead), such as those described elsewhere herein. For example, nucleic acid barcode molecule **990** may be attached to the support via a releasable linkage (e.g., comprising a labile bond), such as those described elsewhere herein. In various instances, reporter oligonucleotide **940** comprises one or more additional functional sequences, such as those described above.

In various instances, the labelling agent **910** is a protein or polypeptide (e.g., an antigen or prospective antigen) comprising reporter oligonucleotide **940.** Reporter oligonucleotide **940** comprises barcode sequence **942** that identifies polypeptide **910** and can be used to infer the presence of an analyte, e.g., a binding partner of polypeptide **910** (i.e., a molecule or compound to which polypeptide **910** can bind). In various instances, the labelling agent **910** is a lipophilic moiety (e.g., cholesterol) comprising reporter oligonucleotide **940,** where the lipophilic moiety is selected such that labelling agent **910** integrates into a membrane of a cell or nucleus. Reporter oligonucleotide **940** comprises barcode sequence **942** that identifies lipophilic moiety **910** which in various instances is used to tag cells (e.g., groups of cells, cell samples, etc.) and may be used for multiplex analyses as described elsewhere herein. In various instances, the labelling agent is an antibody **920** (or an epitope binding fragment thereof) comprising reporter oligonucleotide **940.** Reporter oligonucleotide **940** comprises barcode sequence **942** that identifies antibody **920** and can be used to infer the presence of, e.g., a target of antibody **920** (i.e., a molecule or compound to which antibody **920** binds). In other embodiments, labelling agent **930** comprises an MHC molecule **931** comprising peptide **932** and reporter oligonucleotide **940** that identifies peptide **932.** In various instances, the MHC molecule is coupled to a support **933.** In various instances, support **933** may be a polypeptide, such as streptavidin, or a polysaccharide, such as dextran. In various instances, reporter oligonucleotide **940** may be directly or indirectly coupled to MHC labelling agent **930** in any suitable manner. For example, reporter oligonucleotide **940** may be coupled to MHC molecule **931,** support **933,** or peptide **932.** In various embodiments, labelling agent **930** comprises a plurality of MHC molecules, (e.g. is an MHC multimer, which may be coupled to a support (e.g., **933)).** There are many possible configurations of Class I and/or Class II MHC multimers that can be utilized with the compositions, methods, and systems disclosed herein, e.g., MHC tetramers, MHC pentamers (MHC assembled via a coiled-coil domain, e.g., Pro5^{®} MHC Class I Pentamers, (ProImmune, Ltd.), MHC octamers, MHC dodecamers, MHC decorated dextran molecules (e.g., MHC Dextramer^{®} (Immudex)), etc. For a description of exemplary labelling agents, including antibody and MHC-based labelling agents, reporter oligonucleotides, and methods of use, see, e.g., U.S. Pat. 10,550,429 and U.S. Pat. Pub. 20190367969.

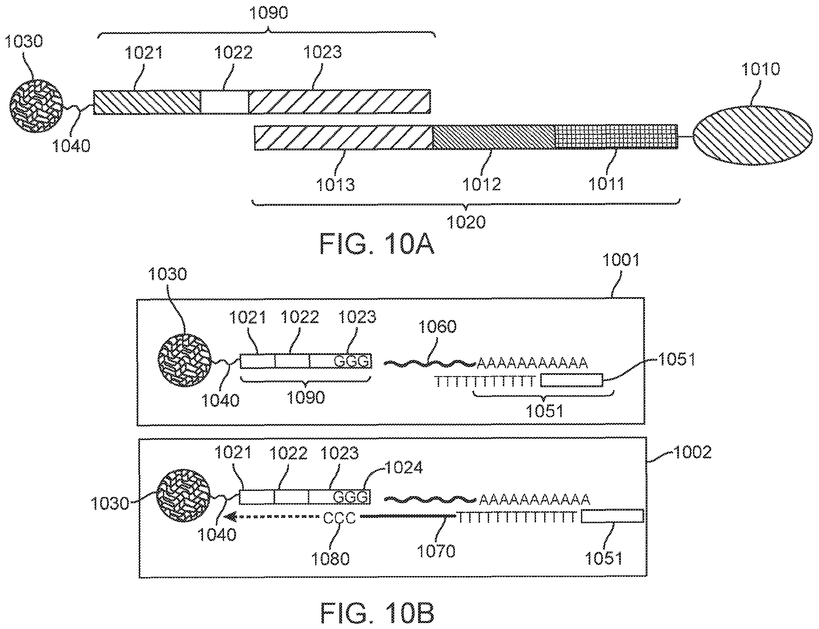

**FIG. 11** illustrates another example of a barcode carrying bead. In various embodiments, analysis of multiple analytes (e.g., RNA and one or more analytes using labelling agents described herein) may comprise nucleic acid barcode molecules as generally depicted in **FIG. 11****.** In various embodiments, nucleic acid barcode molecules **1110** and **1120** are attached to support **1130** via a releasable linkage **1140** (e.g., comprising a labile bond) as described elsewhere herein. Nucleic acid barcode molecule **1110** may comprise adapter sequence **1111,** barcode sequence **1112** and capture sequence **1113.** Nucleic acid barcode molecule **1120** may comprise adapter sequence **1121,** barcode sequence **1112,** and capture sequence **1123,** wherein capture sequence **1123** comprises a different sequence than capture sequence **1113.** In various instances, adapter **1111** and adapter **1121** comprise the same sequence. In various instances, adapter **1111** and adapter **1121** comprise different sequences. Although support **1130** is shown comprising nucleic acid barcode molecules **1110** and **1120,** any suitable number of barcode molecules comprising common barcode sequence **1112** are contemplated herein. For example, in various embodiments, support **1130** further comprises nucleic acid barcode molecule **1150.** Nucleic acid barcode molecule **1150** may comprise adapter sequence **1151,** barcode sequence **1112** and capture sequence **1153,** wherein capture sequence **1153** comprises a different sequence than capture sequence **1113** and **1123.** In various instances, nucleic acid barcode molecules (e.g., **1110, 1120, 1150)** comprise one or more additional functional sequences, such as a UMI or other sequences described herein. The nucleic acid barcode molecules **1110, 1120** or **1150** may interact with analytes as described elsewhere herein, for example, as depicted in **FIGS. 10A-C****.**

Referring to **FIG. 10A****,** in an instance where cells are labelled with labeling agents, capture sequence **1023** may be complementary to an adapter sequence of a reporter oligonucleotide. Cells may be contacted with one or more reporter oligonucleotide **1020** conjugated labelling agents **1010** (e.g., polypeptide, antibody, or others described elsewhere herein). In various cases, the cells may be further processed prior to barcoding. For example, such processing steps may include one or more washing and/or cell sorting steps. In various instances, a cell that is bound to labelling agent **1010** which is conjugated to oligonucleotide **1020** and support **1030** (e.g., a bead, such as a gel bead) comprising nucleic acid barcode molecule **1090** is partitioned into a partition amongst a plurality of partitions (e.g., a droplet of a droplet emulsion or a well of a microwell array). In various instances, the partition comprises at most a single cell bound to labelling agent **1010.** In various instances, reporter oligonucleotide **1020** conjugated to labelling agent **1010** (e.g., polypeptide, an antibody, pMHC molecule such as an MHC multimer, etc.) comprises a first adapter sequence **1011** (e.g., a primer sequence), a barcode sequence **1012** that identifies the labelling agent **1010** (e.g., the polypeptide, antibody, or peptide of a pMHC molecule or complex), and an capture handle sequence **1013.** Capture handle sequence **1013** may be configured to hybridize to a complementary sequence, such as a capture sequence **1023** present on a nucleic acid barcode molecule **1090.** In various instances, oligonucleotide **1020** comprises one or more additional functional sequences, such as those described elsewhere herein.

Barcoded nucleic may be generated (e.g., via a nucleic acid reaction, such as nucleic acid extension or ligation) from the constructs described in **FIGS. 10A-C****.** For example, capture handle sequence **1013** may then be hybridized to complementary sequence, such as capture sequence **1023** to generate (e.g., via a nucleic acid reaction, such as nucleic acid extension or ligation) a barcoded nucleic acid molecule comprising cell (e.g., partition specific) barcode sequence **1022** (or a reverse complement thereof) and reporter barcode sequence **1012** (or a reverse complement thereof). In various embodiments, the nucleic acid barcode molecule **1090** (*e.g.*, partition-specific barcode molecule) further includes a UMI (not shown). Barcoded nucleic acid molecules can then be optionally processed as described elsewhere herein, e.g., to amplify the molecules and/or append sequencing platform specific sequences to the fragments. See, e.g., U.S. Pat. Pub. 2018/0105808. Barcoded nucleic acid molecules, or derivatives generated therefrom, can then be sequenced on a suitable sequencing platform.

In various instances, analysis of multiple analytes (e.g., nucleic acids and one or more analytes using labelling agents described herein) may be performed. For example, the workflow may comprise a workflow as generally depicted in any of **FIGS. 10A-C****,** or a combination of workflows for an individual analyte, as described elsewhere herein. For example, by using a combination of the workflows as generally depicted in **FIGS. 10A-C****,** multiple analytes can be analyzed.

In various instances, analysis of an analyte (e.g. a nucleic acid, a polypeptide, a carbohydrate, a lipid, etc.) comprises a workflow as generally depicted in **FIG. 10A****.** A nucleic acid barcode molecule **1090** may be co-partitioned with the one or more analytes. In various instances, nucleic acid barcode molecule **1090** is attached to a support **1030** (e.g., a bead, such as a gel bead), such as those described elsewhere herein. For example, nucleic acid barcode molecule **1090** may be attached to support **1030** via a releasable linkage **1040** (e.g., comprising a labile bond), such as those described elsewhere herein. Nucleic acid barcode molecule **1090** may comprise a functional sequence **1021** and optionally comprise other additional sequences, for example, a barcode sequence **1022** (*e.g*., common barcode, partition-specific barcode, or other functional sequences described elsewhere herein), and/or a UMI sequence (not shown). The nucleic acid barcode molecule **1090** may comprise a capture sequence **1023** that may be complementary to another nucleic acid sequence, such that it may hybridize to a particular sequence, e.g., capture handle sequence **1013.**

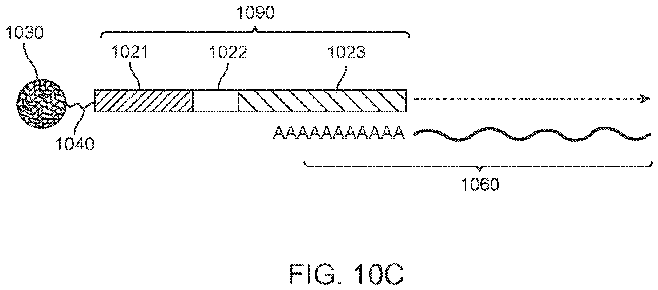

For example, capture sequence **1023** may comprise a poly-T sequence and may be used to hybridize to mRNA. Referring to **FIG. 10C****,** in various embodiments, nucleic acid barcode molecule **1090** comprises capture sequence **1023** complementary to a sequence of RNA molecule **1060** from a cell. In various instances, capture sequence **1023** comprises a sequence specific for an RNA molecule. Capture sequence **1023** may comprise a known or targeted sequence or a random sequence. In various instances, a nucleic acid extension reaction may be performed, thereby generating a barcoded nucleic acid product comprising capture sequence **1023,** the functional sequence **1021,** barcode sequence **1022,** any other functional sequence, and a sequence corresponding to the RNA molecule **1060.**

In another example, capture sequence **1023** may be complementary to an overhang sequence or an adapter sequence that has been appended to an analyte. For example, referring to **FIG. 10B****,** panel **1001,** in various embodiments, primer **1050** comprises a sequence complementary to a sequence of nucleic acid molecule **1060** (such as an RNA encoding for a BCR sequence) from a biological particle. In various instances, primer **1050** comprises one or more sequences **1051** that are not complementary to RNA molecule **1060.** Sequence **1051** may be a functional sequence as described elsewhere herein, for example, an adapter sequence, a sequencing primer sequence, or a sequence the facilitates coupling to a flow cell of a sequencer. In various instances, primer **1050** comprises a poly-T sequence. In various instances, primer **1050** comprises a sequence complementary to a target sequence in an RNA molecule. In various instances, primer **1050** comprises a sequence complementary to a region of an immune molecule, such as the constant region of a TCR or BCR sequence. Primer **1050** is hybridized to nucleic acid molecule **1060** and complementary molecule **1070** is generated (*see* Panel **1202).** For example, complementary molecule **1070** may be cDNA generated in a reverse transcription reaction. In various instances, an additional sequence may be appended to complementary molecule **1070.** For example, the reverse transcriptase enzyme may be selected such that several non-templated bases **1080** (e.g., a poly-C sequence) are appended to the cDNA. In another example, a terminal transferase may also be used to append the additional sequence. Nucleic acid barcode molecule **1090** comprises a sequence **1024** complementary to the non-templated bases, and the reverse transcriptase performs a template switching reaction onto nucleic acid barcode molecule **1090** to generate a barcoded nucleic acid molecule comprising cell (e.g., partition specific) barcode sequence **1022** (or a reverse complement thereof) and a sequence of complementary molecule **1070** (or a portion thereof). In various instances, sequence **1023** comprises a sequence complementary to a region of an immune molecule, such as the constant region of a TCR or BCR sequence. Sequence **1023** is hybridized to nucleic acid molecule **1060** and a complementary molecule **1070** is generated. For example, complementary molecule **1070** may be generated in a reverse transcription reaction generating a barcoded nucleic acid molecule comprising cell (e.g., partition specific) barcode sequence **1022** (or a reverse complement thereof) and a sequence of complementary molecule **1070** (or a portion thereof). Additional methods and compositions suitable for barcoding cDNA generated from mRNA transcripts including those encoding V(D)J regions of an immune cell receptor and/or barcoding methods and composition including a template switch oligonucleotide are described in International Patent Application WO2018/075693, U.S. Patent Publication No. 2018/0105808, U.S. Patent Publication No. 2015/0376609, filed June 26, 2015, and U.S. Patent Publication No. 2019/0367969.

The methods described herein may comprise templated ligation. A templated ligation process may comprise contacting a nucleic acid molecule (e.g., an RNA molecule) with a probe molecule. The probe molecule may interact with one or more other probe molecules, for example, comprising a barcode sequence, to generate a probe-barcode complex. An extension reaction may be performed on at least a portion of the probe-barcode complex to generate a nucleic acid product that comprises the barcode sequence and is associated with a sequence of the nucleic acid molecule. Beneficially, the methods described herein may allow barcoding of the nucleic acid molecule without performing reverse transcription on the nucleic acid molecule. The methods herein may comprise ligation-mediated reactions.

A method may comprise contacting a nucleic acid molecule (e.g., an RNA molecule) with a first probe molecule, comprising a first sequence and a second sequence, under conditions sufficient for the first sequence to hybridize to a sequence of the nucleic acid molecule. A second probe molecule comprising a third sequence may hybridize to the second sequence of the first probe molecule. The first probe or the second probe molecule may comprise a barcode sequence (e.g., as described herein). For example, the second probe molecule may be a nucleic acid molecule (e.g., as described herein). In some cases, a splint molecule may be used to link the first and second probe molecules. For example, a fourth sequence of the splint molecule may hybridize to the second sequence of the first probe molecule and a fifth sequence of the splint molecule may hybridize to the third sequence of the second probe molecule.

In another example, a first probe molecule with a first reactive moiety and a second probe molecule with a second reactive moiety may be used. A first sequence of the first probe molecule may hybridize to a first sequence of a nucleic acid molecule and a second sequence of the second probe molecule may hybridize to a second sequence of the nucleic acid molecule. The first and second sequences of the nucleic acid molecule may be adjacent or may be separated by a gap of one or more nucleotides, which gap may optionally be filled (e.g., using a polymerase). The first reactive moiety of the first probe molecule and the second reactive moiety of the second probe molecule may be subjected to conditions sufficient for the first and second reactive moieties to react to provide a linking moiety. For example, a click chemistry reaction involving an alkyne moiety and an azide moiety may be used to provide a triazole linking moiety. In other examples, an iodide moiety may be chemically ligated to a phosphorothioate moiety to form a phosphorothioate bond, an acid may be ligated to an amine to form an amide bond, or a phosphate may be ligated to an amine to form a phosphoramidate bond. In some cases, the probes may be subjected to an enzymatic ligation reaction, using a ligase, e.g., SplintR ligases, T4 ligases, KOD ligases, PBCV1 enzymes, etc. to form a probe-linked nucleic acid molecule. Where the two probes are non-adjacent, gap regions between the probes may be filled prior to ligation. In some instances, ribonucleotides or deoxyribonucleotides are ligated between the first and second probes.

Prior to, in parallel, or subsequent to linking of the first and second probe molecules (e.g., via reaction between their respective reactive moieties), a third probe molecule (e.g., a nucleic acid barcode molecule) may be subjected to conditions sufficient to hybridize to a third sequence of the first probe molecule. The third probe molecule may comprise a barcode sequence. In some cases, a splint molecule may be used to link the first and third probe molecules. In some cases, the first and second probe molecules may be linked to one another such that a loop or "padlock" is formed after hybridization of the first sequence of the first probe molecule to the first sequence of the nucleic acid molecule and the second sequence of the second probe molecule to the second sequence of the nucleic acid molecule. A linkage between the first and second probe molecules may be generated after hybridization of the first and second probe molecules to the nucleic acid molecule, such as via reaction between two reactive moieties to form a linking moiety. Alternatively, the first and second probe molecules may be linked to one another before the first and second probe molecules hybridize to the nucleic acid molecule.

All or a portion of the templated ligation processes described herein may be performed within a partition (e.g., as described herein). Alternatively, one or more such processes may be performed within a bulk solution. For example, one or more probe molecules may be subjected to conditions sufficient to hybridize to a nucleic acid molecule (e.g., a nucleic acid molecule included in an biological particle such as a cell) within a bulk solution. The nucleic acid molecule may be partitioned within various reagents (e.g., as described herein) including a nucleic acid barcode molecule, such as a nucleic acid barcode molecule releasably coupled to a bead (e.g., as described herein). Within the partition, the nucleic acid barcode molecule may hybridize to a sequence of a probe molecule hybridized to the nucleic acid molecule, thereby generated a barcode-linked nucleic acid molecule. Templated ligation processes may permit indirect barcoding of a nucleic acid molecule without the use of reverse transcription. Details of such processes and additional schemes are included in, for example, International Patent Publication No. WO2019/165318.

In some instances, barcoding of a nucleic acid molecule may be done using a combinatorial approach. In such instances, one or more nucleic acid molecules (which may be comprised in a biological particle, e.g., a cell, e.g., a fixed cell, organelle, nucleus, or cell bead) may be partitioned (e.g., in a first set of partitions, e.g., wells or droplets) with one or more first nucleic acid barcode molecules (optionally coupled to a bead). The first nucleic acid barcode molecules or derivative thereof (e.g., complement, reverse complement) may then be attached to the one or more nucleic acid molecules, thereby generating barcoded nucleic acid molecules, e.g., using the processes described herein. The first nucleic acid barcode molecules may be partitioned to the first set of partitions such that a nucleic acid barcode molecule, of the first nucleic acid barcode molecules, that is in a partition comprises a barcode sequence that is unique to the partition among the first set of partitions. Each partition may comprise a unique barcode sequence. For example, a set of first nucleic acid barcode molecules partitioned to a first partition in the first set of partitions may each comprise a common barcode sequence that is unique to the first partition among the first set of partitions, and a second set of first nucleic acid barcode molecules partitioned to a second partition in the first set of partitions may each comprise another common barcode sequence that is unique to the second partition among the first set of partitions. Such barcode sequence (unique to the partition) may be useful in determining the cell or partition from which the one or more nucleic acid molecules (or derivatives thereof) originated.

The barcoded nucleic acid molecules from multiple partitions of the first set of partitions may be pooled and re-partitioned (e.g., in a second set of partitions, e.g., one or more wells or droplets) with one or more second nucleic acid barcode molecules. The second nucleic acid barcode molecules or derivative thereof may then be attached to the barcoded nucleic acid molecules. As with the first nucleic acid barcode molecules during the first round of partitioning, the second nucleic acid barcode molecules may be partitioned to the second set of partitions such that a nucleic acid barcode molecule, of the second nucleic acid barcode molecules, that is in a partition comprises a barcode sequence that is unique to the partition among the second set of partitions. Such barcode sequence may also be useful in determining the cell or partition from which the one or more nucleic acid molecules or first barcoded nucleic acid molecules originated. The barcoded nucleic acid molecules may thus comprise two barcode sequences (e.g., from the first nucleic acid barcode molecules and the second nucleic acid barcode molecules).

Additional barcode sequences may be attached to the barcoded nucleic acid molecules by repeating the processes any number of times (e.g., in a split-and-pool approach), thereby combinatorically synthesizing unique barcode sequences to barcode the one or more nucleic acid molecules. For example, combinatorial barcoding may comprise at least 1, 2, 3, 4, 5, 6, 7, 8, 9, 10 or more operations of splitting (e.g., partitioning) and/or pooling (e.g., from the partitions). Additional examples of combinatorial barcoding may also be found in International Patent Publication No. WO2019/165318.

Beneficially, the combinatorial barcode approach may be useful for generating greater barcode diversity, and synthesizing unique barcode sequences on nucleic acid molecules derived from a cell or partition. For example, combinatorial barcoding comprising three operations, each with 100 partitions, may yield up to 106 unique barcode combinations. In some instances, the combinatorial barcode approach may be helpful in determining whether a partition contained only one cell or more than one cell. For instance, the sequences of the first nucleic acid barcode molecule and the second nucleic acid barcode molecule may be used to determine whether a partition comprised more than one cell. For instance, if two nucleic acid molecules comprise different first barcode sequences but the same second barcode sequences, it may be inferred that the second set of partitions comprised two or more cells.

In some instances, combinatorial barcoding may be achieved in the same compartment. For instance, a unique nucleic acid molecule comprising one or more nucleic acid bases may be attached to a nucleic acid molecule (e.g., a sample or target nucleic acid molecule) in successive operations within a partition (e.g., droplet or well) to generate a barcoded nucleic acid molecule. A second unique nucleic acid molecule comprising one or more nucleic acid bases may be attached to the barcoded nucleic acid molecule. In some instances, all the reagents for barcoding and generating combinatorially barcoded molecules may be provided in a single reaction mixture, or the reagents may be provided sequentially.

In some instances, cell beads comprising nucleic acid molecules may be barcoded. Methods and systems for barcoding cell beads are further described in PCT/US2018/067356 and U.S. Pat. Pub. No. 2019/0330694.

In some instances wherein a partition is a volume wherein diffusion of contents beyond the volume is inhibited, the partition contains a diffusion resistant material. Such partition may also be referred to herein as a diffusion resistant partition. The diffusion resistant material may have an increased viscosity. The diffusion resistant material may be or comprise a matrix, e.g., a polymeric matrix, or a gel. Suitable polymers or gels are disclosed herein. The matrix can be a porous matrix capable of entraining and/or retaining materials within its matrix. In some embodiments, a diffusion resistant partition comprises a single biological particle and a single bead, the single bead comprising a plurality of nucleic acid barcode molecules comprising a partition specific barcode sequence. In some embodiments the partition specific barcode sequence is unique to the diffusion resistant partition. In some embodiments, partitioning comprises contacting a plurality of biological particles with a plurality of beads in a diffusion resistant material to provide a diffusion resistant partition comprising a single biological particle and a single bead. In some embodiments, partitioning comprises contacting a plurality of biological particles with a plurality of beads in a liquid comprising a polymeric precursor material that may be capable of being formed into a gel or other solid or semi-solid matrix, and subjecting the liquid to conditions sufficient to polymerize or gel the precursors, e.g., as described herein. In some embodiments, the biological particle may be lysed or permeabilized in the diffusion resistant partition. In some embodiments, a nucleic acid analyte of the biological particle (which may include a reporter oligonucleotide associated with a labelling agent disclosed herein) may be coupled with a nucleic acid barcode molecule in the diffusion resistant partition. In some cases, further processing, e.g., generation of barcoded nucleic acid molecules, may be performed in the diffusion resistant partition or in bulk. For example, nucleic acid analytes, once coupled to nucleic acid barcode molecules in partitions, may be pooled and then subjected to further processing in bulk (e.g., extension, reverse transcription, or other processing) to generate barcoded nucleic acid molecules. For other example, nucleic acid analytes, one coupled to nucleic acid barcode molecules in diffusion resistant partitions, may be subjected to further processing in the diffusion resistant partitions to generate barcoded nucleic acid molecules.

## Claims

1. A method for assessing enzymatic activity, the method comprising:
(i) contacting a substrate complex with an enzyme, wherein the substrate complex comprises:
a cleavable substrate comprising a first portion linked to a second portion;
a substrate barcoded oligonucleotide (SBO) construct, comprising:
a first oligonucleotide comprising a barcode sequence; and
a first linker that connects the first oligonucleotide to the first portion of the substrate;
a blocking construct, comprising a second oligonucleotide that is complementary to at least a portion of the first oligonucleotide; and
a second linker that connects the second oligonucleotide to the second portion of the substrate;
(ii) restricting movement of the blocking construct relative to the SBO construct to inhibit generation of a reverse complement of the barcode sequence with the blocking construct;
(iii) cleaving the cleavable substrate with the enzyme; and
(iv) releasing the blocking construct from the SBO construct.

2. The method of claim 1, further comprising the step of:
(v) conducting an amplification reaction on the first oligonucleotide comprising the barcode sequence to produce an extension product.

3. The method of claim 2, wherein the amplification reaction comprises the steps of:
annealing a first adapter to a first adapter binding site adjacent to a 5' end of the barcode sequence and a second adapter to a second adapter binding site adjacent to a 3' end of the barcode sequence; and
generating a reverse complement of the barcode sequence.

4. The method of claim 3, wherein the generating step uses an amplification enzyme, which is optionally a polymerase or a reverse transcriptase.

5. The method according to any one of claims 2-4, further comprising the step of:
(vi) assessing enzymatic activity based on a reaction product of the amplification reaction, wherein the reaction product optionally comprises a detectable label or encodes a detectable label.

6. The method according to any one of claims 1-5, wherein the barcode sequence of the first oligonucleotide identifies the cleavable substrate, and wherein the method further comprises the step of identifying the enzyme or cleavable substrate using the barcode sequence.

7. The method according to any one of claims 1-6, further comprising the step of modifying an amino acid sequence of the enzyme, optionally wherein the step of modifying occurs before contacting the substrate complex with the enzyme.

8. The method according to claim 7, wherein modifying the amino acid sequence of the enzyme comprises a CRISPR-mediated amino acid sequence modification.

9. The method according to any one of claims 1-8, wherein:
(i) the substrate comprises a protein or a polynucleotide; and/or
(ii) the first and second linkers each comprise an inert polymer, which optionally comprises polyethylene glycol (PEG).

10. The method according to any one of claims 1-9, further comprising the step of increasing an affinity between the SBO construct and the blocking construct.

11. The method according to any one of claims 1-10, wherein:
(i) the second oligonucleotide comprises at least one LNA, or optionally comprises only LNAs;
(ii) the portion of the first oligonucleotide that is complementary to the second nucleotide comprises at least one LNA, or optionally comprises only LNAs; and/or
(iii) the second oligonucleotide comprises a non-reactive 3' end, which is optionally a phosphorylated 3' end.

12. The method according to any one of claims 1-11, wherein the method is conducted within a partition, optionally wherein the partition is a droplet, and optionally the partition comprises: a third oligonucleotide comprising a nucleic acid barcode molecule, a partition-specific barcode sequence, a capture sequence, a unique molecular identifier (UMI), a splint oligonucleotide, or any combination thereof.

13. The method of claim 12, wherein the partition comprises a bead.

14. A kit comprising:
(i) a cleavable substrate comprising a first portion linked to a second portion;
(ii) a substrate barcoded oligonucleotide (SBO) construct, comprising:
a first oligonucleotide comprising a barcode sequence that identifies the cleavable substrate; and
a first linker that connects the first oligonucleotide to the first portion of the substrate;
(iii) a blocking construct, comprising:
a second oligonucleotide that is complementary to at least a portion of the first oligonucleotide; and
a second linker that connects the second oligonucleotide to the second portion of the substrate.

15. The kit of claim 14, further comprising:
(iv) a plurality of first adapters comprising a sequence that is complementary to at least a portion of the first oligonucleotide; and
(v) a plurality of second adapters comprising a sequence that is complementary to at least a portion of the first oligonucleotide.

16. The kit according to claim 14 or 15, further comprising:
(vi) one or more reagents for conducting an amplification reaction;
(vii) a bead coupled to a plurality of third oligonucleotides, wherein each of the plurality of third oligonucleotides comprises:
a nucleic acid barcode molecule comprising a bead specific barcode; and
a capture sequence, wherein the capture sequence is complementary to at least a portion of the first oligonucleotide of the SBO construct, optionally
wherein each of the third oligonucleotides further comprises a unique molecular identifier (UMI); and/or
(viii) one or more reagents for conducting a CRISPR modification reaction.

17. The kit of claim 14, wherein the first portion of the cleavable substrate and the second portion of the cleavable substrate are covalently linked.

## Patentansprüche

1. Verfahren zur Bewertung der enzymatischen Aktivität, das Verfahren umfassend:
(i) Kontaktieren eines Substratkomplexes mit einem Enzym, wobei der Substratkomplex umfasst:
ein spaltbares Substrat umfassend einen ersten Abschnitt, der mit einem zweiten Abschnitt verknüpft ist;
ein Substrat-Barcodiertes-Oligonukleotid(SBO)-Konstrukt, umfassend:
ein erstes Oligonukleotid umfassend eine erste Barcodesequenz; und
einen ersten Linker, der das erste Oligonukleotid mit dem ersten Abschnitt des Substrats verbindet;
ein blockierendes Konstrukt, umfassend ein zweites Oligonukleotid, das zu mindestens einem Abschnitt des ersten Oligonukleotids komplementär ist; und einen zweiten Linker, der das zweite Oligonukleotid mit dem zweiten Abschnitt des Substrats verbindet;
(ii) Beschränken der Bewegung des blockierenden Konstrukts in Bezug auf das SBO-Konstrukt, um eine Erzeugung eines reversen Komplements der Barcodesequenz mit dem blockierenden Konstrukt zu hemmen;
(iii) Spalten des spaltbaren Substrats mit dem Enzym; und
(iv) Freisetzen des blockierenden Konstrukts aus dem SBO-Konstrukt.

2. Verfahren nach Anspruch 1, ferner umfassend den Schritt:
(v) Durchführung einer Amplifikationsreaktion mit dem die Barcodesequenz umfassenden ersten Oligonukleotid zur Herstellung eines Verlängerungsprodukts.

3. Verfahren nach Anspruch 2, wobei die Amplifikationsreaktion die folgenden Schritte umfasst:
Anlagern eines ersten Adapters an eine erste Adapterbindungsstelle angrenzend an ein 5'-Ende der Barcodesequenz und eines zweiten Adapters an eine zweite Adapterbindungsstelle angrenzend an ein 3'-Ende der Barcodesequenz; und
Erzeugen eines reversen Komplements der Barcodesequenz.

4. Verfahren nach Anspruch 3, wobei der Erzeugungsschritt unter Verwendung eines Amplifikationsenzyms erfolgt, das gegebenenfalls eine Polymerase oder eine reverse Transkriptase ist.

5. Verfahren nach einem der Ansprüche 2 bis 4, ferner umfassend den Schritt:
(vi) Bewerten der enzymatischen Aktivität basierend auf einem Reaktionsprodukt der Amplifikationsreaktion, wobei das Reaktionsprodukt gegebenenfalls eine nachweisbare Markierung umfasst oder eine nachweisbare Markierung codiert.

6. Verfahren nach einem der Ansprüche 1 bis 5, wobei die Barcodesequenz des ersten Oligonukleotids das spaltbare Substrat identifiziert, und wobei das Verfahren ferner den Schritt des Identifizierens des Enzyms oder des spaltbaren Substrats unter Verwendung der Barcodesequenz umfasst.

7. Verfahren nach einem der Ansprüche 1 bis 6, ferner umfassend den Schritt des Modifizierens einer Aminosäuresequenz des Enzyms, wobei der Schritt des Modifizierens gegebenenfalls vor dem Kontaktieren des Substratkomplexes mit dem Enzym erfolgt.

8. Verfahren nach Anspruch 7, wobei das Modifizieren der Aminosäuresequenz des Enzyms eine CRISPR-vermittelte Aminosäuresequenzmodifikation umfasst.

9. Verfahren nach einem der Ansprüche 1 bis 8, wobei:
(i) das Substrat ein Protein oder ein Polynukleotid umfasst; und/oder
(ii) der erste und der zweite Linker jeweils ein inertes Polymer umfassen, das gegebenenfalls Polyethylenglykol (PEG) umfasst.

10. Verfahren nach einem der Ansprüche 1 bis 9, ferner umfassend den Schritt des Erhöhens einer Affinität zwischen dem SBO-Konstrukt und dem blockierenden Konstrukt.

11. Verfahren nach einem der Ansprüche 1 bis 10, wobei:
(i) das zweite Oligonukleotid mindestens eine LNA umfasst oder gegebenenfalls nur LNAs umfasst;
(ii) der Abschnitt des ersten Oligonukleotids, der zu dem zweiten Nukleotid komplementär ist, mindestens eine LNA umfasst oder gegebenenfalls nur LNAs umfasst; und/oder
(iii) das zweite Oligonukleotid ein nicht reaktives 3'-Ende umfasst, das gegebenenfalls ein phosphoryliertes 3'-Ende ist.

12. Verfahren nach einem der Ansprüche 1 bis 11, wobei das Verfahren innerhalb einer Partition durchgeführt wird, wobei die Partition gegebenenfalls ein Tröpfchen ist, und wobei die Partition gegebenenfalls umfasst: ein drittes Oligonukleotid umfassend ein Nukleinsäure-Barcodemolekül, eine partitionsspezifische Barcodesequenz, eine Erfassungssequenz, eine eindeutige molekulare Kennung (UMI), ein Splint-Oligonukleotid oder eine beliebige Kombination davon.

13. Verfahren nach Anspruch 12, wobei die Partition ein Kügelchen umfasst.

14. Kit, umfassend:
(i) ein spaltbares Substrat umfassend einen ersten Abschnitt, der mit einem zweiten Abschnitt verknüpft ist;
(ii) ein Substrat-Barcodiertes-Oligonukleotid(SBO)-Konstrukt, umfassend:
ein erstes Oligonukleotid umfassend eine Barcodesequenz, die das spaltbare Substrat identifiziert; und
einen ersten Linker, der das erste Oligonukleotid mit dem ersten Abschnitt des Substrats verbindet;
(iii) ein blockierendes Konstrukt, umfassend:
ein zweites Oligonukleotid, das zu mindestens einem Abschnitt des ersten Oligonukleotids komplementär ist; und
einen zweiten Linker, der das zweite Oligonukleotid mit dem zweiten Abschnitt des Substrats verbindet.

15. Kit nach Anspruch 14, ferner umfassend:
(iv) eine Vielzahl von ersten Adaptern, umfassend eine Sequenz, die mindestens zu einem Abschnitt des ersten Oligonukleotids komplementär ist; und
(v) eine Vielzahl von zweiten Adaptern, umfassend eine Sequenz, die mindestens zu einem Abschnitt des ersten Oligonukleotids komplementär ist.

16. Kit nach Anspruch 14 oder 15, ferner umfassend:
(vi) ein oder mehrere Reagenzien für ein Durchführen einer Amplifikationsreaktion;
(vii)ein mit einer Vielzahl von dritten Oligonukleotiden gekoppeltes Kügelchen,
wobei jedes der Vielzahl von dritten Oligonukleotiden umfasst:
ein Nukleinsäure-Barcodemolekül, umfassend einen kügelchenspezifischen Barcode; und
eine Erfassungssequenz, wobei die Erfassungssequenz zu mindestens einem Abschnitt des ersten Oligonukleotids des SBO-Konstrukts komplementär ist, gegebenenfalls
wobei jedes der dritten Oligonukleotide ferner eine eindeutige molekulare Kennung (UMI) umfasst; und/oder
(viii) ein oder mehrere Reagenzien für ein Durchführen einer CRISPR-Modifikationsreaktion.

17. Kit nach Anspruch 14, wobei der erste Abschnitt des spaltbaren Substrats und der zweite Abschnitt des spaltbaren Substrats kovalent verknüpft sind.

## Revendications

1. Procédé pour évaluer une activité enzymatique, le procédé comprenant :
(i) la mise en contact d'un complexe de substrat avec une enzyme, dans lequel le complexe de substrat comprend :
un substrat clivable comprenant une première portion liée à une deuxième portion ;
une construction d'oligonucléotide à code à barres de substrat (SBO), comprenant :
un premier oligonucléotide comprenant une séquence de code à barres ; et
un premier lieur qui connecte le premier oligonucléotide à la première portion du substrat ;
une construction de blocage, comprenant un deuxième oligonucléotide qui est complémentaire d'au moins une portion du premier oligonucléotide ; et
un deuxième lieur qui connecte le deuxième oligonucléotide à la deuxième portion du substrat ;
(ii) la limitation du mouvement de la construction de blocage par rapport à la construction SBO pour inhiber la génération d'un complément inverse de la séquence de code à barres avec la construction de blocage ;
(iii) le clivage du substrat clivable avec l'enzyme ; et
(iv) la libération de la construction de blocage de la construction SBO.

2. Procédé selon la revendication 1, comprenant en outre l'étape consistant à :
(v) réaliser une réaction d'amplification sur le premier oligonucléotide comprenant la séquence de code à barres pour produire un produit d'extension.

3. Procédé selon la revendication 2, dans lequel la réaction d'amplification comprend les étapes consistant à :
anneler un premier adaptateur à un premier site de liaison d'adaptateur adjacent à une extrémité 5' de la séquence de code à barres et un deuxième adaptateur à un deuxième site de liaison d'adaptateur adjacent à une extrémité 3' de la séquence de code à barres ; et
la génération d'un complément inverse de la séquence de codes à barres.

4. Procédé selon la revendication 3, dans lequel l'étape de génération utilise une enzyme d'amplification, qui est facultativement une polymérase ou une transcriptase inverse.

5. Procédé selon l'une quelconque des revendications 2 à 4, comprenant en outre l'étape consistant à :
(vi) évaluer l'activité enzymatique sur la base d'un produit de réaction de la réaction d'amplification, dans lequel le produit de réaction comprend facultativement un marqueur détectable ou code pour un marqueur détectable.

6. Procédé selon l'une quelconque des revendications 1 à 5, dans lequel la séquence de code à barres du premier oligonucléotide identifie le substrat clivable, et dans lequel le procédé comprend en outre l'étape consistant à identifier l'enzyme ou le substrat clivable en utilisant la séquence de code à barres.

7. Procédé selon l'une quelconque des revendications 1 à 6, comprenant en outre l'étape consistant à modifier une séquence d'acides aminés de l'enzyme, facultativement dans lequel l'étape de modification a lieu avant la mise en contact du complexe de substrat avec l'enzyme.

8. Procédé selon la revendication 7, dans lequel la modification de la séquence d'acides aminés de l'enzyme comprend une modification de séquence d'acides aminés médiée par CRISPR.

9. Procédé selon l'une quelconque des revendications 1 à 8, dans lequel :
(i) le substrat comprend une protéine ou un polynucléotide ; et/ou
(ii) les premier et deuxième lieurs comprennent chacun un polymère inerte, qui comprend facultativement du polyéthylène glycol (PEG).

10. Procédé selon l'une quelconque des revendications 1 à 9, comprenant en outre l'étape consistant à augmenter une affinité entre la construction SBO et la construction de blocage.

11. Procédé selon l'une quelconque des revendications 1 à 10, dans lequel :
(i) le deuxième oligonucléotide comprend au moins un LNA, ou comprend éventuellement uniquement des LNA ;
(ii) la portion du premier oligonucléotide qui est complémentaire du deuxième nucléotide comprend au moins un LNA, ou comprend facultativement uniquement des LNA ; et/ou
(iii) le deuxième oligonucléotide comprend une extrémité 3' non réactive, qui est facultativement une extrémité 3' phosphorylée.

12. Procédé selon l'une quelconque des revendications 1 à 11, dans lequel le procédé est mis en œuvre au sein d'un compartiment, facultativement dans lequel le compartiment est une gouttelette, et facultativement le compartiment comprend : un troisième oligonucléotide comprenant une molécule de code à barres d'acide nucléique, une séquence de code à barres spécifique de compartiment, une séquence de capture, un identifiant moléculaire unique (UMI), un oligonucléotide d'attelle, ou toute combinaison de ceux-ci.

13. Procédé selon la revendication 12, dans lequel le compartiment comprend une bille.

14. Kit, comprenant :
(i) un substrat clivable comprenant une première portion liée à une deuxième portion ;
(ii) une construction d'oligonucléotide à code à barres de substrat (SBO), comprenant :
un premier oligonucléotide comprenant une séquence de code à barres qui identifie le substrat clivable ; et
un premier lieur qui connecte le premier oligonucléotide à la première portion du substrat ;
(iii) une construction de blocage, comprenant :
un deuxième oligonucléotide qui est complémentaire d'au moins une portion du premier oligonucléotide ; et
un deuxième lieur qui connecte le deuxième oligonucléotide à la deuxième portion du substrat.

15. Kit selon la revendication 14, comprenant en outre :
(iv) une pluralité de premiers adaptateurs comprenant une séquence qui est complémentaire d'au moins une portion du premier oligonucléotide ; et
(v) une pluralité de deuxièmes adaptateurs comprenant une séquence qui est complémentaire d'au moins une portion du premier oligonucléotide.

16. Kit selon la revendication 14 ou 15, comprenant en outre :
(vi) un ou plusieurs réactifs pour mettre en œuvre une réaction d'amplification ;
(vii) une bille couplée à une pluralité de troisièmes oligonucléotides, dans lequel chacun de la pluralité de troisièmes oligonucléotides comprend :
une molécule de code à barres d'acide nucléique comprenant un code à barres spécifique de bille ; et
une séquence de capture, dans lequel la séquence de capture est complémentaire d'au moins une portion du premier oligonucléotide de la construction SBO, facultativement
dans lequel chacun des troisièmes oligonucléotides comprend en outre un identifiant moléculaire unique (UMI) ; et/ou
(viii) un ou plusieurs réactifs pour mettre en œuvre une réaction de modification CRISPR.

17. Kit selon la revendication 14, dans lequel la première portion du substrat clivable et la deuxième portion du substrat clivable sont liées de manière covalente.
